# EUROPEAN PATENT APPLICATION

(11) **EP 2 500 344 A1**
(43) Date of publication of application: **19.09.2012**
(21) Application number: 10829723.5
(22) Date of filing: 12.11.2010
(51) Int. Cl.: C07D 413/12, A61K 31/5355, A61K 31/541, A61P 25/28, A61P 43/00, C07D 417/12

(54) **AMINOTHIAZINE OR AMINOOXAZINE DERIVATIVE HAVING AMINO LINKER**

(30) Priority: 13.11.2009 JP 2009260514
(71) Applicant: Shionogi & Co., Ltd., Osaka 541-0045 (JP)
(72) Inventor: MASUI, Moriyasu, Toyonaka-shi Osaka 561-0825 (JP); ANAN, Kousuke, Toyonaka-shi Osaka 561-0825 (JP)
(74) Representative: Vossius & Partner
(86) International application number: PCT/JP2010/006680
(87) International publication number: WO 2011/058763

(57) **Abstract**

The present invention provide a medicament for treating the diseases induced by production, secretion or deposition of amyloid-β proteins, for example, a compound of the following formula (I) wherein R¹, R^{2a}, R^{2b}, R³, R⁴, R⁵, X, L¹, L², ring A, ring B and the dotted line are defined in the specification, its pharmaceutically acceptable salt or a solvate thereof.

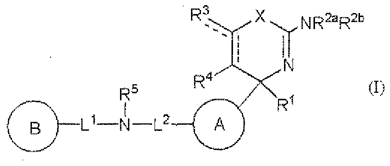

## Description

### [Technical field]

The present invention relates to a compound which has amyloid β production inhibitory activity, and is useful as an agent for treating disease induced by production, secretion and/or deposition of amyloid β protein.

### [Background Art]

In the brain of Alzheimer's patient, the peptide composed of about 40 amino acids residue as is called amyloid β protein, that accumulates to form insoluble specks (senile specks) outside nerve cells is widely observed. It is concerned that these senile specks kill nerve cells to cause Alzheimer's disease, so the therapeutic agents for Alzheimer's disease, such as decomposition agents of amyloid β protein and amyloid vaccine, are under investigation.

Secretase is an enzyme which cleaves a protein called amyloid β precursor protein (APP) in cell and produces amyloid β protein. The enzyme which controls the production of N terminus of amyloid β protein is called as β-secretase (beta-site APP-cleaving enzyme 1, BACE1). It is thought that inhibition of this enzyme leads to reduction of producing amyloid β protein and that the therapeutic agent for Alzheimer's disease will be created due to the inhibition.

Patent Literature 5 describes the compounds which are structurally similar to those of the present invention but the compounds are useful for pigment.

β secretase inhibitor are described in Patent Literatures 1 to 4, 6 and 7, however, all compounds in these literatures have different structures from the present invention-[Prior Art Literatures]

### [Patent Literatures]

[Patent Literature 1] International Patent Application Publication WO 2007/049532
[Patent Literature 2] International Patent Application Publication WO 2008/133274
[Patent Literature 3] International Patent Application Publication WO 2008/133273
[Patent Literature 4] International Patent Application Publication WO 2009/091016
[Patent Literature 5] East Germany Patent Application Publication 140,144
[Patent Literature 6] International Patent Application Publication WO 2010/038686
[Patent Literature 7] International Patent Application Publication WO 2009/151098

### [Disclosure of Invention]

### [Problems to be solved by the Invention]

The present invention provides compounds which have reducing effects to produce amyloid β protein, especially BACE1 inhibitory activity, and are useful as an agent for treating disease induced by production, secretion and/or deposition of amyloid β protein.

### [Means to Solve the Problems]

The present invention, for example, provides the inventions described in the following items.
(1) A compound of formula (I): 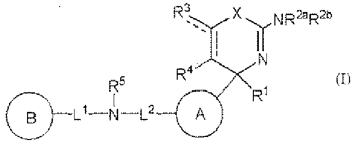 wherein R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group, R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
   X is S or O,
   when X is S, 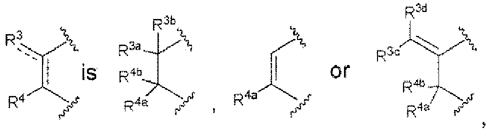 when X is O, 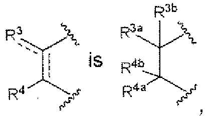 R^{3a}, R^{3b}, R^{4a} and R^{4b} are each independently
   hydrogen, halogen, hydroxy, substituted or unsubstituted alkyl, substituted or unsubstituted alkenyl, substituted or unsubstituted alkynyl, substituted or unsubstituted acyl, substituted or unsubstituted acyloxy, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, carboxy, cyano, nitro, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, a substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
   R^{3a} and R^{3b} together with the carbon atom to which they are attached may form a substituted or unsubstituted carbocycle or a substituted or unsubstituted heterocycle, R^{4a} and R^{4b} together with the carbon atom to which they are attached may form a substituted or unsubstituted carbocycle or a substituted or unsubstituted heterocycle, R^{3c} and R^{3d} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl, or R^{3c} and R^{3d} together with the carbon atom to which they are attached may form a substituted or unsubstituted non-aromatic carbocycle or a substituted or unsubstituted non-aromatic heterocycle,
   R⁵ is hydrogen, substituted or unsubstituted alkyl, substituted or unsubstituted alkenyl, substituted or unsubstituted alkynyl or substituted or unsubstituted acyl,
   L¹ and L² are each independently a bond;
   substituted or unsubstituted alkylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy;
   substituted or unsubstituted alkenylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; or
   substituted or unsubstituted alkynylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; and
   ring A is a substituted or unsubstituted carbocycle or a substituted or unsubstituted heterocycle,
provided that
1) when both of L¹ and L² are a bond, and 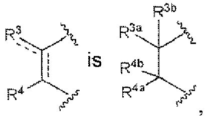 then ring B is a substituted or unsubstituted carbocycle, substituted or unsubstituted pyridine, substituted or unsubstituted pyrimidine or a substituted or unsubstituted 5-membered heterocycle,
2) when both of L¹ and L² are a bond, and 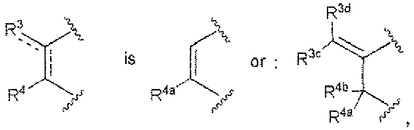 then ring B is a substituted or unsubstituted carbocycle or a substituted or unsubstituted heterocycle, and
3) when at least one of L¹ and L² is substituted or unsubstituted alkylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy;
   substituted or unsubstituted alkenylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonyl imino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; or
   substituted or unsubstituted alkynylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with halogen, alkyl, hydroxy and alkoxy;
   then ring B is substituted nitrogen-containing aromatic monocycle,
   excluding the following compound 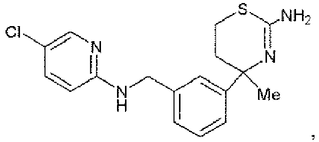
its pharmaceutically acceptable salt, or a solvate thereof.

(1') A compound of formula (I'):

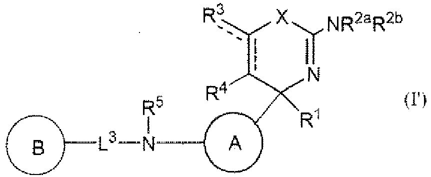

wherein R¹ is substituted or unsubstituted alkyl, substituted or unsubstituted alkenyl, substituted or unsubstituted alkynyl, cyano, a substituted or unsubstituted carbocyclic group or a substituted or unsubstituted heterocyclic group,
R^{2a} and R^{2b} are each independently hydrogen, substituted or unsubstituted alkyl, or substituted or unsubstituted acyl
X is S or O,
when X is S,

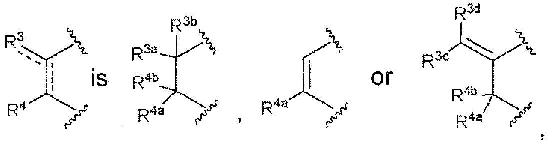

when X is O,

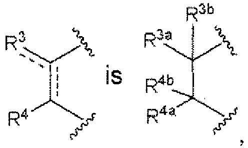

R^{3a} and R^{3b} are each independently
hydrogen, halogen, hydroxy, substituted or unsubstituted alkyl, substituted or unsubstituted alkenyl, substituted or unsubstituted alkynyl, substituted or unsubstituted acyl, substituted or unsubstituted alkoxy, substituted or unsubstituted arylalkyl, substituted or unsubstituted heteroaryalky, substituted or unsubstituted arylalkoxy, substituted or unsubstituted heteroarylalkoxy, substituted or unsubstituted alkylthio, carboxy, cyano, substituted or unsubstituted alkoxycarbonyl, substituted or unsubstituted amino, substituted or unsubstituted carbamoyl, a substituted or unsubstituted carbocyclic group or a substituted or unsubstituted heterocyclic group, R^{3c} and R^{3d} are each independently hydrogen, halogen, substituted or unsubstituted alkyl, or R^{3c} and R^{3d} together with the carbon atom to which they are attached may form a substituted or unsubstituted non-aromatic carbocycle,
R^{4a} and R^{4b} are each independently hydrogen or substituted or unsubstituted alkyl,
R⁵ is hydrogen, substituted or unsubstituted alkyl, substituted or unsubstituted alkenyl, substituted or unsubstituted alkynyl or substituted or unsubstituted acyl,
L³ is a bond, substituted or unsubstituted alkylene wherein the substituent thereof does not include oxo nor thioxo, substituted or unsubstituted alkenylene wherein the substituent thereof does not include oxo nor thioxo, or substituted or unsubstituted alkynylene wherein the substituent thereof does not include oxo nor thioxo, and ring A is a substituted or unsubstituted carbocycle or a substituted or unsubstituted heterocycle,
provided that
1) when L³ is a bond, and

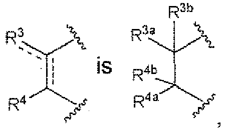

then ring B is a substituted or unsubstituted carbocycle, substituted or unsubstituted pyridine, substituted or unsubstituted pyrimidine or a substituted or unsubstituted 5-membered heterocycle, 2) when L³ is a bond, and

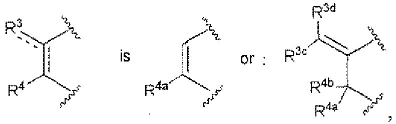

then ring B is a substituted or unsubstituted carbocycle or a substituted or unsubstituted heterocycle, and 3) when L³ is substituted or unsubstituted alkylene, substituted or unsubstituted alkenylene or substituted or unsubstituted alkynylene,
then ring B is substituted nitrogen-containing aromatic monocycle,
its pharmaceutically acceptable salt, or a solvate thereof.

(2) The compound according to the item (1) or (1') wherein both of L¹ and L² are a bond or L³ is a bond, its pharmaceutically acceptable salt or a solvate thereof.

(3) The compound according to the item (1), (1') or (2) wherein ring A is substituted or unsubstituted benzene, ring B is substituted or unsubstituted pyridine or substituted or unsubstituted pyrimidine, its pharmaceutically acceptable salt or a solvate thereof.

(4) The compound according to any one of the items (1), (1'), (2) and (3) wherein 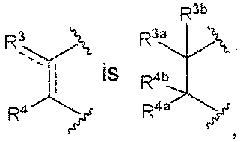 its pharmaceutically acceptable salt or a solvate thereof.

(5)
The compound according to any one of the items (1), (1'),and (2) to (4) wherein R^{3a} is hydrogen, substituted or unsubstituted alkyl, cyano, substituted or unsubstituted alkoxycarbonyl, substituted or unsubstituted carbamoyl, a substituted or unsubstituted carbocyclic group or a substituted or unsubstituted heterocyclic group, R^{3b}, R^{4a} and R^{4b} are hydrogen, its pharmaceutically acceptable salt or a solvate thereof.

(6) The compound according to any one of the items (1), (1') and (2) to (5) wherein X is S, its pharmaceutically acceptable salt or a solvate thereof.

(7) The compound according to any one of the items (1), (1') (2) or (3) wherein 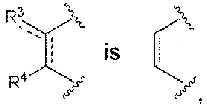 its pharmaceutically acceptable salt or a solvate thereof.

(8) The compound according to any one of the items (1), (1'), and (2) to (7) wherein R¹ is C1 to C3 unsubstituted alkyl, its pharmaceutically acceptable salt or a solvate thereof.

(9) The compound according to any one of the items (1), (1') and (2) to (8) wherein both of R^{2a} and R^{2b} are hydrogen, its pharmaceutically acceptable salt or a solvate thereof.

(10) A pharmaceutical composition comprising the compound according to any one of the items (1), (1') and (2) to (9), its pharmaceutically acceptable salt or a solvate thereof as an active ingredient.

(11) A pharmaceutical composition having BACE1 inhibitory activity comprising the compound according to any one of the items (1), (1') and (2) to (9), its pharmaceutically acceptable salt or a solvate thereof as an active ingredient.

(12) The pharmaceutical composition according to the item (10) or (11), which is a medicament for treating the diseases induced by production, secretion or deposition of amyloid-β proteins.

(13) The pharmaceutical composition according to any one of the items (10) to (12), which is a medicament for treating Alzheimer's disease.

(14) A method for inhibiting BACE1 activity comprising administering the compound according to any one of the items (1), (1') and (2) to (9), its pharmaceutically acceptable salt or a solvate thereof.

(15) The compound according to any one of the items (1), (1') and (2) to (9), its pharmaceutically acceptable salt or a solvate thereof for use in a method for inhibiting BACE1 activity.

(16) A method for treating diseases induced by production, secretion or deposition of amyloid-β proteins comprising administering the compound according to any one of the items (1), (1') and (2) to (9), its pharmaceutically acceptable salt or a solvate thereof.

(17) The compound according to any one of the items (1), (1'), (2) to (9), its pharmaceutically acceptable salt or a solvate thereof for use in a method for treating diseases induced by production, secretion or deposition of amyloid-β proteins.

(18) A method for treating Alzheimer's disease comprising administering the compound according to any one of the items (1), (1') and (2) to (9), its pharmaceutically acceptable salt or a solvate thereof.

(19) The compound according to any one of the items (1), (1') and (2) to (9), its pharmaceutically acceptable salt or a solvate thereof for use in a method for treating Alzheimer's disease.

(20) Use of the compound according to any one of the item (1), (1') and (2) to (9), or its pharmaceutically acceptable salt; or a solvate thereof in the manufacture of a medicament for inhibiting β secretase activity,

(21) Use of the compound according to any one of the item (1), (1') and (2) to (9), or its pharmaceutically acceptable salt; or a solvate thereof in the manufacture of a medicament for treating disease induced by production, secretion or deposition of amyloid β protein,

(22) A method, system, equipment, kit or the like for preparing the compound according to any one of the item (1), (1') and (2) to (9), or its pharmaceutically acceptable salt; or a solvate thereof,

(23) A method, system, equipment, kit or the like for preparing the pharmaceutical composition comprising the compound according to any one of the item (1), (1') and (2) to (9), or its pharmaceutically acceptable salt; or a solvate thereof,

### [Effect of the Invention]

The compounds of the present invention are useful as an agent for treating disease induced by production, secretion or deposition of amyloid β protein (Alzheimer's disease and the like).

### [Best Mode for Carrying Out the Invention]

In the specification, the "halogen" includes fluorine, chlorine, bromine, and iodine.

In the specification, the "alkyl" includes straight or branched alkyl of a carbon number of 1 to 15, for example, a carbon number of 1 to 10, for example, a carbon number of 1 to 6, and for example, a carbon number of 1 to 3. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decyl.

In the specification, an alkyl part of the "alkoxy", the "halogenoalkyl", the "hydroxyalkyl", the "halogenoalkoxy", the "hydroxyalkoxy", the "alkoxycarbonyl", the "halogenoalkoxycarbonyl", the "alkoxycarbonylalkyl", the "alkylamino", the "alkoxyalkyl", the "hydroxyiminoalkyl", the "alkoxyiminoalkyl,", the "aminoalkyl", the "alkoxyalkoxy", the "alkoxyalkenyl", the "alkoxyalkenyloxy", the "alkoxycarbonylalkenyl", the "alkoxyalkynyl", the "alkoxycarbonylalkynyl", the "alkylcarbamoyl", the "hydroxyalkylcarbamoyl", the "alkoxyimino", the "alkylthio", the "alkylsulfonyl", the "alkylsulfonyloxy", the "alkylsulfonylamino", the "alkylsulfonylalkylamino", the "alkylsulfonylimino", the "alkylsulfinylamino", the "alkylsulfinylalkylamino", the "alkylsulfinylimino", the "alkylsulfamoyl", the "alkylsulfinyl", the "carbocyclylalkyl", the "carbocyclylalkoxy", the "carbocyclylalkoxycarbonyl", the "carbocyclylalkylamino", the "carbocyclylalkylcarbamoyl", the "cycloalkylalkyl", the "cycloalkylalkoxy", the "cycloalkylalkylamino", the "cycloalkylalkoxycarbonyl", the "cycloalkylalkylcarbamoyl", the "arylalkyl", the "arylalkoxy", the "arylalkylamino", the "arylalkoxycarbonyl", the "arylalkylcarbamoyl", the "heterocyclylalkyl", the "heterocyclylalkoxy", the "heterocyclylalkylamino", the "heterocyclylalkoxycarbonyl", the "heterocyclylalkylcarbamoyl", the "heteroarylalkyl", and the "heteroarylalkoxy" is the same as the above "alkyl".

In the specification, the "substituted or unsubstituted alkyl" may be substituted with one or more substituents selected from a substituent group α.

As used herein, the substituent group α is a group consisting of halogen, hydroxy, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein the carbocycle and the heterocycle may be each substituted with one or more substituent(s) selected from a group consisting of halogen, alkyl, hydroxy and alkoxy.

In the specification, examples of the substituent of the "substituted or un substituted alkoxy", the "substituted or un substituted alkoxycarbonyl", the "substituted or un substituted alkylthio", the "substituted or un substituted alkylsulfinyl" and the "substituted or un substituted alkylsulfonyl" include one or more groups selected from the above substituent group α.

In the specification, examples of an embodiment of the "halogenoalkyl" include trifluoromethyl, fluoromethyl and trichloromethyl.

In the specification, the "alkylidene" includes a divalent group of the above "alkyl", and examples include methylidene, ethylidene, propylidene, isopropylidene, butylidene, pentylidene and hexylidene.

The "alkenyl" includes straight or branched alkenyl of a carbon number of 2 to 15, for example, a carbon number of 2 to 10, for example, a carbon number of 2 to 6, and for example, a carbon number of 2 to 4, having one or more double bonds at any available position. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.

The "alkynyl" includes straight or branched alkynyl of a carbon number of 2 to 10, for example, a carbon number of 2 to 8, and for example, a carbon number of 3 to 6, having one or more triple bonds at any available position. Examples include ethynyl, propynyl, butynyl, pentynyl, hexynyl, heptynyl, octynyl, nonynyl and decynyl. These may have further a double bond at any available position.

Examples of the substituent of the "substituted or unsubstituted alkenyl", the "substituted or unsubstituted alkenyloxycarbony", the "substituted or unsubstituted alkenyloxy", the "substituted or unsubstituted alkenylthio", the "substituted or unsubstituted alkenylsulfinyl", the "substituted or unsubstituted alkenylsulfonyl", the "substituted or unsubstituted alkynyl", the "substituted or unsubstituted alkynyloxycarbony", the "substituted or unsubstituted alkynyloxy", the "substituted or unsubstituted alkynylthio", the "substituted or unsubstituted alkynylsulfinyl" and the "substituted or unsubstituted alkynylsulfonyl" include one or more substituents selected from the above substituent group α.

An alkenyl part of the "hydroxyalkenyl", the "alkoxyalkenyl", the "alkoxycarbonylalkenyl", the "carbocyclylalkenyl", the "alkenyloxy", the "alkenyloxycarbonyl", the "alkoxylalkenyloxy", the "alkenylthio", the "alkenylsulfinyl", the "alkenylsulfonyl" and the "alkenylamino" is the same as the above "alkenyl".

An alkynyl part of the "hydroxyalkynyl", the "alkoxyalkynyl", the "alkoxycarbonylalkynyl", the "carbocyclylalkynyl", the "alkynyloxy", the "alkynyloxycarbonyl", the "alkoxyalkynyloxy", the "alkynylthio", the "alkynylsulfinyl", the "alkynylsulfonyl" and the "alkynylamino" is the same as the above "alkynyl".

Examples of the substituent of the "substituted or unsubstituted amino", the "substituted or unsubstituted carbamoyl", the "substituted or unsubstituted thiocarbamoyl" and the "substituted or unsubstituted sulfamoyl" include 1 or 2 substituents selected from alkyl, acyl, hydroxy, alkoxy, alkoxycarbonyl, a carbocyclic group and a heterocyclic group.

The "acyl" includes aliphatic acyl, carbocyclylcarbonyl and heterocyclylcarbonyl of a carbon number of 1 to 10. Examples include formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, piperidinecarbonyl and thiomorpholino.

An acyl part of the "acyloxy "is the same as the above "acyl".

Examples of the substituent of the "substituted or unsubstituted acyl" and the "substituted or unsubstituted acyloxy" include one or more substituents selected from the substituent group α. In addition, a ring part of the carbocyclylcarbonyl and the heterocyclylcarbonyl may be substituted with one or more substituents selected from alkyl, the substituent group α, and alkyl substituted with one or more groups selected from the substituent group α.

In the specification, the "carbocyclic group" includes cycloalkyl, cycloalkenyl, aryl and a non-aromatic fused carbocyclic group.

In the specification, the "cycloalkyl" is a carbocyclic group of a carbon number of 3 to 10, for example, a carbon number of 3 to 8, and for example, a carbon number of 4 to 8. Examples include cyclopropyl, cyclobutyl, cyclopentyl, cyclohexyl, cycloheptyl, cyclooctyl, cyclononyl and cyclodecyl.

In the specification, a cycloalkyl part of the "cycloalkylalkyl", the "cycloalkyloxy", the "cycloalkylalkoxy", the "cycloalkylthio", the "cycloalkylamino", the "cycloalkylalkylamino", the "cycloalkylsulfamoyl", the "cycloalkylsulfonyl", the "cycloalkylcarbamoyl", the "cycloalkylalkylcarbamoyl", the "cycloalkylalkoxycarbonyl", and the "cycloalkyloxycarbonyl" is the same as the above "cycloalkyl".

In the specification, the "cycloalkenyl" includes the cycloalkyl having one or more double bonds at any available position in the ring, and examples include cyclopropenyl, cyclobutenyl, cyclopentenyl, cyclohexenyl, cycloheptynyl, cyclooctynyl and cyclohexadienyl.

In the specification, the "aryl" includes phenyl, naphthyl, anthryl and phenanthryl, and specific example is phenyl.

In the specification, the "non-aromatic fused carbocyclic group" includes a non-aromatic group in which two or more cyclic groups selected from the above "cycloalkyl", the above "cycloalkenyl" and the above "aryl" are fused, and examples include indanyl, indenyl, tetrahydronaphthyl and fluorenyl.

In the specification, the "together with the carbon atom to which they are attached may form a substituted or unsubstituted carbocycle" refers to that two substituents are taken together to form the "carbocycle".
In the specification, the "together with the carbon atom to which they are attached may form a substituted or unsubstituted non-aromatic carbocycle" refers to that two substituents are taken together to form the "cycloalkyl" or the "cycloalkenyl".

For example, 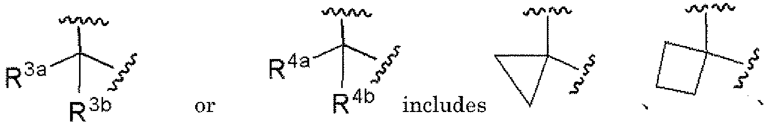 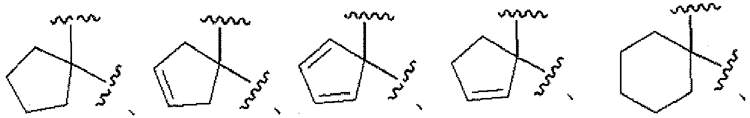 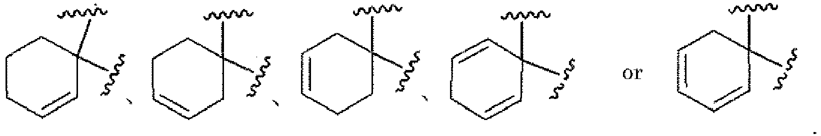 These rings may be substituted at any position(s) with one or more substituent(s) selected from the group consisting of alkyl substituted with the substituent group α, unsubstituted alkyl and the substituent group α.

In the specification, a carbocyclyl part of the "carbocycle", the "carbocyclylalkyl", the "carbocyclylalkenyl", the "carbocyclylalkynyl", the "carbocyclylalkoxy", the "carbocyclylalkoxycarbonyl", the "carbocyclyloxy", the "carbocyclylthio", the "carbocyclylamino", the "carbocyclylalkylamino", the "carbocyclylcarbonyl", the "carbocyclylsulfamoyl", the "carbocyclylsulfinyl", the "carbocyclylsulfonyl", the "carbocyclylcarbamoyl", the "carbocyclylalkylcarbamoyl" and the "carbocyclyloxycarbonyl" is the same as the "carbocyclic group".

In the specification, an aryl part of the "arylalkyl", the "aryloxy", the "aryloxycarbonyl", the "arylalkoxycarbonyl", the "arylthio", the "arylamino", the "arylalkoxy", the "arylalkylamino", the "arylsulfonyl", the "arylsulfamoyl", the "arylcarbamoyl" and the "arylalkylcarbamoyl" is the same as the "aryl".

In the specification, the "heterocyclic group" includes a heterocyclic group having one or more hetero atoms optionally selected from O, S and N in a ring, and examples include 5- to 6-membered heteroaryl such as pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl and thiadiazolyl; non-aromatic heterocyclic groups such as dioxanyl, thiiranyl, oxyranyl, oxetanyl, oxathioranyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydroazepinyl, tetrahydrodiazepinyl and tetrahydropyridazinyl; dicyclic fused heterocyclic groups such as indolyl, isoindolyl, indazolyl, indolidinyl, indolinyl, isoindolinyl, quinolyl, isoquinolyl, cinnolinyl, phthalazinyl, quinazolinyl, naphthrydinyl, quinoxalinyl, purinyl, pteridinyl, benzopyranyl, benzimidazolyl, benzotriazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, thienopyridyl, thienopyrrolyl, thienopyrazolyl, thienopyrazinyl, furopyrrolyl, thienothienyl, imidazopyridyl, imidazopyrazolyl, pyrazolopyridyl, pyrazolopyrazinyl, thiazolopyridyl, pyrazolopyrimidinyl, pyrazolotrianidyl, pyridazolopyridyl, triazolopyridyl, imidazothiazolyl, pyrazinopyridadinyl, dihydrothiazolopyrimidinyl, tetrahydroquinolyl, tetrahydroisoquinolyl, dihydrobenzofuryl, dihydrobenzoxazinyl, dihydrobenzimidazolyl, tetrahydrobenzothienyl, tetrahydrobenzofuryl, benzodioxolyl, benzodioxonyl, chromanyl, chromenyl, octahydrochromenyl, dihydrobenzodioxynyl, dihydrobenzoxezinyl, dihydrobenzodioxepynyl and dihydrothienodioxynyl; tricyclic fused heterocyclic groups such as carbazolyl, acrydinyl, xanthenyl, phenothiadinyl, phenoxathiinyl, phenoxadinyl, dibenzofuryl, imidazoquinolyl and tetrahydrocarbazolyl. Specific example is a 5- to 6-membered heteroaryl or a non-aromatic heterocyclic group.

In the specification, a heterocyclyl part of the "hetorocycle", the "heterocyclylalkyl", the "heterocyclyloxy", the "heterocyclylthio", the "heterocyclycarbonyl", the "heterocyclylalkoxy", the "heterocyclylamino", the "heterocyclylsulfamoyl", the "heterocyclylsulfinyl", the "heterocyclylsulfonyl", the "heterocyclylcarbamoyl", the "heterocyclyloxycarbonyl", the "heterocyclylalkylamino", the "heterocyclylalkoxycarbonyl" and the "heterocyclylalkylcarbamoyl" is the same as the "heterocyclic group".

A bond of the above "heterocyclic group" may be situated on any ring.

In the specification, the "heteroaryl" includes an aromatic cyclic group among the "heterocyclic group". A heteroaryl part of the "heteroarylalkyl" and the "heteroarylalkoxy" is the same.

In the specification, "non-aromatic heterocycle" includes non-aromatic ring derived from the above "heterocyclic group" a heterocycle part of which is non-aromatic. Examples include dioxane, thiirane, oxyrane, oxetane, oxathiorane, azetidine, thiane, thiazolidine, pyrrolidine, pyrroline, imidazolidine, imidazoline, pyrazolidine, pyrazoline, piperidine, piperazine, morpholine, thiomorpholine, dihydropyridine, tetrahydropyridine, tetrahydrofuran, tetrahydropyran, dihydrothiazole, tetrahydrothiazole, tetrahydroisothiazole, dihydrooxazine, hexahydroazepine, tetrahydrodiazepine and tetrahydropyridazine.

In the specification, the "together with the carbon atom to which they are attached may form a substituted or unsubstituted heterocycle" refers to that two substituents are taken together to form the "heterocycle".
In the specification, the "together with the carbon atom to which they are attached may form a substituted or unsubstituted non-aromatic heterocycle" refers to that two substituents are taken together to form the "non-aromatic heterocycle".

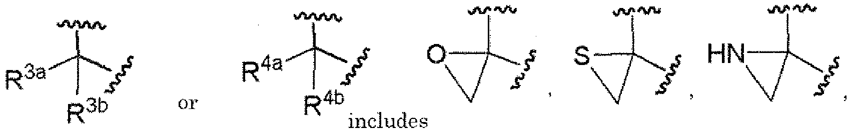

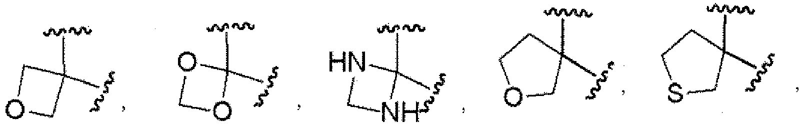

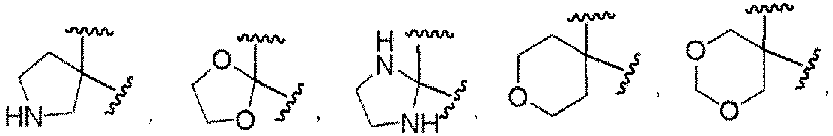

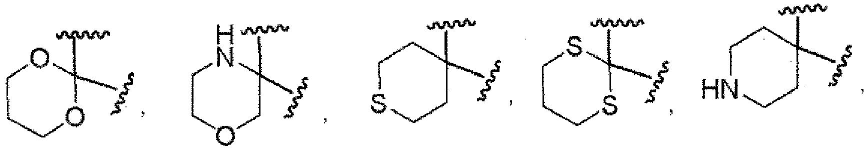

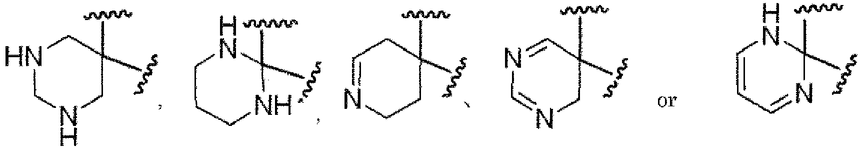

These rings may be substituted at any position(s) with one or more substituent(s) selected from the group consisting of alkyl substituted with the substituent group α, unsubstituted alkyl and the substituent group α.

In the specification, "5-membered heterocyle" includes 5-membered ring derived from the "heterocyclic group" a heterocycle part of which is 5-membered. Examples include pyrrole, imidazole, pyrazole, tetrazole, furan, thiophen, isoxazole, oxazole, oxadiazole, isothiazole, thiazole, thiadiazole, thiazolidine, pyrrolidine, pyrroline, imidazolidine, imidazoline, pyrazolidine, pyrazoline, tetrahydrofuran, dihydrothiazole, tetrahydrothiazole, tetrahydroisothiazole and the like.

In the specification, "nitrogen-containing aromatic monocycle" includes monocyclic heterocycle containing at least one nitrogen atom(s) in the ring derived from the above "heterocyclic group". Examples include pyrrole, imidazole, pyrazole, pyridine, pyridazine, pyrimidine, pyrazine, triazole, triazine, tetrazole, isoxazole, oxazole, oxadiazole, isothiazole, thiazole, thiadiazole, azetidine, thiazolidine, pyrrolidine, pyrroline, imidazolidine, imidazoline, pyrazolidine, pyrazoline, piperidine, piperazine, morpholine, thiomorpholine, dihydropyridine, tetrahydropyridine, dihydrothiazole, tetrahydrothiazole, tetrahydroisothiazole, tetrahydropyridazine and the like.

In the specification, examples of the substituent of the "substituted or unsubstituted carbocycle", the "substituted or unsubstituted benzene", the "substituted or unsubstituted heterocycle", the "substituted or unsubstituted pyridine" , the "substituted or unsubstituted pyrimidine" , the "substituted or unsubstituted 5-membered heterocycle" and "substituted nitrogen-containing aromatic monocycle" in ring A and ring B include:
a substituent selected from the substituent group α, for example, halogen, hydroxy, acyl, acyloxy, carboxy, alkoxycarbonyl, carbamoyl, amino, cyano, alkylamino and/or alkylthio etc.;,
alkyl substituted with one or more substituents selected from the group consisting of the substituent group α, hydroxyimino and alkoxyimino, wherein the substituent is, for example, halogen, hydroxy, alkoxy and/or alkoxycarbonyl etc. or unsubstituted alkyl;
aminoalkyl substituted with one or more groups selected from the substituent group α; wherein the substituent is, for example, acyl, alkyl and/or alkoxy etc.;
alkenyl substituted with one or more substituents selected from the substituent group α, wherein the substituent is, for example, alkoxycarbonyl, halogen and/or
halogenoalkoxycarbonyl etc. or unsubstituted alkenyl;
alkynyl substituted with one or more substituents selected from the substituent group α, wherein the substituent is, for example, alkoxycarbonyl etc. or unsubstituted alkynyl;
alkoxy substituted with one or more substituents selected from the substituent group α, wherein the substituent is, for example, halogen, carbamoyl, alkylcarbamoyl and/or hydroxyalkylcarbamoyl etc. or unsubstituted alkoxy;
alkoxyalkoxy, substituted with one or more substituents selected from the substituent group α;
alkenyloxy substituted with one or more substituents selected from the substituent group α, wherein the substituent is, for example, halogen, hydroxy, amino and/or
alkylamino etc. or unsubstituted alkenyloxy;
alkoxyalkenyloxy substituted with one or more substituents selected from the substituent group α or unsubstituted alkoxyalkenyloxy;
alkynyloxy substituted with one or more substituents selected from the substituent group α,wherein the substituent is, for example, halogen and/or hydroxy etc. or unsubstituted alkynyloxy;
alkoxyalkynyloxy substituted with one or more groups selected from the substituent group α;
alkylthio substituted with one or more substituents selected from the substituent group α or unsubstituted alkylthio;
alkenylthio substituted with one or more substituents selected from the substituent group α or unsubstituted alkenylthio;
alkynylthio substituted with one or more substituents selected from the substituent group α or unsubstituted alkynylthio;
alkylamino substituted with one or more substituents selected from the substituent group α;
alkenylamino substituted with one or more substituents selected from the substituent group α;
alkynylamino substituted with one or more substituents selected from the substituent group α;
aminooxy substituted with one or more substituents selected from the substituent group α and alkylydene, or unsubstituted aminooxy;
acyl substituted with one or more substituents selected from the substituent group α; alkylsulfonyl substituted with one or more substituents selected from the substituent group α or unsubstituted alkylsulfonyl;
alkylsulfinyl substituted with one or more substituents selected from the substituent group α or unsubstituted alkylsulfinyl;
alkylsulfamoyl substituted with one or more substituents selected from the substituent group α or unsubstituted alkylsulfamoyl;
carbocyclic group, e.g. cycloalkyl, aryl and the like, substituted with one or more substituents selected from the group consisting of the substituent group α, azide, alkyl and halogenoalkyl, or unsubstituted carbocyclic group, e.g. cycloalkyl, aryl and the like; heterocyclic group substituted with one or more substituents selected from the group consisting of the substituent group α, azide, alkyl and halogenoalkyl, or unsubstituted heterocyclic group;
carbocyclylalkyl, e.g. cycloalkylalkyl, arylalkyl and the like, substituted with one or more substituents selected from the group consisting of the substituent group α, azide, alkyl and halogenoalkyl, or unsubstituted carbocyclylalkyl;
heterocyclylalkyl substituted with one or more substituents selected from the group consisting of the substituent group α, azide, alkyl and halogenoalkyl, or unsubstituted heterocyclylalkyl;
carbocyclyloxy, e.g. cycloalkoxy, aryloxy and the like, substituted with one or more substituents selected from the group consisting of the substituent group α, azide, alkyl and halogenoalkyl, or unsubstituted carbocyclyloxy, e.g. cycloalkylalkyl, arylalkyl and the like;
heterocyclyloxy substituted with one or more substituents selected from the group consisting of the substituent group α, azide, alkyl and halogenoalkyl, or unsubstituted heterocyclyloxy;
carbocyclylalkoxy, e.g. cycloalkylalkoxy, arylalkoxy and the like, substituted with one or more substituents selected from the group consisting of the substituent group α, azide, alkyl and halogenoalkyl, or unsubstituted carbocyclylalkoxy, e.g. cycloalkylalkoxy, arylalkoxy and the like;
heterocyclylalkoxy substituted with one or more substituents selected from the group consisting of the substituent group α, azide, alkyl and halogenoalkyl, or unsubstituted heterocyclylalkoxy;
carbocyclylalkoxycarbonyl, e.g. cycloalkylalkoxycarbonyl, arylalkoxycarbonyl and the like, substituted with one or more substituents selected from the group consisting of the substituent group α, azide, alkyl and halogenoalkyl, or unsubstituted carbocyclylalkoxycarbonyl, e.g. cycloalkylalkoxycarbonyl, arylalkoxycarbonyl and the like;
heterocyclylalkoxycarbonyl substituted with one or more substituents selected from the group consisting of the substituent group α, azide, alkyl and halogenoalkyl, or unsubstituted heterocyclylalkoxycarbonyl;
carbocyclylthio, e.g. cycloalkylthio, arylthio and the like, substituted with one or more substituents selected from the group consisting of the substituent group α, azide, alkyl and halogenoalkyl, or unsubstituted carbocyclylthio, e.g. cycloalkylthio, arylthio and the like;
heterocyclylthio substituted with one or more substituents selected from the group consisting of the substituent group α, azide, alkyl and halogenoalkyl, or unsubstituted heterocyclylthio;
carbocyclylamino, e.g. cycloalkylamino, arylamino and the like, substituted with one or more substituents selected from the group consisting of the substituent group α, azide, alkyl and halogenoalkyl, or unsubstituted carbocyclylamino, e.g. cycloalkylamino, arylamino and the like;
heterocyclylamino substituted with one or more substituents selected from the group consisting of the substituent group α, azide, alkyl and halogenoalkyl, or unsubstituted heterocyclylamino;
carbocyclylalkylamino, e.g. cycloalkylalkylamino, arylalkylamino and the like, substituted with one or more substituents selected from the group consisting of the substituent group α, azide, alkyl and halogenoalkyl, or unsubstituted carbocyclylalkylamino, e.g. cycloalkylalkylamino, arylalkylamino and the like;
heterocyclylalkylamino substituted with one or more substituents selected from the group consisting of the substituent group α, azide, alkyl and halogenoalkyl, or unsubstituted heterocyclylalkylamino;
carbocyclylsulfamoyl, e.g. cycloalkylsulfamoyl, arylsulfamoyl and the like, substituted with one or more substituents selected from the group consisting of the substituent group α, azide, alkyl and halogenoalkyl, or unsubstituted carbocyclylsulfamoyl, e.g. cycloalkylsulfamoyl, arylsulfamoyl and the like;
heterocyclylsulfamoyl substituted with one or more substituents selected from the group consisting of the substituent group α, azide, alkyl and halogenoalkyl, or unsubstituted heterocyclylsulfamoyl;
carbocyclylsulfonyl, e.g. cycloalkylsulfonyl, arylsulfonyl and the like, substituted with one or more substituents selected from the group consisting of the substituent group α, azide, alkyl and halogenoalkyl, or unsubstituted carbocyclylsulfonyl, e.g. cycloalkylsulfonyl, arylsulfonyl and the like;
heterocyclylsulfonyl substituted with one or more substituents selected from the group consisting of the substituent group α, azide, alkyl and halogenoalkyl, or unsubstituted heterocyclylsulfonyl;
carbocyclylcarbamoyl, e.g. cycloalkylcarbamoyl, arylcarbamoyl and the like, substituted with one or more substituents selected from the group consisting of the substituent group α, azide, alkyl and halogenoalkyl, or unsubstituted carbocyclylcarbamoyl, e.g. cycloalkylcarbamoyl, arylcarbamoyl and the like;
heterocyclylcarbamoyl substituted with one or more substituents selected from the group consisting of the substituent group α, azide, alkyl and halogenoalkyl, or unsubstituted heterocyclylcarbamoyl;
carbocyclylalkylcarbamoyl, e.g. cycloalkylalkylcarbamoyl, arylalkylcarbamoyl and the like, substituted with one or more substituents selected from the group consisting of the substituent group α, azide, alkyl and halogenoalkyl, or unsubstituted carbocyclylalkylcarbamoyl, e.g. cycloalkylalkylcarbamoyl, arylalkylcarbamoyl and the like;
heterocyclylalkylcarbamoyl substituted with one or more substituents selected from the group consisting of the substituent group α, azide, alkyl and halogenoalkyl, or unsubstituted heterocyclylalkylcarbamoyl;
carbocyclyloxycarbonyl, e.g. cycloalkyloxycarbonyl, aryloxycarbonyl and the like, substituted with one or more substituents selected from the group consisting of the substituent group α, azide, alkyl and halogenoalkyl, or unsubstituted carbocyclyloxycarbonyl, e.g. cycloalkoxycarbonyl, aryloxycarbonyl and the like;
heterocyclyloxycarbonyl substituted with one or more substituents selected from the group consisting of the substituent group α, azide, alkyl and halogenoalkyl, or unsubstituted heterocyclyloxycarbonyl;
alkylenedioxy substituted with halogen, or unsubstituted alkylenedioxy;
oxo; and azide. The ring may be substituted with one or more substituents selected from them.

In the specification, examples of the substituent of "substituted or unsubstituted carbocycle", "substituted or unsubstituted benzene", "substituted or unsubstituted heterocycle", "substituted or unsubstituted pyridine", "substituted or unsubstituted pyrimidine", "substituted or unsubstituted 5-membered heterocycle" and "substituted nitrogen-containing aromatic heteromonocycle", of ring A or ring B include halogen, cyano, hydroxy, nitro, carboxy, alkyl substituted with one or more substituent(s) selected from the substituent group α, unsubstituted alkyl, alkoxy substituted with one or more substituent(s) selected from the substituent group α, unsubstituted alkoxy, amino substituted with one or more substituent(s) selected from the substituent group α, unsubstituted amino, carbamoyl substituted with one or more substituent(s) selected from the substituent group α, unsubstituted carbamoyl, alkoxycarbonyl substituted with one or more substituent(s) selected from the substituent group α, unsubstituted alkoxycarbonyl.

In the specification, examples of the substituent of "substituted or unsubstituted carbocyclic group", "substituted or unsubstituted carbocycle", "substituted or unsubstituted carbocyclyloxy", "substituted or unsubstituted carbocyclylthio", "substituted or unsubstituted carbocyclylalkyl", "substituted or unsubstituted carbocyclylalkoxy", "substituted or unsubstituted carbocyclyloxycarbonyl", "substituted or unsubstituted carbocyclylsulfinyl", "substituted or unsubstituted carbocyclylsulfonyl" "substituted or unsubstituted heterocyclic group", "substituted or unsubstituted heterocycle", "substituted or unsubstituted heterocyclyloxy", "substituted or unsubstituted heterocyclylthio", "substituted or unsubstituted heterocyclylalkyl", "substituted or unsubstituted heterocyclylalkoxy", "substituted or unsubstituted heterocyclyloxycarbonyl", "substituted or unsubstituted heterocyclylsulfinyl", "substituted or unsubstituted heterocyclylsulfonyl", "substituted or unsubstituted non-aromatic carbocycle" or "substituted or unsubstituted non-aromatic heterocycle", when these are located other than ring A or ring B, include one or more substituent(s) selected from the group consisting of alkyl substituted with one or more substituent(s) selected from the substituent group α, unsubstituted alkyl and the substituent group α.

In the specification, "alkylene" includes a straight or branched divalent carbon chain of a carbon number of 1 to 10, for example, a carbon number of 1 to 6, for example, a carbon number of 1 to 3. Examples include methylene, dimethylene, trimethylene, tetramethylene, and methyltrimethylene.

In the specification, a alkylene part of the "alkylenedioxy" is the same as the "alkylene".

In the specification, the "alkenylene" includes a straight or branched divalent carbon chain of a carbon number of 2 to 10, for example, a carbon number of 2 to 6,for example, a carbon number of 2 to 4, having a double bond at any available position. Examples include vinylene, propenylene, butenylene, butadienylene, methylpropenylene, pentenylene and hexenylene.

In the specification, the "alkynylene" includes a straight or branched divalent carbon chain of a carbon number of 2 to 10, for example, a carbon number of 2 to 6, for example, a carbon number of 2 to 4, having a triple bond at any available position and, further, optionally having a double bond. Examples include ethynylene, propynylene, butynylene, pentynylene and hexynylene.

Examples of the substituent of the "substituted or unsubstituted alkylene", the "substituted or unsubstituted alkenylene" and the "substituted or unsubstituted alkynylene" include (a) substituent(s) selected from a group consisting of halogen, alkoxy, halogenoalkoxy, hydroxyalkoxy alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein the carbocycle and the heterocycle may be each substituted with one or more substituent(s) selected from a group consisting of halogen, alkyl, hydroxy and alkoxy. Examples include halogen.

In the specification, the term "solvate" includes, for example, solvates with organic solvents and hydrates. It can be prepared in accordance with the known methods. Examples of solvate include a solvate with acetone, 2-butanol, 2-propanol, ethanol, ethyl acetate, tetrahydrofuran or diethylether. For example, it includes a non-toxic and water-soluble hydrate or solvate such as a solvate with ethanol. In the case that a hydrate or solvate is formed, the compound or salt may be coordinated with any number of solvate molecules or water molecules.

The compound represented by the formula (I) and (I') includes a pharmaceutically acceptable salt. Examples include salts with alkali metals such as lithium, sodium or potassium; alkaline earth metals such as calcium; magnesium; transition metals such as zinc or iron; ammonium; organic bases; and amino acids; or salts with inorganic acids such as hydrochloric acid, sulfuric acid, nitric acid, hydrobromic acid, phosphoric acid or hydroiodic acid; and organic acids such as acetic acid, trifluoroacetic acid, citric acid, lactic acid, tartaric acid, oxalic acid, maleic acid, fumaric acid, mandelic acid, glutaric acid, malic acid, benzoic acid, phthalic acid, benzenesulfonic acid, p-toluenesulfonic acid, methanesulfonic acid or ethane sulfonic acid. Specific examples are hydrochloric acid, phosphoric acid, tartaric acid and methanesulfonic acid. These salts can be formed by ordinary methods.

In addition, the compound represented by the formula (I) and (I') is not limited to a specific isomer, but includes all possible isomers, such as keto-enol isomers, imine-enamine isomers, diastereoisomers, optical isomers and rotation isomers; and racemate. For example, the compound represented by the formula (I) in which R^{2a} is hydrogen includes the following tautomers.

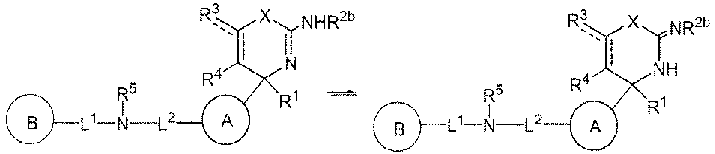

The compound of the formula (I) and (I') has an asymmetric carbon atom and includes any optical isomers described below. 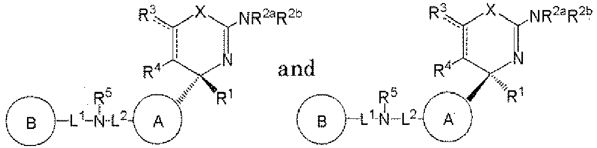 Preferable is

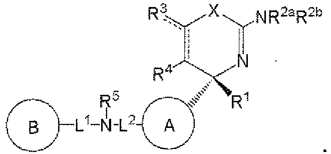

The compound of the formula (I') also includes tautomer as described above.

In addition, one or more hydrogen, carbon or other atoms of the compound of formula (I) and (I') can be replaced by an isotope of the hydrogen, carbon or other atoms. Compounds of formula (I) and (I') include all radiolabeled forms of compounds of formula (I) and (I'). The "radiolabeled," "radiolabeled form" and the like of the compound of formula (I) and (I') are encompassed by the present invention and useful as a research and/or diagnostic tool in metabolism pharmacokinetic studies and in binding assays. It is also useful for a medicament.

Examples of isotopes that can be incorporated into the compound of formula (I) of the invention include isotopes of hydrogen, carbon, nitrogen, oxygen, phosphorous, sulfur, fluorine, iodine and chlorine, such as ²H, ³H, ¹¹C, ¹³C, ¹⁴C, ¹⁵N, ¹⁸O, ¹⁷O, ³¹P, ³²P, ^{B5}S, ¹⁸F, ¹²³I and ³⁶Cl, respectively. Radiolabeled compounds of the invention can be prepared by methods known in the art. For example, tritiated compounds of formula (I) can be prepared by introducing tritium into the particular compound of formula (I), for example, by catalytic dehalogenation with tritium. This method may include reacting a suitably halogen-substituted precursor of a compound of formula (I) with tritium gas in the presence of a suitable catalyst such as Pd/C, in the presence or absence of a base. Other suitable methods for preparing tritiated compounds can be found in Isotopes in the Physical and Biomedical Sciences, Vol. 1, Labeled Compounds (Part A) Chapter 6, (1987). ¹⁴C-labeled compounds can be prepared by employing starting materials having a ¹⁴C carbon.

For example, the compound of the formula (I) and (I') can be prepared in accordance with Patent Literature 1, Journal of heterocyclic chemistry, 14, 717-723 (1977) or the method described below.

In the following all steps, when a substituent which impedes a reaction, e.g. hydroxy, mercapto, amino, formyl, carbonyl, carboxy, is possessed, the substituent is protected by the method described in Protective Groups in organic Synthesis, and Theodora W Greene (John Wiley & Sons) in advance, and the protecting group may be removed at a desirable stage.

In addition, in the all steps, an order of steps to be implemented may be appropriately changed, and each intermediate may be isolated, and used in a next step.

### (General Preparation)

### A. Preparation of a compound represented by the formula (I)

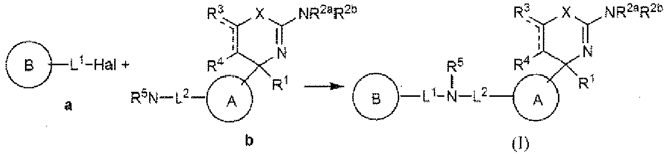

wherein Hal is halogen and the other symbols are as defined above.
The compounds of the general formula (a) are commercially available products or prepared by publicly known method (Tetrahedron, 2009, vol. 65, 757 - 764 pages) and the corresponding method thereof. The compounds of the general formula (b), for example, can be prepared by the method described in Patent Literature 1 (WO2007/049532) Patent Literature 2 (WO2008/133274 and Patent Literature 3 (WO2008/133273 or by general methods described below.

### Method A: Condensation under acidic condition

The compound of the general formula (I) can be prepared by reacting a compound (a) and a compound (b) with an acid such as hydrochloric acid, sulfuric acid, trifluoroacetic acid, methanesulfonic acid, trifluoromethanesulfonic acid, perchloric acid in a solution such as methanol, ethanol, isopropyl alcohol, butanol, isobutanol, sec-butanol, acetic acid, water or the mixed solution thereof at 0°C to 180°C, preferably 20°C to 140°C for 0,1 hour to 120 hours, preferably 0.5 hour to 72 hours.

### Method B: Condensation under basic condition

The compound of the general formula (I) can be prepared by reacting a compound (a) and a compound (b) with a base such as triethylamine, sodium carbonate, potassium carbonate, cesium carbonate, sodium methoxide, potassium tert-butoxide, n-butyllithium, lithium hexamethyldisilazide, sodium hexamethyldisilazide and potassium hexamethyldisilazide in a solution such as toluene, tetrahydrofuran, dimethylformamide, 1,2-dimethoxyethane, 1, 4-dioxane, methanol at 0°C to 180°C, preferably 20°C to 140°C for 0.5 hour to 120 hours, preferably 0.5 hour to 72 hours.

The reaction can be performed in the presence of tris(dibenzylideneacetone) dipalladium, palladium acetate or palladium (0) prepared in situ or the like and a phosphine ligand such as triphenylphosphine, tritert-butylphosphine, dicyclohexylbiphenylphosphine, 9,9-dimethyl-4,6-bis(diphenylphosphino)xanthene (Xantphos), 2-dicyclohexylphosphino-2,4',6'-triisopropylbiphenyl (X-Phos), 2-dicyclobexylphosphino-2', 6 '-diisopropoxy-1,1'-biphenyl (Ruphos). In the above method, the compounds of the general formula (I) can be prepared by the reaction at 0°C to 150°C, preferably 10°C to 100°C for 0.5 hour to 72 hours, preferably 1 hour to 24 hours with microwave irradiation or without microwave irradiation.

### B. Preparation of a compound represented by the formula (b)

The compounds of the formula (b) can be prepared in accordance with the methods for preparing the compounds b-1, b-2, b-3, b-4, b-5 or b-6, as shown below.

### B-1) Preparation of Compound b-1

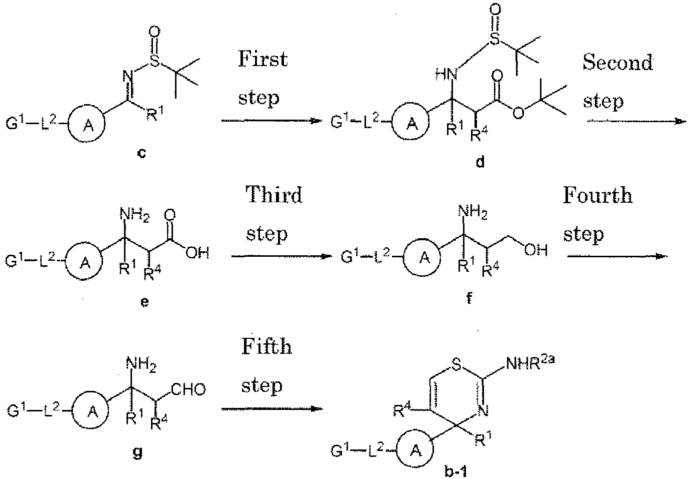

wherein G¹ is amino which may be protected, halogen or nitro and the other symbols are as defined above.

### First step

Compound d can be prepared by adding a titanium reagent such as chlorotitanium triisopropoxide to enolate, which is obtained by reacting an objective ester such as t-butyl propionate in the presence of a base such as lithium diisopropylamide in a solvent such as toluene, dichloromethane and tetrahydrofuran, or a mixed solvent thereof, adding Compound c which can be prepared by the known method, and reacting them at -80°C to 30°C, preferably -80°C to 0°C, for 0.1 to 24 hours, preferably 0.1 to 12 hours.

### Second step

Compound e can be prepared by reacting Compound d at 0°C to 80°C, preferably 0°C to 30°C, for 0.5 to 48 hours, preferably 1 to 24 hours in the presence of an acid such as hydrochloric acid, hydrobromic acid, sulfuric acid and trifluoroacetic acid in a solvent such as dioxane, methanol, and dichloromethane, or a mixed solvent thereof.

### Third step

Compound f can be prepared by adding a reducing agent such as borane, sodium hydride and lithium aluminum hydride to Compound e, and reacting at -80°C to 80°C, preferably -20°C to 30°C, for 0.5 to 48 hours, preferably 1 to 12 hours in a solvent such as dioxane, tetrahydrofuran, and toluene, or a mixed solvent thereof.

### Fourth step

Compound g can be prepared by adding an oxidizing agent such as 2-iodoxybenzoic acid to Compound f and reacting at 0°C to 80°C, preferably 10°C to 40°C, for 0.5 to 48 hours, preferably 1 to 12 hours in a solvent such as dimethyl sulfoxide, and dichloromethane.

In third step and fourth step, amine and/or aldehyde groups of Compound f and Compound g can be protected by the method described in Protective Groups in Organic Synthesis, Theodora W Green (John Wiley & Sons), and deprotected at an appropriate time, if necessary.

### Fifth step

Compound b-1 can be prepared by adding isothiocyanate having a protecting group, e.g. benzoyl isothiocyanate, which is commercially available or prepared by the known method, to Compound g, reacting at -30°C to 50°C, preferably -10°C to 25°C, for 0.1 to 12 hours, preferably 0.1 to 3 hours in a solvent such as dioxane, tetrahydrofuran, toluene and acetone, or a mixed solvent thereof, and subsequently, adding concentrated sulfuric acid or concentrated nitric acid, followed by a reaction at 0°C to 100°C, preferably 0°C to 60°C, for 0. 5 to 24 hours, preferably 1 to 12 hours.

### B-2) Preparation of Compound b-2

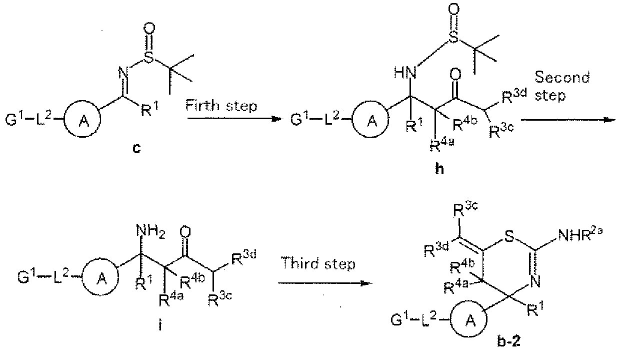

wherein each symbol is as defined above.

### First step

Compound h can be prepared by adding Compound c, which can be prepared by the publicly known method, to enolate, which can be prepared from the corresponding alkylketone, in a solution of toluene, dichloromethane, tetrahydrofuran, or mixed solvent thereof in the presence of bases, such as lithium diisopropylamide and potassium hexamethyldisilazide, and reacting them at -80°C to 30°C, preferably -80°C to 0°C, for 0.1 to 24 hours, preferably 0.1 to 12 hours.

### Second step

Compound i can be prepared by reacting Compound h obtained at the first step with an acid such as hydrochloric acid, hydrobromic acid or trifluoroacetic acid at 0°C to 60°C, preferably 0°C to 30°C, for 0.1 to 24 hours, preferably 0.5 to 12 hours.

### Third step

Compound b-2 can be prepared by adding isothiocyanate having a protecting group, e.g. benzoyl isothiocyanate, which is commercially available or prepared by the known method, to Compound i, reacting at -30°C to 70°C, preferably -20°C to 50°C, for 0.1 to 12 hours, preferably 0.1 to 6 hours in a solvent such as dioxane, tetrahydrofuran, toluene and acetone, or a mixed solvent thereof, and subsequently, adding concentrated sulfuric acid or concentrated nitric acid, followed by a reaction at -30°C to 70°C, preferably -20°C to 50°C, for 1 to 12 hours, preferably 1 to 6 hours.

### B-3) Preparation of Compound b-3

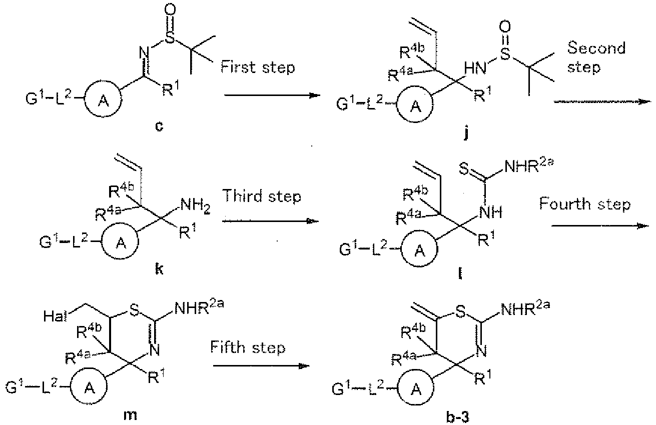

wherein each symbol is as defined above.

### First step

Compound j can be prepared by reacting Compound c, which can be prepared by the known method, with a Grignard reagent such as allylmagnesium bromide at -80°C to 30°C, preferably -80°C to 0°C, for 0.1 to 24 hours, preferably 0.1 to 12 hours in a solvent such as toluene, dichloromethane, and tetrahydrofuran, or a mixed solvent thereof.

### Second step

Compound k can be prepared by adding a hydrogen chloride solution to Compound j obtained in the first step, and reacting at -20°C to 80°C, preferably 0°C to 30°C, for 0.1 to 24 hours, preferably 0.1 to 12 hours in a solvent such as methanol, ethanol and water, or a mixed solvent thereof.

### Third step

Compound 1 can be prepared by adding isothiocyanate having a protecting group, e.g. benzoyl isothiocyanate, which is commercially available or is prepared by the known method, to Compound k, and reacting at -30°C to 70°C, preferably -20°C to 50°C, for 0.1 to 12 hours, preferably 0.1 to 6 hours, in a solvent such as dichloromethane, dioxane, tetrahydrofuran, toluene, and acetone, or a mixed solvent thereof.

### Fourth step

Compound m can be prepared by adding a halogenium cation source such as iodine, bromine and NBS to Compound I, and reacting at -20°C to 40°C, preferably 0°C to 20°C, for 0.1 to 12 hours, preferably 0.1 to 6 hours in a solvent such as dichloromethane.

### Fifth step

Compound b-3 can be prepared by adding a base such as pyrrolidine, piperidine, piperazine and morpholine to Compound m, and reacting at 20°C to 100°C, preferably 40°C to 80°C, for 0.1 to 24 hours, preferably 1 to 12 hours in a solvent such as dioxane, tetrahydrofuran, and toluene, or a mixed solvent thereof.

### B-4) Preparation of Compound b-4

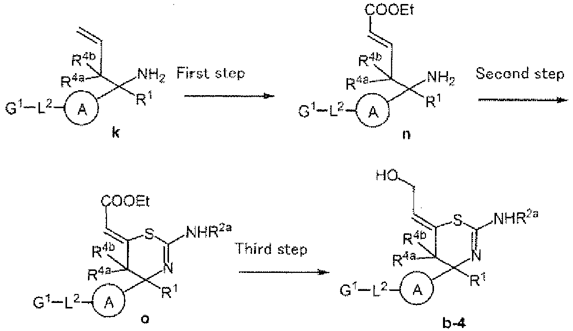

wherein each symbol is as defined above.

### First step

Compound n can be prepared by adding ethyl acrylate and Grubbs' reagent to Compound k in which an amino group is appropriately protected with a protecting group, and subjecting to an olefinmetathesis reaction in a solvent such as toluene, dichloromethane and tetrahydrofuran, or a mixed solvent thereof. A reaction temperature is -20°C to 60°C, preferably 0°C to 30°C, and a reaction time is 0.5 to 24 hours, preferably 1. to 12 hours.

### Second step

Compound o can be prepared by adding isothiocyanate having a protecting group, e.g. benzoyl isothiocyanate, which is commercially available or is prepared by the known method, to Compound n, and reacting at -30°C to 70°C, preferably -20°C to 50°C for 0.1 to 12 hours, preferably 0.1 to 6 hours in a solvent such as dichloromethane, dioxane, tetrahydrofuran, toluene, and acetone, or a mixed solvent thereof.

Compound b-4 can be prepared by adding diisobutylaluminum hydride, lithium aluminum hydride, or sodium hydride to Compound o, subjecting to a reducing reaction, and reacting them at -80°C to 0°C, preferably -80°C to -20°C, for 0.1 to 12 hours, preferably 0.1 to 3 hours in a solvent such as dioxane, tetrahydrofuran, and toluene, or a mixed solvent of them.

Compound b-4 can be subjected to an appropriately reaction to further convert an alcohol group.

### B-5) Preparation of Compound b-5

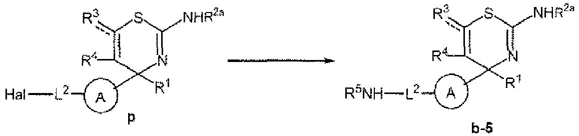

wherein each symbol is as defined above.

Compound b-5 can be prepared by adding tris(dibenzylideneacetone) dipalladium, palladium acetate, palladium (0) prepared in situ or the like, and a phosphine ligand such as tritert-butylphosphine, and dicyclohexylbiphenylphosphine to Compound p in a solvent such as tetrahydrofuran, toluene, and xylene, adding a reagent having a substituent corresponding to an objective compound such as lithium hexamethyldisilazide, and benzophenoneimine at -10°C to 30°C, and reacting them at 30°C to 120°C, preferably 50°C to 100°C, for 0.5 to 48 hours, preferably 3 to 20 hours.

The amino protecting group may be a substituent which can be deprotected by the method described in Protective Groups in Organic Synthesis, Theodora W Green (John Wiley & Sons), and examples include lower alkoxycarbonyl, lower alkenyloxycarbonyl, trialkylsilyl, acyl, methanesulfonyl, trifluoroethanesulfonyl, toluenesulfonyl and the like.

### B-6) Preparation of Compound b-6

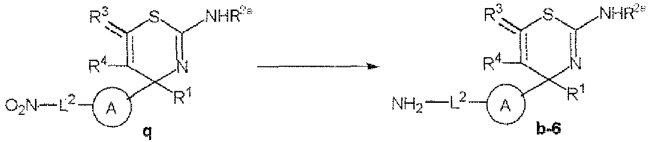

wherein each symbol is as defined above.
Compound b-6 can be prepared by adding iron to Compound q in a mixed solvent of acetic acid and water, followed by a reaction at 20°C to 120°C, preferably 50°C to 80°C, for 0.5 to 48 hours, preferably 6 to 20 hours.

Besides, Compound b-6 can be also prepared by adding a catalytic reducing catalyst such as 10% palladium/carbon to Compound q in a solvent such as tetrahydrofuran, ethyl acetate, and methanol, and reacting them at 30°C to 120°C, preferably 50°C to 80°C, for 0.5 to 48 hours, preferably 6 to 20 hours under the hydrogen atmosphere at a normal pressure to 5 atm, preferably a normal pressure to 2 atm, or by the method described in Comprehensive Organic Transformations, Richard C Larock (Mcgraw-Hill).

### B-7) Preparation of Compound b-7

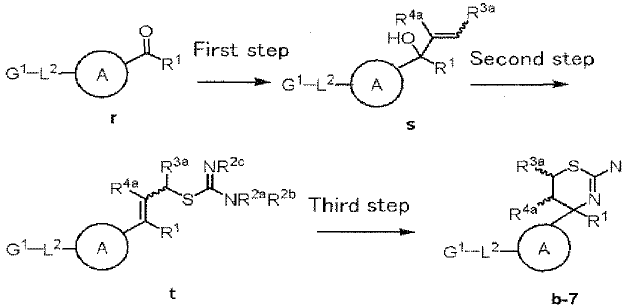

wherein one of R^{2b} and R^{2c} is at least hydrogen, and each other symbol is as defined above.

### First step

Compound s can be prepared by reacting Compound r, which can be commercially available or prepared by the known method, with a Grignard reagent having a substituent corresponding to that of the objective compound, such as vinylmagnesium chloride, vinylmagnesium bromide, propenylmagnesium bromide at -100°C to 50°C, preferably -80°C to 0°C, for 0.2 to 24 hours, preferably 0.5 to 5 hours in a solvent such as ether, tetrahydrofuran, or a mixed solvent such as ether-tetrahydrofuran.

Compound t can be prepared by reacting Compound s with a substituted thiourea having a substituent corresponding to that of the objective compound, such as thiourea, N-methylthiourea, N,N'-dimethylthiourea in an acid such as acetic acid, trifluoroacetic acid, hydrochloric acid, sulfuric acid, or those mixtures in the presence or absence of a solvent of toluene, at -20°C to 100°C, preferably 0°C to 50°C, for 0.5 to 120 hours, preferably 1 to 72 hours

Compound b-7 can be prepared by reacting Compound t with an acid such as trifluoroacetic acid, methanesulfonic acid, Trifluoromethanesulfonic acid, or those mixtures in the presence or absence of a solvent of toluene, at -20°C to 100°C, preferably 0°C to 50°C, for 0.5 to 120 hours, preferably 1 to 72 hours

### B-8) Preparation of Compound b-8

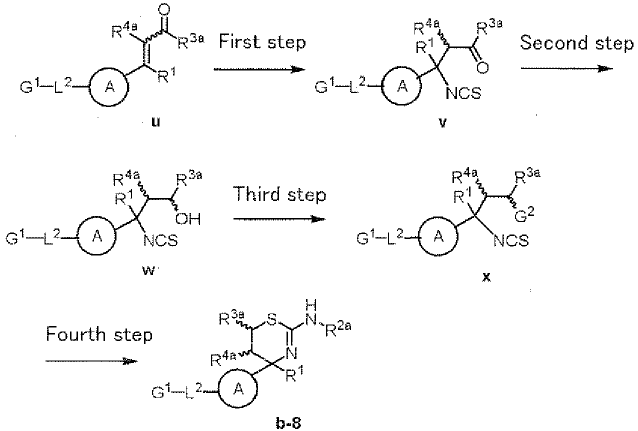

wherein G¹ is a leaving group such as halogen or sulfonyloxy, and the other symbols are as defined above.

### First step

Compound v can be prepared by reacting Compound u, which can be commercially available or prepared by the known method, with thiocyanate such as sodium thiocyanate and ammonium thiocyanate in the presence of water and an acid such as hydrochloric acid and sulfuric acid at 0°C to 150°C, preferably 20°C to 100°C, for 0.5 to 24 hours, preferably 1 to 12 hours in a solvent such as toluene, chloroform and tetrahydrofuran.

### Second process

Compound w can be prepared by adding a reducing agent such as sodium borohydride to Compound v, and reacting them at -80°C to 50°C, preferably -20°C to 20°C, for 0.1 to 24 hours, preferably 0.5 to 12 hours in a solvent such as tetrahydrofuran methanol, ethanol and water, or a mixed solvent such as ethanol-water in the presence or absence of buffer agent such as sodium dihydrogen phosphate.

### Third step

Compound x can be prepared by reacting Compound w with a halogenating agent such as thionyl chloride, phosphorus oxychloride and carbon tetrabromide-triphenylphosphine in the presence or absence of a solvent such as toluene and dichloromethane at -80°C to 50°C, preferably -20°C to 20°C, for 0.1 to 24 hours, preferably 0.5 to 12 hours, or can be prepared by reacting Compound w with a sulfonylating agent such as methanesulfonyl chloride and p-toluene sulfonyl chloride in the presence pf a base such as triethylamine at -80°C to 50°C, preferably -20°C to 20°C, for 0.1 to 24 hours, preferably 0.5 to 12 hours in a solvent of such as toluene and dichloromethane.

### Fourth step

Compound b-8 can be prepared by reacting Compound x with ammonia or a primary amine such as methylamine at -20°C to 80°C, preferably 0°C to 40°C, for 0.5 to 48 hours, preferably 1 to 24 hours in a solvent such as methanol, ethanol and water, or mixed solvent such as methanol-water.

### B-9) Preparation of Compound b-9

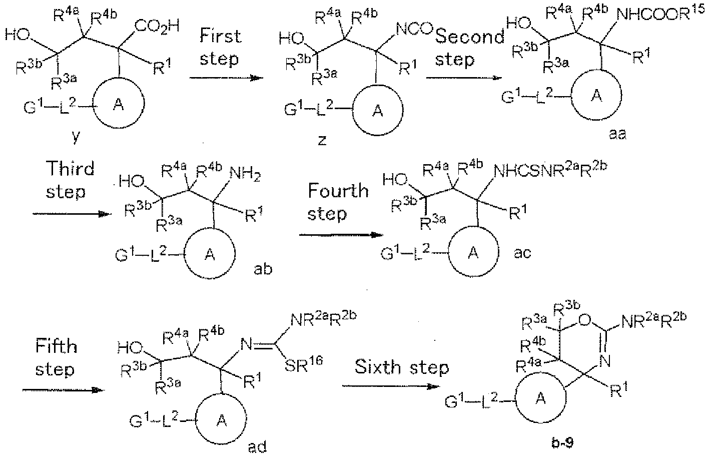

wherein R¹⁵ is substituted or unsubstituted lower alkyl such as t-butyl and benzyl, R¹⁶ is hydrogen or lower alkyl, and the other symbols are as defined above.

### First step

Compound z can be prepared by reacting Compound y, which can be commercially available or prepared by the known method, with azide agent such as diphenylphosphoryl azide in the presence of a base such as diisopropylethyl amine, triethylamine and pyridine at 0°C to 200°C, preferably 40°C to 150°C, for 1 to 48 hours, preferably 0.5 to 24 hours in a solvent such as toluene, t-butyl alcohol and tetrahydrofuran.

### Second step

Compound aa can be prepared by reacting compound z with an alcohol such as t-butylalcohol, 3,4-dimethoxybenzylalcohol and 4-methoxybenzylalcohol at 0°C to 30°C, preferably 50°C to 200°C, for 1 to 800 hours, preferably 5 to 500 hours in a solvent such as toluene, xylene, dimethylformamide and tetrahydrofuran.

Compound ab can be prepared by reacting Compound aa with an acid such as hydrochloric acid, sulfuric acid, hydrobromic acid and trifluoroacetic acid in the presence or absence of a solvent such as water, toluene, dichloromethane, methanol, 1,4-dioxane, acetic acid, ethyl acetate at 0°C to 200°C, preferably 25°C to 150°C, for 0.1 to 48 hours, preferably 0.5 to 24 hours.

### Fourth step

Compound ac can be prepared by reacting Compound ab with isothiocyanate corresponding to the objective compound such as methylisothiocyanate and ethylisothiocyanate, or thiocarbamoylhalide corresponding to the objective compound such as N,N-dimethylthiocarbamoylchloride and N,N-diethylthiocarbamoylchloride in the presence of a base such as diisopropylethylamine, triethylamine and pyridine in a solvent such as acetone, toluene, chloroform, tetrahydrofuran and water, or those mixed solvents at 0°C to 150°C, preferably 20°C to 100°C, for 0.5 to 120 hours, preferably 1 to 72 hours.

### Fifth step

Compound ad can be prepared by reacting Compound ac with an alkylating agent such as methyl iodide, diethyl sulfuric acid and benzyl bromide in the presence or absence of a base such as diisopropylethyl amine, triethylamine, pyridine and sodium hydroxide at 0°C to 200°C, preferably 40°C to 150°C, for 1 to 48 hours, preferably 0.5 to 24 hours in a solvent such as acetone, acetonitrile, dimethylformamide and tetrahydrofuran.

Compound b-9 can be prepared by reacting Compound ad with a base such as diisopropyl ethyl amine, triethylamine, pyridine and sodium hydroxide at 0°C to 200°C, preferably 10°C to 150°C, for 1 to 120 hours, preferably 0.5 to 100 hours in a solvent such as acetone, acetonitrile, dimethylformamide, tetrahydrofuran and dichloromethane.

### B-10) Preparation of Compound b-10 or b-11

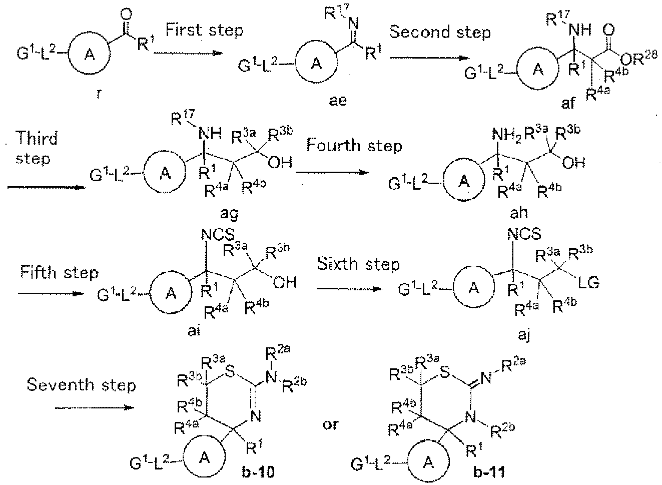

wherein R¹⁷ is sulfoxide having substituted or unsubstituted alkyl, substituted or unsubstituted lower alkenyl, a substituted or unsubstituted carbocyclic group, a substituted or unsubstituted heterocyclic group or the like, or α-methylbenzyl; LG is a leaving group such as halogen, lower alkylsulfonyloxy; and the other symbols are as defined above.
The above compounds ae and af can be prepared in accordance with the method described in (1) T. Fujisawa et al., Tetrahedron Lett., 37,3881-3884 (1996), (2) D. H. Hua et al., Sulfur Reports, vol.21, pp.211-239 (1999), (3) Y. Koriyama et al., Tetrahedron, 58, 9621-9628 (2002) or (4) T. Vilavan et al., Current Organic Chemistry, 9, 1315-1392 (2005), or the methods described below.

### First step

Compound ae can be prepared by reacting Compound r, which can be commercially available or prepared by the known method, with an agent having the substituent corresponding to that of the objective compound, such as α-methyl benzylamine, p-toluene sulfinic amide or tert-butyl sulfinic amide, and drying continuously in the presence of molecular sieve or magnesium sulfate, or under the Dean Stark equipment or in accordance with the above described method, at 60°C to 120°C, preferably 80°C to 100°C, for 0.5 to 24 hours, preferably 0.5 to 5 hours in a solvent such as ether, tetrahydrofuran, toluene, benzene or the mixed solvent such as ether-tetrahydrofuran.

### Second step

Compound af can be prepared by reacting lithium, aluminium, zinc, or titanium enolate derived from an agent having the substituent corresponding to that of the objective compound, such as ethyl acetate, which can be commercially available or prepared by the known method, or ketene silyl acetate prepared from an agent having the substituent corresponding to that of the objective compound, such as ethyl acetate, with Compound ae in the presence or absence of Lewis acid such as titanium tetrachloride or boron trifluoride ether complex, at -100°C to 50°C, preferably -80°C to -30°C, for 0.5 to 24 hours, preferably 0.5 to 5 hours in a solvent such as ether, tetrahydrofuran, toluene, methylene chloride or the mixed solvent such as ether-tetrahydrofuran.

### Third step

Compound ag can be prepared by adding Grignard agent having the substituent corresponding to that of the objective compound, such as methylmagnesium chloride or ethylmagnesium bromide, to Compound af at -100°C to 50°C, preferably -80°C to -30°C, or reacting Weinreb amide derived from Compound af with Grignard agent having the substituent corresponding to that of the objective compound, such as R^{3a}MgBr or R^{3b}MgBr, for 0.2 to 24 hours, preferably 0.2 to 5 hours in a solvent such as ether or tetrahydrofuran or the mixed solvent such as ether-tetrahydrofuran.

### Fourth step

Compound ah can be prepared by treating Compound ag with a solution of hydrogen chloride, trifluoroacetic acid or the like in ethanol, ether, 1, 4-dioxane, methylene chloride or ethyl acetate or with trifluoroacetic acid in the absence of a solvent, at -30°C to 100°C, preferably -10°C to 90°C, for 0.5 to 12 hours, preferably 0.5 to 5 hours.

### Fifth step

Compound ai can be prepared by adding calcium carbonate, potassium carbonate or the like to a solution of Compound ah in methylene chloride, toluene or the like or in the mixed solvent such as methylene chloride-water, and adding thio phosgene thereto at -30°C to 50°C, preferably -10°C to 25°C, for 0.5 to 12 hours, preferably 0.5 to 5 hours.

### Sixth step

Compound aj can be prepared by adding oxalyl chloride, thionyl chloride or the like and catalytic amount of N, N-dimethylformamide to a solution of Compound ai in methylene chloride, tetrahydrofuran, toluene or the like at 0°C to 100°C, preferably 20°C to 90°C, for 0.5 to 12 hours, preferably 0.5 to 5 hours or by the method described in Comprehensive Organic Transformations (Richard C Larock (Mcgraw-Hill)).

### Seventh step

Compound b-10 or b-11 can be prepared by adding 15 to 30% aqueous ammonia or an agent having the substituent corresponding to that of the objective compound such as tert-butyl amine or the like at -30°C to 50°C, preferably -10°C to 30°C to a solution of Compound aj in ethyl acetate, methylene chloride, tetrahydrofuran, toluene or the like, and reacting them at -10°C to 30°C, preferably 0°C to 30°C, for 0.5 to 72 hours.

When R^{2a} and R^{2b} of Compound b-10 or b-11 are hydrogen, the objective R^{2a} and R^{2b} can be introduced to such compound in accordance with the usual method, if necessary.

The above Compounds b-1, b-2, b-3, b-4, b-5, b-6, b-7, b-8, b-9, b-10, b-11, (I) and (I') can be prepared in accordance with the method described in (I) T.Fujisawa et al., Tetrahedron Lett., 37, 3881-3884 (1996), (2) D.H.Hua et al, Sulfur Reports, vol.21, pp.211-239 (1999), (3) Y.Koriyama et al., Tetrahedron, 58, 9621-9628 (2002), (4) T.Vilavan et al, Current Organic Chemistry, 9, 1315-1392 (2005), Patent Literature 1, Patent Literature 2, Patent Literature 3 or the like.

Moreover, the optically active isomer of the compound (I) can be prepared by using an optically active compound as a staring agent, performing an asymmetric synthesis in the suitable stage to prepare an optically active intermediate, or optical resolution of the racemate of the intermediate or the objective compound in the appropriate stage. The method of optical resolution include the separation of optical isomer using an optically active column, the kinetics optical resolution using enzyme reaction or the like, the crystallization and separation of diastereomers by the salt formation using chiral acids or chiral bases, the priority crystallization or the like.

Moreover, optically active compounds of formula (I) and (I') can be prepared by using optically active sulfinyl imines c as starting materials in the above preparation method.

Preferable embodiments of the present invention are illustrated below. Each symbol is as defined above.

In the formula (I), the followings are preferable.

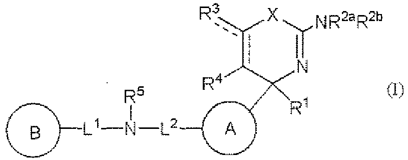

R¹ includes substituted or unsubstituted alkyl, substituted or unsubstituted alkenyl, substituted or unsubstituted alkynyl, cyano, a substituted or unsubstituted, carbocyclic group, or a substituted or unsubstituted heterocyclic group.

Examples of R¹ include Cl to C3 unsubstituted alkyl.

R^{2a} and R^{2b} include each independently hydrogen, substituted or unsubstituted alkyl or substituted or unsubstituted acyl.

Examples of R^{2a} and R^{2b} include hydrogen at the same time.

X includes S or O.

Examples of X include S.

Examples of X include O.

When X is S,

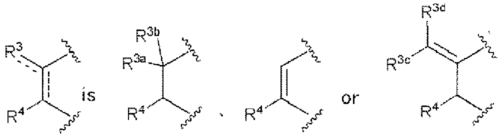

When X is S,

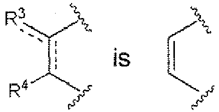

For example, when X is S,

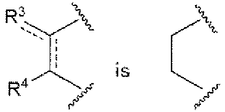

When X is O,

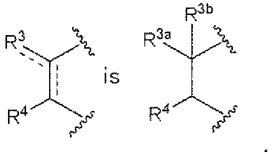

For example, when X is O,

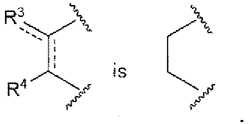

R^{3a} and R^{3b} include each independently, hydrogen, halogen, hydroxy, substituted or unsubstituted alkyl, substituted or unsubstituted alkenyl, substituted or unsubstituted alkynyl, substituted or unsubstituted acyl, substituted or unsubstituted alkoxy, substituted or unsubstituted carbocyclylalkyl, substituted or unsubstituted heterocyclylalkyl, substituted or unsubstituted carbocyclylalkoxy, substituted or unsubstituted heterocyclylalkoxy, substituted or unsubstituted alkylthio, carboxy, cyano, substituted or unsubstituted alkoxycarbonyl, substituted or unsubstituted amino, substituted or unsubstituted carbamoyl, a substituted or unsubstituted carbocyclic group, a substituted or unsubstituted heterocyclic group.

R^{3a} and R^{3b}, for example, include each independently, hydrogen, substituted or unsubstituted alkyl, substituted or unsubstituted alkoxycarbonyl, substituted or unsubstituted carbamoyl, a substituted or unsubstituted carbocyclic group, a substituted or unsubstituted heterocyclic group.

Examples of R^{3a} and R^{3b} include hydrogen.

R^{3c} and R^{3d} include each independently hydrogen, halogen, substituted or unsubstituted alkyl, or R^{3c} and R^{3d} together with the carbon atom to which they are attached may form a substituted or unsubstituted non-aromatic carbocycle.

R^{3c} and R^{3d}, for example, include each independently hydrogen, halogen, substituted or unsubstituted alkyl.

R^{4a} and R^{4b} include each independently hydrogen or substituted or unsubstituted alkyl.

Examples of R^{4a} and R^{4b} include hydrogen.

R⁵ includes hydrogen, substituted or unsubstituted alkyl, substituted or unsubstituted alkenyl, substituted or unsubstituted alkynyl or substituted or unsubstituted acyl.

Examples of R⁵ include hydrogen or substituted or unsubstituted alkyl.

L¹ and L² include each independently, bond, substituted or unsubstituted alkylene wherein the substituent thereof does not include oxo nor thioxo, substituted or unsubstituted alkenylene wherein the substituent thereof does not include oxo nor thioxo, or substituted or unsubstituted alkynylene wherein the substituent thereof does not include oxo nor thioxo,
L¹ and L² include each independently a bond;
substituted or unsubstituted alkylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfanylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy;
substituted or unsubstituted alkenylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfanylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; or
substituted or unsubstituted alkynylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfanylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy.

Examples af L¹ and L² include a bond.

Ring A is a substituted or unsubstituted carbocycle or a substituted or unsubstituted heterocycle.

Examples of ring A include substituted or unsubstituted benzene.

1) when both of L¹ and L² are a bond, and

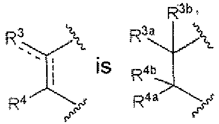

then ring B is a substituted or unsubstituted carbocycle, substituted or unsubstituted pyridine, substituted or unsubstituted pyrimidine or a substituted or unsubstituted 5-membered heterocycle.

Ring B, for example, includes a substituted or unsubstituted benzene, substituted or unsubstituted pyridine or substituted or unsubstituted pyrimidine.

2) when both of L¹ and L² are a bond, and

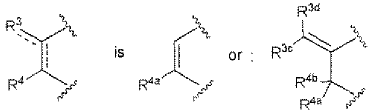

then ring B is a substituted or unsubstituted carbocycle or a substituted or unsubstituted heterocycle.

Ring B is a substituted or unsubstituted carbocycle, substituted or unsubstituted pyridine, substituted or unsubstituted pyrimidine or a substituted or unsubstituted 5-membered heterocycle.

Examples of ring B include substituted or unsubstituted pyridine, substituted or unsubstituted pyrimidine.

3) when at least one of L¹ and L² is substituted or unsubstituted alkylene, substituted or unsubstituted alkenylene or substituted or unsubstituted alkynylene, then ring B is substituted nitrogen-containing aromatic monocycle.

Examples of ring B include substituted pyridine or substituted pyrimidine.

Specific examples of the compound of the formula (I) are illustrated below.
The compound wherein
R¹ is substituted or unsubstituted alkyl,
both of R^{2a} and R^{2b} are hydrogen,
X is S, and 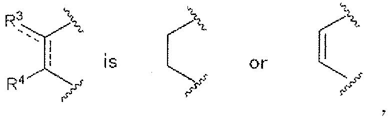 or
X is O, and 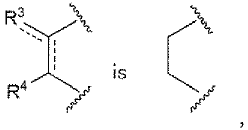 R⁵ is hydrogen,
L¹ and L² are bonds,
ring A is a substituted or unsubstituted carbocycle,
ring B is a substituted or unsubstituted carbocycle, substituted or unsubstituted pyridine or substituted or unsubstituted pyrimidine,
its pharmaceutically acceptable salt, or a solvate thereof.

The compound wherein
R¹ is C 1 to C3 unsubstituted alkyl,
both of R^{2a} and R^{2b} are hydrogen,
X is S, and 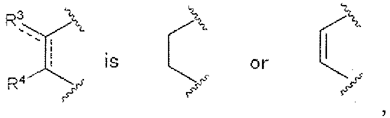 or
X is O, and 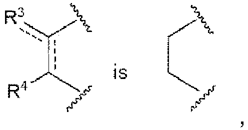 R⁵ is hydrogen,
L¹ and L² are bonds,
ring A is benzene optionally substituted with halogen,
ring B is a substituted or unsubstituted carbocycle, substituted or unsubstituted pyridine or substituted or unsubstituted pyrimidine, wherein the substituent on ring B is one or more selected from the group of halogen, hydroxy, alkyl, halogenoalkyl, hydroxyalkyl, alkoxyalkoxy, alkoxy, alkoxyalkoxy, alkoxycarbonyl, amino, alkylamino, carbamoyl, cyano and nitro,
its pharmaceutically acceptable salt, or a solvate thereof.

In the above formula (I), more specific embodiments are illustrated below.
The compound of the formula (I) wherein 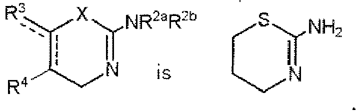 Hereinafter referred to as "ring X is X1".
The compound of the formula (I) wherein 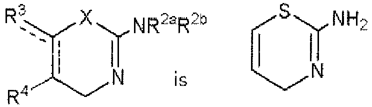 Hereinafter referred to as "ring X is X2".
The compound of the formula (I) wherein 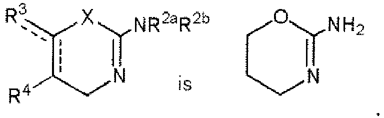 Hereinafter referred to as "ring X is X3".
The compound of the formula (I) wherein 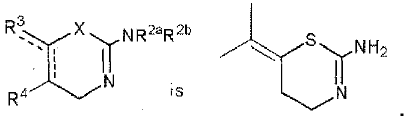 Hereinafter referred to as "ring X is X4".
The compound of the formula (I) wherein R¹ is methyl. Hereinafter referred to as "R¹ is R11".
The compound of the formula (I) wherein R¹ is trifluoromethyl. Hereinafter referred to as "R¹ is R12".
The compound of the formula (I) wherein ring A is 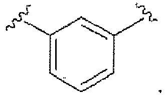 Hereinafter referred to as "ring A is A1".
The compound of the formula (I) wherein ring A is 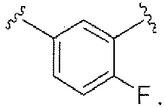 Hereinafter referred to as "ring A is A2".
The compound of the formula (I) wherein ring A is 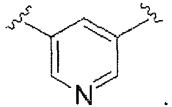 Hereinafter referred to as "ring A is A3".
The compound of the formula (I) wherein 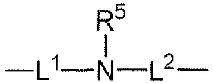 is

—NH—

Hereinafter referred to as "L is L1".
The compound of the formula (I) wherein 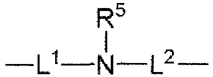 is

—CH₂-NH—

Hereinafter referred to as "L is L2".
The compound of the formula (I) wherein 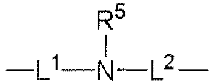 is

-NH-CH₂—.

Hereinafter referred to as "L is L3".
The compound of the formula (I) wherein ring B is 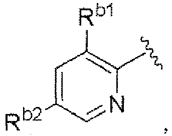 wherein R^{b1} and R^{b2} are each independently hydrogen, chloro, methoxy, hydroxymethyl, cyano, amino or carbamoyl. Hereinafter referred to as "ring B is B 1".
The compound of the formula (I) wherein ring B is 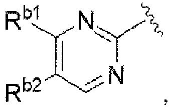 wherein R^{b1} and R^{b2} are defined above. Hereinafter referred to as "ring B is B2".
The compound of the formula (I) wherein ring B is 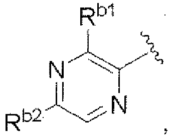 wherein R^{b1} and R^{b2} are defined above. Hereinafter referred to as "ring B is B3".
The compound of the formula (I) wherein ring B is 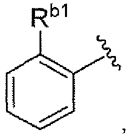 wherein R^{b1} and R^{b2} are defined above. Hereinafter referred to as "ring B is B4".

The compound of the formula (I) wherein the combination of ring X, R¹, ring A, L and ring B (ring X, R¹, ring A, L, ring B) is as follows:
(X1,R11,A1,L1,B1),(X1,R11,A1,L1,B2),(X1,R11,A1,L1,B3),(X1,R11,A1,L1,B4),(X1,R11,A 1,L2,B1),(X1,R11,A1,L2,B2),(X1,R11,A1,L2,B3),(X1,R11,A1,L2,B4),(X1,R11,A1,L3,B1),( X1,R11,A1,L3,B2),(X1,R11,A1,L3,B3),(X1,R11,A1,L3,B4),(X1,R11,A2,L1,B1),(X1,R11,A2 ,L1,B2),(X1,R11,A2,L1,B3),(X1,R11,A2,L!,B4),(X1,R11,A2,L2,B1),(X1,R11,A2,L2,B2),(X 1,R11,A2,L2,B3),(X1,R11,A2,L2,B4),(X1,R11,A2,L3,B1),(X1,R11,A2,L3,B2),(X1,R11,A2, L3,B3),(X1,R11,A2,L3,B4),(X1,R11,A3,L1,B1),(X1,R11,A3,L1,B2),(X1,R11,A3,L1,B3),(X1 ,R11,A3,L1,B4),(X1,R11,A3,L2,B1),(X1,R11,A3,L2,B2),(X1,R11,A3,L2,B3),(X1,R11,A3,L 2,B4),(X1,R11,A3,L3,B1),(X1,R11,A3,L3,B2),(X1,R11,A3,L3,B3),(X1,R11,A3,L3,B4),(X1, R12,A1,L1,B1),(X1,R12,A1,L1,B2),(X1,R12,A1,L1,B3),(X1,R12,A1,L1,B4),(X1,R12,A1,L 2,B1),(X1,R12,A1,L2,B2),(X1,R12,A1,L2,B3),(X1,R12,A1,L2,B4),(X1,R12,A1,L3,B1),(X1, R12,A1,L3,B2),(X1,R12,A1,L3,B3),(X1,R12,A1,L3,B4),(X1,R12,A2,L1,B1),(X1,R12,A2,L 1,B2),(X1,R12,A2,L1,B3),(X1,R12,A2,L1,B4),(X1,R12,A2,L2,B1),(X1,R12,A2,L2,B2),(X1, R12,A2,L2,B3),(X1,R12,A2,L2,B4),(X1,R12,A2,L3,B1),(X1,R12,A2,L3,B2),(X1,R12,A2,L 3,B3),(X1,R12,A2,L3,B4),(X1,R12,A3,L1,B1),(X1,R12,A3,L1,B2),(X1,R12,A3,L1,B3),(X1, R12,A3,L1,B4),(X1,R12,A3,L2,B1),(X1,R12,A3,L2,B2),(X1,R12,A3,L2,B3),(X1,R12,A3,L 2,B4),(X1,R12,A3,L3,B1),(X1,R12,A3,L3,B2),(X1,R12,A3,L3,B3),(X1,R12,A3,L3,B4),(X2, R11,A1,L1,B1),(X2,R11,A1,L1,B2),(X2,R11,A1,L1,B3),(X2,R11,A1,L1,B4),(X2,R11,A1,L2 ,B1),(X2,R11,A1,L2,B2),(X2,R11,A1,L2,B3),(X2,R11,A1,L2,B4),(X2,R11,A1,L3,B1),(X2,R 11,A1,L3,B2),(X2,R11,A1,L3,B3),(X2,R11,A1,L3,B4),(X2,R11,A2,L1,B1),(X2,R11,A2,L1, B2),(X2,R11,A2,L1,B3),(X2,R11,A2,L1,B4),(X2,R11,A2,L2,B1),(X2,R11,A2,L2,B2),(X2,R1 1,A2,L2,B3),(X2,R11,A2,L2,B4),(X2,R11,A2,L3,B1),(X2,R11,A2,L3,B2),(X2,R11,A2,L3,B 3),(X2,R11,A2,L3,B4),(X2,R11,A3,L1,B1),(X2,R11,A3,L1,B2),(X2,R11,A3,L1,B3),(X2,R11 ,A3,L1,B4),(X2,R11,A3,L2,B1),(X2,R11,A3,L2,B2),(X2,R11,A3,L2,B3),(X2,R11,A3,L2,B4) ,(X2,R11,A3,L3,B1),(X2,R11,A3,L3,B2),(X2,R11,A3,L3,B3),(X2,R11,A3,L3,B4),
(X2,R12,A1,L1,B1),(X2,R12,A1,L1,B2),(X2,R12,A1,L1,B3),(X2,R12,A1,L1,B4),(X2,R12,A 1,L2,B1),(X2,R12,A1,L2,B2),(X2,R12,A1,L2,B3),(X2,R12,A1,L2,B4),(X2,R12,A1,L3,B1),( X2,R12,A1,L3,B2),(X2,R12,A1,L3,B3),(X2,R12,A1,L3,B4),(X2,R12,A2,L1,B1),(X2,R12,A 2,L1,B2),(X2,R12,A2,L1,B3),(X2,R12,A2,L1,B4),(X2,R12,A2,L2,B1),(X2,R12,A2,L2,B2),( X2,R12,A2,L2,B3),(X2,R12,A2,L2,B4),(X2,R12,A2,L3,B1),(X2,R12,A2,L3,B2),(X2,R12,A 2,L3,B3),(X2,R12,A2,L3,B4),(X2,R12,A3,L1,B1),(X2,R12,A3,L1,B2),(X2,R12,A3,L1,B3),( X2,R12,A3,L1,B4),(X2,R12,A3,L2,B1),(X2,R12,A3,L2,B2),(X2,R12,A3,L2,B3),(X2,R12,A 3,L2,B4),(X2,R12,A3,L3,B1),(X2,R12,A3,L3,B2),(X2,R12,A3,L3,B3),(X2,R12,A3,L3,B4),( X3,R11,A1,L1,B1),(X3,R11,A1,L1,B2),(X3,R11,A1,L1,B3),(X3,R11,A1,L1,B4),(X3,R11,A1 ,L2,B1),(X3,R11,A1,L2,B2),(X3,R11,A1,L2,B3),(X3,R11,A1,L2,B4),(X3,R11,A1,L3,B1),(X 3,R11,A1,L3,B2),(X3,R11,A1,L3,B3),(X3,R11,A1,L3,B4),(X3,R11,A2,L1,B1),(X3,R11,A2, L 1,B2),(X3,R11,A2,L1,B3),(X3,R11,A2,L1,B4),(X3,R11,A2,L2,B 1),(X3,R11,A2,1,2,B2),(X3 ,R11,A2,L2,B3),(X3,R11,A2,L2,B4),(X3,R11,A2,L3,B 1),(X3,R11,A2,L3,B2),(X3,R11,A2,L 3,B3),(X3,R11,A2,L3,B4),(X3,R11,A3,L1,B 1),(X3,R11,A3,L1,B2),(X3,R11,A3,L1,B3),(X3, R11 1,A3,L1,B4),(X3,R 11,A3,L2,B 1),(X3,R11,A3,L2,B2),(X3,R11,A3,L2,B3),(X3,R11,A3,L2 ,B4),(X3,R11,A3,L3,B 1),(X3,R11,A3,L3,B2),(X3,R11,A3,L3,B3),(X3,R11,A3,L3,B4),(X3,R 12,A1,L1,B 1),(X3,R12,A1,L1,B2),(X3,R12,A1,L1,B3),(X3,R12,A1,L1,B4),(X3,R12,A1,L2, B 1),(X3,R12,A1,L2,82),(X3,R12,A1,L2,83),(X3,R12,A1,L2,84),(X3,R12,A1,L3,B 1),(X3,R 12,A1,L3,B2),(X3,R12,A1,L3,B3),(X3,R12,A1,L3,B4),(X3,R12,A2,L1,B1),(X3,R12,A2,L1, B2),(X3,R12,A2,L1,B3),(X3,-R12,A2,L1,B4),(X,3,R12,A2,L2,B 1),(X3,R12,A2,L2,B2),(X3,R 12,A2,L2,83),(X3,R12,A2,L2,84),(X3,R12,A2,L3,B),(X3,R12,A2,L3,82),(X3,R12,A2,L3, B3),(X3,R12,A2,1,3,B4),(X3,R12,A3,L1,B1),(X3,R12,A3,L1,B2),(X3,R12,A3,L1,B3),(X3,R 12,A3,L1,B4),(X3,R12,A3,L2,B1),(X3,R12,A3,L2,B2),(X3,R12,A3,L2,B3),(X3,R12,A3,L2, B4),
(X3,R12,A3,L3,B1),(X3,R12,A3,L3,B2),(X3,R12,A3,L3,B3),(X3,R12,A3,L3,B4),(X4,R11,A 1,L1,B1),(X4,R11,A1,L1,B2),(X4,R11,A1,L1,B3),(X4,R11,A1,L1,B4),(X4,R11,A1,L2,B1),( X4,R11,A1,L2,B2), (X4,R 11,A1,L2,B3),(X4,R11,A1,L2,B4),(X4,R11,A1,L3,B 1),(X4,R11,A1 ,L3,82),(X4,R11,A1,L3,83),(X4,R11,A1,L3,84),(X4,R11,A2,L1,B 1),(X4,R1I,A2,L1,B2),(X 4,R11,A2,L1,B3),(X4,R11,A2,L1,B4),(X4,R11,A2,L2,B1),(X4,R11,A2,L2,B2),(X4,R11,A2, L2,83),(X4,R11,A2,L2,B4),(X4,R11,A2,L3,B1),(X4,R11,A2,L3,82),(X4,R11,A2,L3,83),(X4 ,R11,A2,L3,B4),(X4,R11,A3,L1,B1),(X4,R11,A3,L1,B2),(X4,R11,A3,L1,B3),(X4,R11,A3,L 1,84),(X4,R11,A3,L2,B 1), (X4,R11,A3,L2,B2), (X4,R11,A3,L2,B3),(X4,R11,A3,L2,B4),(X4, R11,A3,L,3,B 1),(X4,R11,A3,L3,B2),(X4,R11,A3,L3,B3),(X4,R11,A3,L3,B4),(X4,R12,A1,L1 ,B1),(X4,R12,A1,L1,B2),(X4,R12,A1,L1,B3),(X4,R12,A1,L1,B4),(X4,R12,A1,L2,B1),(X4,R 12,A1,L2,B2),(X4,R12,A1,L2,B3),(X4,R12,A1,L2,B4),(X4,R12,A1,L3,B1),(X4,R12,A1,L3, B2),(X4,R12,A1,L3,B3),(X4,R12,A1,L3,B4),(X4,R12,A2,L1,B1),(X4,R12,A2,L1,B2),(X4,R 12,A2,L1,B3),(X4,R12,A2,L1,B4),(X4,R12,A2,L2,B1),(X4,R12,A2,L2,B2),(X4,R12,A2,L2, B3),(X4.R12,A2,L2,B4),(X4,R12,A2,L3,B1),(X4,R12,A2,L3,B2),(X4,R12,A2,L3,B3),(X4,R 12,A2,L3,84),(X4,R12,A3,L1,B 1),(X4,R 12,A3,L1,B2),(X4,R12,A3,L1,B3),(X4,-R 12,A3,L1, B4), (X4,R 12,A3,L2,B 1),(X4,R12,A3,L2,82),(X4,R12,A3,L2,83),(X4,R12,A3,L2,84),(X4,R 12,A3,L3,B1),(X4,R12,A3,L3,B2),(X4,R12,A3,L3,B3) or (X4,R12,A3,L3,B4).

The compound of the formula (I) wherein ring B is 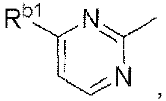 wherein R^{b1} is hydrogen, chloro, methoxy, hydroxymethyl, cyano, amino or carbamoyl. Hereinafter referred to as "ring B is B5".

The compound of the formula (I) wherein the combination of ring B, R^{b1} and R^{b2} (ring B, R^{b1}, R^{b2}) is as follows:
(B1, hydrogen, hydrogen) Hereinafter referred to as "ring B is b1".
(B1, hydrogen, chloro) Hereinafter referred to as "ring B is b2".
(B1, hydrogen, methoxy) Hereinafter referred to as "ring B is b3".
(B1, hydrogen, hydroxymethyl) Hereinafter referred to as "ring B is b4".
(B1, hydrogen, cyano) Hereinafter referred to as "ring B is b5".
(B1, hydrogen, amino) Hereinafter referred to as "ring B is b6".
(B1, hydrogen, carbamoyl) Hereinafter referred to as "ring B is b7".
(B1, chloro, hydrogen) Hereinafter referred to as "ring B is b8".
(B1, chloro, chloro) Hereinafter referred to as "ring B is b9".
(B1, chloro, methoxy) Hereinafter referred to as "ring B is b10".
(B1, chloro, hydroxymethyl) Hereinafter referred to as "ring B is b11".
(B1, chloro, cyano) Hereinafter referred to as "ring B is b12".
(B1, chloro, amino) Hereinafter referred to as "ring B is b13".
(B1, chloro, carbamoyl) Hereinafter referred to as "ring B is b14".
(B1, methoxy, hydrogen) Hereinafter referred to as "ring B is b15".
(B1, methoxy, chloro) Hereinafter referred to as "ring B is b16".
(B1, methoxy, methoxy) Hereinafter referred to as "ring B is b17".
(B1, methoxy, hydroxymethyl) Hereinafter referred to as "ring B is b18".
(B1, methoxy cyano) Hereinafter referred to as "ring B is b19".
(B1, methoxy, amino) Hereinafter referred to as "ring B is b20".
(B1, methoxy, carbamoyl) Hereinafter referred to as "ring B is b21".
(B1, cyano, hydrogen) Hereinafter referred to as "ring B is b22".
(B1, cyano, chloro) Hereinafter referred to as "ring B is b23".
(B1, cyano, methoxy) Hereinafter referred to as "ring B is b24".
(B1, cyano, hydroxymethyl) Hereinafter referred to as ring B is b25.
(B1, cyano, cyano) Hereinafter referred to as "ring B is b26".
(B1, cyano, amino) Hereinafter referred to as "ring B is b27".
(B1, cyano, carbamoyl) Hereinafter referred to as "ring B is b28".
(B1, hydroxymethyl, hydrogen) Hereinafter referred to as "ring B is b29".
(B1, hydroxymethyl, chloro) Hereinafter referred to as "ring B is b30".
(B1, hydroxyethyl, methoxy) Hereinafter referred to as "ring B is b31".
(B1, hydroxymethyl, cyano) Hereinafter referred to as "ring B is b32".
(B1, amino, hydrogen) Hereinafter referred to as "ring B is b33".
(B1, amino, chloro) Hereinafter referred to as "ring B is b34".
(B1, amino, methoxy) Hereinafter referred to as "ring B is b35".
(B1, amino, cyano) Hereinafter referred to as "ring B is b36".
(B1, carbamoyl, hydrogen) Hereinafter referred to as "ring B is b37".
(B1, carbamoyl, chloro) Hereinafter referred to as "ring B is b38".
(B1, carbamoyl, methoxy) Hereinafter referred to as "ring B is b39".
(B1, carbamoyl, cyano) Hereinafter referred to as "ring B is b40".

(B3, hydrogen, hydrogen) Hereinafter referred to as "ring B is b41".
(B3, hydrogen, chloro) Hereinafter referred to as "ring B is b42".
(B3, hydrogen, methoxy) Hereinafter referred to as "ring B is b43".
(B3, hydrogen, hydroxymethyl) Hereinafter referred to as "ring B is b44".
(B3, hydrogen, cyano) Hereinafter referred to as "ring B is b45".
(B3, hydrogen, amino) Hereinafter referred to as "ring B is b46".
(B3, hydrogen, carbamoyl) Hereinafter referred to as "ring B is b47".
(B3, chloro, hydrogen) Hereinafter referred to as "ring B is b48".
(B3, chloro, chloro) Hereinafter referred to as "ring B is b49".
(B3, chloro, methoxy) Hereinafter referred to as "ring B is b50".
(B3, chloro, hydroxymethyl) Hereinafter referred to as "ring B is b51".
(B3, chloro, cyano) Hereinafter referred to as "ring B is b52".
(B3, chloro, amino) Hereinafter referred to as "ring B is b53".
(B3, chloro, carbamoyl) Hereinafter referred to as "ring B is b54".
(B3, methoxy, hydrogen) Hereinafter referred to as "ring B is b55".
(B3, methoxy, chloro) Hereinafter referred to as "ring B is b56".
(B3, methoxy, methoxy) Hereinafter referred to as "ring B is b57".
(B3, methoxy, hydroxymethyl) Hereinafter referred to as "ring B is b58".
(B3, methoxy, cyano) Hereinafter referred to as "ring B is b59".
(B3, methoxy, amino) Hereinafter referred to as "ring B is b60".
(B3, methoxy, carbamoyl) Hereinafter referred to as "ring B is b61".
(B3, cyano, hydrogen) Hereinafter referred to as "ring B is b62".
(B3, cyano, chloro) Hereinafter referred to as "ring B is b63".
(B3, cyano, methoxy) Hereinafter referred to as "ring B is b64".
(B3, cyano, hydroxymethyl) Hereinafter referred to as "ring B is b65".
(B3, cyano, cyano) Hereinafter referred to as "ring B is b66".
(B3, cyano, amino) Hereinafter referred to as "ring B is b67".
(B3, cyano, carbamoyl) Hereinafter referred to as "ring B is b68".
(B3, hydroxymethyl, hydrogen) Hereinafter referred to as "ring B is b69".
(B3, hydroxymethyl, chloro) Hereinafter referred to as "ring B is b70".
(B3, hydroxymethyl, methoxy) Hereinafter referred to as "ring B is b71".
(B3, hydroxymethyl, cyano) Hereinafter referred to as "ring B is b72".
(B3, amino, hydrogen) Hereinafter referred to as "ring B is b73".
(B3, amino, chloro) Hereinafter referred to as "ring B is b74".
(B3, amino, methoxy) Hereinafter referred to as "ring B is b75".
(B3, amino, cyano) Hereinafter referred to as "ring B is b76".
(B3, carbamoyl, hydrogen) Hereinafter referred to as "ring B is b77".
(B3, carbamoyl, chloro) Hereinafter referred to as "ring B is b78".
(B3, carbamoyl, methoxy) Hereinafter referred to as "ring B is b79".
(B3, carbamoyl, cyano) Hereinafter referred to as "ring B is b80".

The compound of the formula (I) wherein the combination of ring B and R^{1b}, (ring B, R^{1b}) is as follows:
(B4, hydrogen) Hereinafter referred to as "ring B is b81".
(B4, chloro) Hereinafter referred to as "ring B is b82".
(B4, methoxy) Hereinafter referred to as "ring B is b83".
(B4, hydroxymethyl) Hereinafter referred to as "ring B is b84".
(B4, cyano) Hereinafter referred to as "ring B is b85".
(B4, amino) Hereinafter referred to as "ring B is b86".
(B4, carbamoyl) Hereinafter referred to as "ring B is b87".
(B5, hydrogen) Hereinafter referred to as "ring B is b88",
(B5, chloro) Hereinafter referred to as "ring B is b89".
(B5, methoxy) Hereinafter referred to as "ring B is b90".
(B5, hydroxymethyl) Hereinafter referred to as "ring B is b91".
(B5, cyano) Hereinafter referred to as "ring B is b92".
(B5, amino) Hereinafter referred to as "ring B is b93".
(B5, carbamoyl) Hereinafter referred to as "ring B is b94".

The compound of the formula (I) wherein the combination of ring X, R¹, ring A, and ring B (ring X, R¹, ring A, ring B) is as follows and L is L1:
(X1,R11,A1,b1),(X1,R11,A1,b2),(X1,R11,A1,b3),(X1,R11,A1,b4),(X1,R11,A1,b5),(X1,R11, A1,b6),(X1,R11,A1,b7),(X1 ,R11,A1,b8),(X1,R11,A1,b9),(X1,R11,A1,b10),(X1,R11,A1,b11), (X1,R11,A1,b12),(X1,R11,A1,b13),(X1,R11,A1,b14),(X1,R11,A1,b15),(X1,R11,A1,b16),(X1 ,R11,A1,b17),(X1,R11,A1,b18),(X1,R11,A1,b19),(X1,R11,A1,b20),(X1,R11,A1,b21),(X1,R1 1,A1,b22),(X1,R11,A1,b23),(X1,R11,A1,b24),(X1,R11,A1,b25),(X1,R11,A1,b26),(X1,R11,A 1,b27),(X1,R11,A1,b28),(X1,R11,A1,b29),(X1,R11,A1,b30),(X1,R11,A1,b31),(X1,R11,A1,b 32),(X1,R11,A1,b33),(X1,R11,A1,b34),(X1,R11,A1,b35),(X1,R11,A1,b36),(X1,R11,A1,b37) ,(X1,R11,A1,b38),(X1,R11,A1,b39),(X1,R11,A1,b40),(X1,R11,A1,b41),(X1,R11,A1,b42),(X 1,R11,A1,b43),(X1,R11,A1,b44),
(X1,R11,A1,b45),(X1,R11,A1,b46),(X1,R11,A1,b47),(X1,R11,A1,b48),(X1,R11,A1,b49),(X1 ,R11,A1,b50),(X1,R11,A1,b51),(X1,R11,A1,b52),(X1,R11,A1,b53),(X1,R11,A1,b54),(X1,R1 1,A1,b55),(X1,R11,A1,b56),(X1,R11,A1,b57),(X1,R11,A1,b58),(X1,R11,A1,b59),(X1,R11,A 1,b60),(X1,R11,A1,b61),(X1,R11,A1,b62),(X1,R11,A1,b63),(X1,R11,A1,b64),(X1,R11,A1,b 65),(X1,R11,A1,b66),(X1,R11,A1,b67),(X1,R11,A1,b68),(X1,R11,A1,b69),(X1,R11,A1,b70) ,(X1,R11,A1,b71),(X1,R11,A1,b72),(X1,R11,A1,b73),(X1,R11,A1,b74),(X1,R11,A1,b75),(X 1,R11,A1,b76),(X1,R11,A1,b77),(X1,R11,A1,b78),(X1,R11,A1,b79),(X1,R11,A,b80),(X1,R 11,A1,b81),(X1,R11,A1,b82),(X1,R11,A1,b83),(X1,R11,A1,b84),(X1,R11,A1,b85),(X1,R11, A1,b86),(X1,R11,A1,b87),(X1,R11,A1,b88),(X1,R11,A1,b89),(X1,R11,A1,b90),(X1,R11,A1, b91),(X1;R11,A1,b92),(X1,R11,A1,b93),(X1,R11,A1,b94),(X1,R11,A2,b1),(X1,R11,A2,b2), ( X1,R11,A2,b3),(X1,R11,A2,b4),(X1,R11,A2,b5),(X1,R11,A2,b6),(X1,R11,A2,b7),(X1,R11,A 2,b8),(X1,R11,A2,b9),(X1,R11,A2,b10),(X1,R11,A2,b11),(X1,R11,A2,b12),(X1,R11,A2,b13) ,(X1,R11,A2,b14),(X1,R11,A2,b15),(X1,R11,A2,b16),(X1,R11,A2,b17),(X1,R11,A2,b18),(X 1,R11,A2,b19),(X1,R11,A2,b20),(X1,R11,A2,b21),(X1,R11,A2,b22),(X1,R11,A2,b23),(X1,R 11,A2,b24),(X1,R11,A2,b25),(X1,R11,A2,b26),(X1,R11,A2,b27),(X1,R11,A2,b28),(X1,R11, A2,b29),(X,R11,A2,b30),(X1,R11,A2,b31),(X1,R11,A2,b32),(X1,R11,A2,b33),(X1,R11,A2, b34),(X1,R11,A2,b35),(X1,R11,A2,b36),(X1,R11,A2,b37),(X1,R11,A2,b38),(X1,R11,A2,b39 ),(X1,R11,A2,b40),(X1,R11,A2,b41),(X1,R11,A2,b42),(X1,R11,A2,b43),(X1,R11,A2,b44),(X 1,R11,A2,b45),(X1,R11,A2,b46),(X1,R11,A2,b47),(X1,R11,A2,b48),(X1,R11,A2,b49),(X1,R 11,A2,b50),(X1,R11,A2,b51),(X1,R11,A2,b52),(X1,R11,A2,b53),(X1,R11,A2,b54),(X1,R11, A2,b55),(X1,R11,A2,b56),(X1,R11,A2,b57),(X1,R11,A2,b58),
(X1,R11,A2,b59),(X1,R11,A2,b60),(X1,R11,A2,b61),(X1,R11,A2,b62),(X1,R11,A2,b63),(X1 ,R11,A2,b64),(X1,R11,A2,b65),(X1,R11,A2,b66),(X1,R11,A2,b67),(X1,R11,A2,b68),(X1,R1 1,A2,b69),(X1,R11,A2,b70),(X1,R11,A2,b71),(X1,R11,A2,b72),(X1,R11,A2,b73),(X1,R11,A 2,b74),(X1,R11,A2,b75),(X1,R11,A2,b76),(X1,R11,A2,b77),(X1,R11,A2,b78),(X1,R11,A2,b 79),(X1,R11,A2,b80),(X1,R11,A2,b81),(X1,R11,A2,b82),(X1,R11,A2,b83),(X1,R11,A2,b84) ,(X1,R11,A2,b85),(X1,R11,A2,b86),(X1,R11,A2,b87),(X1,R11,A2,b88),(X1,R11,A2,b89),(X 1,R11,A2,b90),(X1,R11,A2,b91),(X1,R11,A2,b92),(X1,R11,A2,b93),(X1,R11,A2,b94),(X1,R 11,A3,b1),(X1,R11,A3,b2),(X1,R11,A3,b3),(X1,R11,A3,b4),(X1,R11,A3,b5),(X1,R11,A3,b6) ,(X1,R11,A3,b7),(X1,R11,A3,b8),(X1,R11,A3,b9),(X1,R11,A3,b10),(X1,R11,A3,b11),(X1,R1 1,A3,b12),(X1,R11,A3,b13),(X1,R11,A3,b14),(X1,R11,A3,b15),(X1,R11,A3,b16),(X1,R11,A 3,b17),(X1,R11,A3,b18),(X1,R11,A3,b19),(X1,R11,A3,b20),(X1,R11,A3,b21),(X1,R11,A3,b 22),(X1,R11,A3,b23),(X1,R11,A3,b24),(X1,R11,A3,b25),(X1,R11,A3,b26),(X1,R11,A3,b27) ,(X1,R11,A3,b28),(X1,R11,A3,b29),(X1,R11,A3,b30),(X1,R11,A3,b31),(X1,R11,A3,b32),(X 1,R11,A3,b33),(X1,R11,A3,b34),(X1,R11,A3,b35),(X1,R11,A3,b36),(X1,R11,A3,b37),(X1,R 11,A3,b38),(X1,R11,A3,b39),(X1,R11,A3,b40),(X1,R11,A3,b41),(X1,R11,A3,b42),(X1,R11, A3,b43),(X1,R11,A3,b44),(X1,R11,A3,b45),(X1,R11,A3,b46),(X1,R11,A3,b47),(X1,R11,A3, b48),(X1,R11,A3,b49),(X1,R11,A3,b50),(X1,R11,A3,b51),(X1,R11,A3,b52),(X1,R11,A3,b53 ),(X1,R11,A3,b54),(X1,R11,A3,b55),(X1,R11,A3,b56),(X1,R11,A3,b57),(X1,R11,A3,b58),(X 1,R11,A3,b59),(X1,R11,A3,b60),(X1,R11,A3,b61),(X1,R11,A3,b62),(X1,R11,A3,b63),(X1,R 11,A3,b64),(X1,R11,A3,b65),(X1,R11,A3,b66),(X1,R11,A3,b67),(X1,R11,A3,b68),(X1,R11, A3,b69),(X1,R11,A3,b70),(X1,R11,A3,b71),(X1,R11,A3,b72),(X1,R11,A3,b73),(X1,R11,A3, b74),(X1,R11,A3,b75),(X1,R11,A3,b76),(X1,R11,A3,b77),(X1,R11,A3,b78),(X1,R11,A3,b79 ),(X1,R11,A3,b80),(X1,R11,A3,b81),(X1,R11,A3,b82),(X1,R11,A3,b83),(X1,R11,A3,b84),(X 1,R11,A3,b85),(X1,R11,A3,b86),(X1,R11,A3,b87),(X1,R11,A3,b88},(X1,R11,A3,b89),(X1,R 11,A3,b90),(X1,R11,A3,b91),(X1,R11,A3,b92),(X1,R11,A3,b93),(X1,R11,A3,b94),(X1,R12, A1,b1),(X1,R12,A1,b2),(X1,R12,A1,b3),(X1,R12,A1,b4),(X1,R12,A1,b5),(X1,R12,A1,b6),( X1,R12,A1,b7),(X1,R12,A1,b8),(X1,R12,A1,b9),(X1,R12,A1,b10),(X1,R12,A1,b11),(X1,R1 2,A1,b12),(X1,R12,A1,b13),(X1,R12,A1,b14),(X1,R12,A1,b15),(X1,R12,A1,b16),(X1,R12, A1,b17),(X1,R12,A1,b18),(X1,R12,A1,b19),(X1,R12,A1,b20),(X1,R12,A1,b21),(X1,R12,A1 ,b22),
(X1,R12,A1,b23),(X1,R12,A1,b24),(X1,R12,A1,b25),(X1,R12,A1,b26),(X1,R12,A1,b27),(X 1,R12,A1,b28),(X1,R12,A1,b29),(X1,R12,A1,b30),(X1,R12,A1,b31),(X1,R12,A1,b32),(X1, R12,A1,b33),(X1,R12,A1,b34),(X1,R12,A1,b35),(X1,R12,A1,b36),(X1,R12,A1,b37),(X1,R1 2,A1,b38),(X1,R12,A1,b39),(X1,R12,A1,b40),(X1,R12,A1,b41),(X1,R12,A1,b42),(X1,R12, A1,b43),(X1,R12,A1,b44),(X1,R12,A1,b45),(X1,R12,A1,b46),(X1,R12,A1,b47),(X1,R12,A1 ,b48),(X1,R12,A1,b49),(X1,R12,A1,b50),(X1,R12,A1,b51),(X1,R12,A1,b52),(X1,R12,A1,b5 3),(X1,R12,A1,b54),(X1,R12,A1,b55),(X1,R12,A1,b56),(X1,R12,A1,b57),(X1,R 12,A1,b58), (X1,R12,A1,b59),(X1,R12,A1,b60),(X1,R12,A1,b61),(X1,R12,A1,b62),(X1,R12,A1,b63),(X 1,R12,A1,b64),(X1,R12,A1,b65),(X1,R12,A1,b66),(X1,R12,A1,b67),(X1,R12,A1,b68),(X1, R12,A1,b69),(X1,R12,A1,b70),(X1,R12,A1,b71),(X1,R12,A1,b72),(X1,R12,A1,b73),(X1,R1 2,A1,b74),(X1,R12,A1,b75),(X1,R12,A1,b76),(X1,R12,A1,b77),(X1,R12,A1,b78),(X1,R12, A1,b79),(X1,R12,A1,b80),(X1,R12,A1,b81),(X1,R12,A1,b82),(X1,R12,A1,b83),(X1,R12,A1 ,b84),(X1,R12,A1,b85),(X1,R12,A1,b86),(X1,R12,A1,b87),(X1,R12,A1,b88),(X1,R12,A1,b8 9),(X1,R12,A1,b90),(X1,R12,A1,b91),(X1,R12,A1,b92),(X1,R12,A1,b93),(X1,R12,A1,b94), (X1,R12,A2,b1),(X1,R12,A2,b2),(X1,R12,A2,b3),(X1,R12,A2,b4),(X1,R12,A2,b5),(X1,R12, A2,b6),(X1,R12,A2,b7),(X1,R12,A2,b8),(X1,R12,A2,b9),(X1,R12,A2,b10),(X1,R12,A2,b11) ,(X1,R12,A2,b12),(X1,R12,A2,b13),(X1,R12,A2,b14),(X1,R12,A2,b15),(X1,R12,A2,b16),(X 1,R12,A2,b17),(X1,R12,A2,b18),(X1,R12,A2,b19),(X1,R12,A2,b20),(X1,R12,A2,b21),(X1, R12,A2,b22),(X1,R12,A2,b23),(X1,R12,A2,b24),(X1,R12,A2,b25),(X1,R12,A2,b26),(X1,R1 2,A2,b27),(X1,R12,A2,b28),(X1,R12,A2,b29),(X1,R12,A2,b30),(X1,R12,A2,b31),(X1,R12, A2,b32),(X1,R12,A2,b33),(X1,R12,A2,b34),(X1,R12,A2,b35),(X1,R12,A2,b36),(X1,R12,A2 ,b37),(X1,R12,A2,b38),(X1,R12,A2,b39),(X1,R12,A2,b40),(X1,R12,A2,b41),(X1,R12,A2,b4 2),(X1,R12,A2,b43),(X1,R12,A2,b44),(X1,R12,A2,b45),(X1,R12,A2,b46),(X1,R12,A2,b47), (X1,R12,A2,b48),(X1,R12,A2,b49),(X1,R12,A2,b50),(X1,R12,A2,b51),(X1,R12,A2,b52),(X 1,R12,A2,b53),(X1,R12,A2,b54),(X1,R12,A2,b55),(X1,R12,A2,b56),(X1,R12,A2,b57),(X1, R12,A2,b58),(X1,R12,A2,b59),(X1,R12,A2,b60),(X1,R12,A2,b61),(X1,R12,A2,b62),(X1,R1 2,A2,b63),(X1,R12,A2,b64),(X1,R12,A2,b65),(X1,R12,A2,b66),(X1,R12,A2,b67),(X1,R12, A2,b68),(X1,R12,A2,b69),(X1,R12,A2,b70),(X1,R12,A2,b71),(X1,R12,A2,b72),(X1,R12,A2 ,b73),(X1,R12,A2,b74),(X1,R12,A2,b75),(X1,R12,A2,b76),(X1,R12,A2,b77),(X1,R12,A2,b78 8),(X1,R12,A2,b79),(X1,R12,A2,b80),(X1,R12,A2,b81),(X1,R12,A2,b82),(X1,R12,A2,b83), (X1,R12,A2,b84),(X1,R12,A2,b85),(X1,R12,A2,b86),(X1,R12,A2,b87),(X1,R12,A2,b88),(X 1,R12,A2,b89),(X1,R12,A2,b90),(X1,R12,A2,b91),(X1,R12,A2,b92),(X1,R12,A2,b93),(X1, R12,A2,b94),(X1,R12,A3,b1),(X1,R12,A3,b2),(X1,R12,A3,b3),(X1,R12,A3,b4),(X1,R12,A3 ,b5),(X1,R12,A3,b6),(X1,R12,A3,b7),(X1,R12,A3,b8),(X1,R12,A3,b9),(X1,R12,A3,b10),(X1 ,R12,A3,b11),(X1,R12,A3,b12),(X1,R12,A3,b13),(X1,R12,A3,b14),(X1,R12,A3,b15),(X1,R 12,A3,b16),(X1,R12,A3,b17),(X1,R12,A3,b18),(X1,R12,A3,b19),(X1,R 12,A3,b20),(X1,R12; A3,b21),(X1,R12,A3,b22),(X1,R12,A3,b23),(X1,R12,A3,b24),(X1,R12,A3,b25),(X1,R12,A3 ,b26),(X1,R12,A3,b27),(X1,R12,A3,b28),(X1,R12,A3,b29),(X1,R12,A3,b30),(X1,R12,A3,b3 1),(X1,R12,A3,b32),(X1,R12,A3,b33),(X1,R12,A3,b34),(X1,R12,A3,b35),(X1,R12,A3,b36), (X1,R12,A3,b37),(X1,R12,A3,b38),(X1,R12,A3,b39),(X1,R12,A3,b40),(X1,R12,A3,b41),(X 1,R12,A3,b42),(X1,R12,A3,b43),(X1,R12,A3,b44),(X1,R12,A3,b45),(X1,R12,A3,b46),(X1, R12,A3,b47),(X1,R12,A3,b48),(X1,R12,A3,b49),(X1,R12,A3,b50), (X1,R12,A3,b51),(X1,R1 2,A3,b52),(X1,R12,A3,b53),(X1,R12,A3,b54),(X1,R12,A3,b55),(X1,R12,A3,b56),(X1,R12, A3,b57),(X1,R12,A3,b58),
(X1,R12,A3,b59),(X1,R12,A3,b60),(X1,R12,A3,b61),(X1,R12,A3,b62),(X1,R12,A3,b63),(X 1,R12,A3,b64),(X1,R12,A3,b65),(X1,R12,A3,b66),(X1,R12,A3,b67),(X1,R12,A3,b68),(X1, R12,A3,b69),(X1,R12,A3,b70),(X1,R12,A3,b71),(X1,R2,A3,b72),(X1,R12,A3,b73),(X1,R1 2,A3,b74),(X1,R12,A3,b75),(X1,R12,A3,b76),(X1,R12,A3,b77),(X1,R12,A3,b78),(X1,R12, A3,b79),(X1,R12,A3,b80),(X1,R12,A3,b81),(X1,R12,A3,b82),(X1,R12,A3,b83),(X1,R12,A3 ,b84),(X1,R12,A3,b85),(X1,R12,A3,b86),(X1,R12,A3,b87),(X1,R12,A3,b88),(X1,R12,A3,b8 9),(X1,R12,A3,b90),(X1,R12,A3,b91),(X1,R12,A3,b92),(X1,R12,A3,b93),(X1,R12,A3,b94), (X2,R11,A1,b1),(X2,R11,A1,b2),(X2,R11,A1,b3),(X2,R11,A1,b4),(X2,R11,A1,b5),(X2,R11, A1,b6),(X2,R11,A1,b7),(X2,R11,A1,b8),(X2,R11,A1,b9),(X2,R11,A1,b10),(X2,R11,A1,b11), (X2,R11,A1,b12),(X2,R11,A1,b13),(X2,R11,A1,b14),(X2,R11,Al,b15),(X2,R11,A1,b16),(X2 ,R11,A1,b17),(X2,R11,A1,b18),(X2,R11,A1,b19),(X2,R11,A1,b20),(X2,R11,A1,b21),(X2,R1 1,A1,b22),(X2,R11,A1,b23),(X2,R11,A1,b24),(X2,R11,A1,b25),(X2,R11,A1,b26),(X2,R11,A 1,b27),(X2,R11,A1,b28),(X2,R11,A1,b29),(X2,R11,A1,b30),(X2,R11,A1,b31),(X2,R11,A1,b 32),(X2,R11,A1,b33),(X2,R11,A1,b34),(X2,R11,A1,b35),(X2,R11,A1,b36),(X2,R11,A1,b37) ,(X2,R11,A1,b38),(X2,R11,A1,b39),(X2,R11,A1,b40),(X2,R11,A1,b41),(X2,R11,A1,b42),(X 2,R11,A1,b43),(X2,R11,A1,b44),(X2,R11,A1,b45),(X2,R11,A1,b46),( X2,R11,A1,b47),(X2,R 11,A1,b48),(X2,R11,A1,b49),(X2,R11,A1,b50),(X2,R11,A1,b51),(X2,R11,A1,b52),(X2,R11, A1,b53),(X2,R11,A1,b54),(X2,R11,A1,b55),(X2,R11,A1,b56),(X2,R11,A1,b57),(X2,R11,A1, b58),(X2,R11,A1,b59),(X2,R11,A1,b60),(X2,R11,A1,b61),(X2,R11,A1,b62),(X2,R11,A1,b63 ),(X2,R11,A1,b64),(X2,R11,A1,b65),(X2,R11,A1,b66),(X2,R11,A1,b67),(X2,R11,A1,b68),(X 2,R11,A1,b69),(X2,R11,A1,b70),(X2,R11,A1,b71),(X2,R11,A1,b72),(X2,R11,A1,b73),(X2,R 11,A1,b74),(X2,R11,A1,b75),(X2,R11,A1,b76),
(X2,R11,A1,b77),(X2,R11,A1,b78),(X2,R11,A1,b79),(X2,R11,A1,b80),(X2,R11,A1,b81),(X2 ,R11,A1,b82),(X2,R11,A1,b83),(X2,R11,A1,b84),(X2,R11,A1,b85),(X2,R11,A1,b86),(X2,R1 1,A1,b87),(X2,R11,A1,b88),(X2,R11,A1,b89),(X2,R11,A1,b90),(X2,R11,A1,b91),(X2,R11,A 1,b92),(X2,R11,A1,b93),(X2,R11,A1,b94),(X2,R11,A2,b1),(X2,R11,A2,b2),(X2,R11,A2,b3), (X2,R11,A2,b4),(X2,R11,A2,b5),(X2,R11,A2,b6),(X2,R11,A2,b7),(X2,R11,A2,b8),(X2,R11, A2,b9),(X2,R11,A2,b10),(X2,R11,A2,b11),(X2,R11,A2,b12),(X2,R11,A2,b13),(X2,R11,A2,b 14),(X2,R11,A2,b15),(X2,R11,A2,b16),(X2,R11,A2,b17),(X2,R11,A2,b18),(X2,R11,A2,b19) ,(X2,R11,A2,b20),(X2,R11,A2,b21),(X2,R11,A2,b22),(X2,R11,A2,b23),(X2,R11,A2,b24),(X 2,R11,A2,b25),(X2,R11,A2,b26),(X2,R11,A2,b27),(X2,R11,A2,b28),(X2,R11,A2,b29),(X2,R 11,A2,b30),(X2,R11,A2,b31),(X2,R11,A2,b32),(X2,R11,A2,b33),(X2,R11,A2,b34) ,(X2,R11, A2,b35),(X2,R11,A2,b36),(X2,R11,A2,b37),(X2,R11,A2,b38),(X2,R11,A2,b39),(X2,R11,A2, b40),(X2,R11,A2,b41),(X2,R11,A2,b42),(X2,R11,A2,b43),(X2,R11,A2,b44),(X2,R11,A2,b45 ),(X2,R11,A2,b46),(X2,R11,A2,b47),(X2,R11,A2,b48),(X2,R11,A2,b49),(X2,R11,A2,b50),(X 2,R11,A2,b51),(X2,R11,A2,b52),(X2,R11,A2,b53),(X2,R11,A2,b54),(X2,R11,A2,b55),(X2,R 11,A2,b56),(X2,R11,A2,b57),(X2,R11,A2,b58),(X2,R11,A2,b59),(X2,R11,A2,b60),(X2,R11, A2,b61),(X2,R11,A2,b62),(X2,R11,A2,b63),(X2,R11,A2,b64),(X2,R11,A2,b65),(X2,R11,A2, b66),(X2,R11,A2,b67),(X2,R11,A2,b68),(X2,R11,A2,b69),(X2,R11,A2,b70),(X2,R11,A2,b71 ),(X2,R11,A2,b72),(X2,R11,A2,b73),(X2,R11,A2,b74),(X2,R11,A2,b75),(X2,R11,A2,b76),(X 2,R11,A2,b17),(X2,R11,A2,b78),(X2,R11,A2,b79),(X2,R11,A2,b80),(X2,R11,A2,b81),(X2,R 11,A2,b82),(X2,R11,A2,b83),(X2,R11,A2,b84),(X2,R11,A2,b85),(X2,R11,A2,b86),(X2,R11, A2,b87),(X2,R11,A2,b88),(X2,R11,A2,b89),(X2,R11,A2,b90),
(X2,R11,A2,b91),(X2,R11,A2,b92),(X2,R11,A2,b93),(X2,R11,A2,b94),(X2,R11,A3,b1),(X2, R11,A8,b2),(X2,R11,A3,b3),(X2,R11,A3,b4),(X2,R11,A3,b5),(X2,R11,A3,b6),(X2,R11,A3,b 7),(X2,R11,A8,b8),(X2,R11,A8,b9),(X2,R11,A3,b10),(X2,R11,A3,b11),(X2,R11,A3,b12),(X2 ,R11,A3,b13),(X2,R11,A3,b14),(X2,R11,A3,b15),(X2,R11,A3,b16),(X2,R11,A3,b 17),(X2,R1 1,A3,b18),(X2,R11,A3,b19),(X2,R11,A3,b20),(X2,R11,A3,b21),(X2,R11,A3,b22),(X2,R11,A 3,b23),(X2,R11,A3,b24),(X2,R11,A3,b25),(X2,R11,A3,b26),(X2,R11,A3,b27),(X2,R11,A8,b 28),(X2,R11,A3,b29),(X2,R11,A3,b30),(X2,R11,A3,b31),(X2,R11,A3,b32),(X2,R11,A3,b33) ,(X2,R11,A3,b34),(X2,R11,A3,b35),(X2,R11,A3,b34),(X2,R11,A8,b37),(X2,R11,A3,b38),(X 2,R11,A8,b39),(X2,R11,A3,b40),(X2,R11,A3,b41),(X2,R11,A3,b42),(X2,R11,A3,b43);(X2,R 11,A3,b44),(X2,R11,A3,b45),(X2,R11,A3,b46),(X2,R11,A3,b47),(X2,R11,A3,b48),(X2,R11, A3,b49),(X2,R11,A3,b50),(X2,R11,A3,b51),(X2,R11,A3,b52),(X2,R11,A3,b53),(X2,R11,A3, b54),(X2,R11,A3,b55),(X2,R11,A3,b56),(X2,R11,A3,b57),(X2,R11,A3,b58),(X2,R11,A3,b59 ),(X2,R11,A3,b60),(X2,R11,A3,b61),(X2,R11,A3,b62),(X2,R11,A8,b63),(X2,R11,A8,b64),(X 2,R11,A3,b65),(X2,R11,A3,b66),(X2,R11,A3,b67),(X2,R11,A3,b68),(X2,R11,A3,b69),(X2,R 11,A3,b70),(X2,R11,A3,b71),(X2,R11,A3,b72),(X2,R11,A3,b73),(X2,R11,A3,b74),(X2,R11, A3,b75),(X2,R11,A3,b76),(X2,R11,A3,b77),(X2,R11,A3,b78),(X2,R11,A3,b79),(X2,R11,A3, b80),(X2,R11,A3,b81),(X2,R11,A3,b82),(X2,R11,A3,b83),(X2,R11,A3,b84),(X2,R11,A3,b85 ),(X2,R11,A3,b86),(X2,R11,A3,b87),(X2,R11,A3,b88),(X2,R11,A3,b89),(X2,R11,A3,b90),(X 2,R11,A3,b91),(X2,R11,A3,b92),(X2,R11,A3,b93),(X2,R11,A3,b94),(X2,R12,A1,b1),(X2,R1 2,A1,b2),(X2,R12,A1,b3),(X2,R12,A1,b4),(X2,R12,A1,b5),(X2,R12,A1,b6),(X2,R12,A1,b7), (X2,R12,A1,b8),(X2,R12,A1,b9),(X2,R12,A1,b10),(X2,R12,A1,b11),(X2,R12,A1,b12),(X2, R12,A1,b13),(X2,R12,A1,b14),(X2,R12,A1,b15),(X2,R12,A1,b16),(X2,R12,A1,b17),(X2,R1 2,A1,b18),(X2,R12,A1,b19),(X2,R12,A1,b20),(X2,R12,Al,b21),(X2,R12,A1,b22),(X2,R12, A1,b23),(X2,R12,A1,b24),(X2,R12,A1,b25),(X2,R12,A1,b26),(X2,R12,A1,b27),(X2,R12,A1 ,b28),(X2,R12,A1,b29),(X2,R12,A1,b30),(X2,R12,A1,b31),(X2,R12,A1,b32),(X2,R12,A1,b3 3),(X2,R12,A1,b34),(X2,R12,A1,b35),(X2,R12,A1,b36),(X2,R12,A1,b37),(X2,R12,A1,b38), (X2,R12,A1,b39),(X2,R12,A1,b40),(X2,R12,A1,b41),(X2,R12,A1,b42),(X2,R12,A1,b43),(X 2,R12,A1,b44),(X2,R12,A1,b45),(X2,R12,A1,b46),
(X2,R12,A1,b47),(X2,R12,A1,b48),(X2,R12,A1,b49),(X2,R12,A1,b50),(X2,R12,A1,b51),(X 2,R12,A1,b52),(X2,R12,A1,b53),(X2,R12,A1,b54),(X2,R12,A1,b55),(X2,R12,A1,b56),(X2, R12,A1,b57),(X2,R12,A1,b58),(X2,R12,A1,b59),(X2,R12,A1,b60),(X2,R12,A1,b61),(X2,R1 2,A1,b62),(X2,R12,A1,b63),(X2,R12,A1,b64),(X2,R12,A1,b65),(X2,R12,A1,b66),(X2,R12, A1,b67),(X2,R12,A1,b68),(X2,R12,A1,b69),(X2,R12,A1,b70),(X2,R12,A1,b71),(X2,R12,A1 ,b72),(X2,R12,A1,b73),(X2,R12,A1,b74),(X2,R12,A1,b75),(X2,R12,A1,b76),(X2,R12,A1,b7 7),(X2,R12,A1,b78),(X2,R12,A1,b79),(X2,R12,A1,b80),(X2,R12,A1,b81),(X2,R12,A1,b82), (X2,R12,A1,b83),(X2,R12,A1,b84),(X2,R12,A1,b85),(X2,R12,A1,b86),(X2,R12,A1,b87),(X 2,R12,A1,b88),(X2,R12,A1,b89),(X2,R12,A1,b90),(X2,R12,A1,b91),(X2,R12,A1,b92),(X2, R12,A1,b93),(X2,R12,A1,b94),(X2,R12,A2,b1),(X2,R12,A2,b2),(X2,R12,A2,b3),(X2,R12,A 2,b4);(X2,R12,A2,b5),(X2,R12,A2,b6),(X2,R12,A2,b7),(X2,R12,A2,b8),(X2,R12,A2,b9),(X2 ,R12,A2,b10),(X2,R12,A2,b11),(X2,R12,A2,b12),(X2,R12,A2,b13),(X2,R12,A2,b14),(X2,R 12,A2,b15),(X2,R12,A2,b16),(X2,R12,A2,b17),(X2,R12,A2,b18),(X2,R12,b19),(X2,R12, A2,b20),(X2,R12,A2,b21),(X2,R12,A2,b22),(X2,R12,A2,b23),(X2,R12,A2,b24),(X2,R12,A2 ,b25),(X2,R12,A2,b26),(X2,R12,A2,b27),(X2,R12,A2,b28),(X2,R12,A2,b29),(X2,R12,A2,b3 0),(X2,R12,A2,b31),(X2,R12,A2,b32),(X2,R12,A2,b33),(X2,R12,A2,b34),(X2,R12,A2,b35), (X2,R12,A2,b36),(X2,R12,A2,b37),(X2,R12,A2,b38),(X2,R12,A2,b39),(X2,R12,A2,b40),(X 2,R12,A2,b41),(X2,R12,A2,b42),(X2,R12,A2,b43),(X2,R12,A2,b44),(X2,R12,A2,b45),(X2, R12,A2,b46),(X2,R12,A2,b47),(X2,R12,A2,b48),(X2,R12,A2,b49),(X2,R12,A2,b50),(X2,R1 2,A2,b51),(X2,R12,A2,b52),(X2,R12,A2,b53),(X2,R12,A2,b54),(X2,R12,A2,b55),(X2,R12, A2,b56),(X2,R12,A2,b57),(X2,R12,A2,b58),(X2,R12,A2,b59),(X2,R12,A2,b60),(X2,R12,A2 ,b61),(X2,R12,A2,b62),(X2,R12,A2,b63),(X2,R12,A2,b64),(X2,R12,A2,b65),(X2,R12,A2,b6 6),(X2,R12,A2,b67),(X2,R12,A2,b68),(X2,R12,A2,b69),(X2,R12,A2,b70),(X2,R12,A2,b71), (X2,R12,A2,b72),
(X2,R12,A2,b73),(X2,R12,A2,b74),(X2,R12,A2,b75),(X2,R12,A2,b76),(X2,R12,A2,b77),(X 2,R12,A2,b78),(X2,R12,A2,b79),(X2,R12,A2,b80),(X2,R12,A2,b81),(X2,R12,A2,b82),(X2, R12,A2,b83),(X2,R12,A2,b84),(X2,R12,A2,b85),(X2,R12,A2,b86),(X2,R12,A2,b87),(X2,R1 2,A2,b88),(X2,R12,A2,b89),(X2,R12,A2,b90),(X2,R12,A2,b91),(X2,R12,A2,b92),(X2,R12, A2,b93),(X2,R12,A2,b94),(X2,R12,A3,b1),(X2,R12,A3,b2),(X2,R12,A3,b3),(X2,R12,A3,b4) ,(X2,R12,A3,b5),(X2,R12,A3,b6),(X2,R12,A3,b7),(X2,R12,A3,b8),(X2,R12,A3,b9),(X2,R12 ,A3,b10),(X2,R12,A3,b11),(X2,R12,A3,b12),(X2,R12,A3,b13),(X2,R12,A,b14),(X2,R12,A 3,b15),(X2,R12,A3,b16),(X2,R12,A3,b17),(X2,R12,A3,b18),(X2,R12,A3,b19),(X2,R12,A3,b 20),(X2,R12,A3,b21),(X2,R12,A3,b22),(X2,R12,A3,b23),(X2,R12,A3,b24),(X2,R12,A3,b25 ),(X2,R12,A3,b26),(X2,R12,A3,b27),(X2,R12,A3,b28),(X2,R12,A3,b29),(X2,R12,A3,b30),( X2,R12,A3,b31),(X2,R12,A3,b32),(X2,R12,A3,b33),(X2,R12,A3,b34),(X2,R12,A3,b35),(X2 ,R12,A3,b36),(X2,R12,A3,b37),(X2,R12,A3,b38),(X2,R12,A3,b39),(X2,R12,A3,b40),(X2,R 12,A3,b41),(X2,R12,A3,b42),(X2,R12,A3,b43),(X2,R12,A3,b44),(X2,R12,A3,b45),(X2,R12, A3,b46),(X2,R12,A3,b47),(X2,R12,A3,b48),(X2,R12,A3,b49),(X2,R12,A3,b50),(X2,R12,A3 ,b51),(X2,R12,A3,b52),(X2,R12,A3,b53),(X2,R12,A3,b54),(X2,R12,A3,b55),(X2,R12,A3,b5 6),(X2,R12,A3,b57),(X2,R12,A3,b58),(X2,R12,A3,b59),(X2,R12,A3,b60),(X2,R12,A3,b61), (X2,R12,A3,b62),(X2,R12,A3,b63),(X2,R12,A3,b64),(X2,R12,A3,b65),(X2,R12,A3,b66),(X 2,R12,A3,b67),(X2,R12,A3,b68),(X2,R12,A3,b69),(X2,R12,A3,b70),(X2,R12,A3,b71),(X2, R12,A3,b72),(X2,R12,A3,b73),(X2,R12,A3,b74),(X2,R12,A3,b75),(X2,R12,A3,b76),(X2,R1 2,A3,b77),(X2,R12,A3,b78),(X2,R12,A3,b79),(X2,R12,A3,b80),(X2,R12,A3,b81),(X2,R12, A3,b82),(X2,R12,A3,b83),(X2,R12,A3,b84),(X2,R12,A3,b85),(X2,R12,A3,b86),(X2,R12,A3 ,b87),(X2,R12,A3,b88),(X2,R12,A3,b89),(X2,R12,A3,b90),
(X2,R12,A3,b91),(X2,R12,A3,b92),(X2,R12,A3,b93),(X2,R12,A3,b94),(X3,R11,A1,b1),(X3, R11,A1,b2),(X3,R11,A1,b3),(X3,R11,A1,b4),(X3,R11,A1,b5),(X3,R11,A1,b6),(X3,R11,A1,b 7),(X3,R11,A1,b8),(X3,R11,A1,b9),(X3,R11,A1,b10),(X3,R11,A1,b11),(X3,R11,A1,b12),(X3 ,R11,A1,b13),(X3,R11,A1,b14),(X3,R11,A1,b15),(X3,R11,A1,b16),(X3,R11,A1,b17),(X3,R1 1,A1,b18),(X3,R11,A1,b19),(X3,R11,A1,b20),(X3,R11,A1,b21),(X3,R11,A1,b22),(X3,R11,A 1,b23),(X3,R11,A1,b24),(X3,R11,A1,b25),(X3,R11,A1,b26),(X3,R11,A1,b27),(X3,R11,A1,b 28),(X3,R11,A1,b29),(X3,R11,A1,b30),(X3,R11,A1,b31),(X3,R11,A1,b32),(X3,R11,A1,b33) ,(X3,R11,A1,b34),(X3,R11,A1,b35),(X3,R11,A1,b36),
(X3,R11,A1,b37),(X3,R11,A1,b38),(X3,R11,A1,b39),(X3,R11,A1,b40),(X3,R11,A1,b41),(X3 ,R11,A1,b42),(X3,R11,A1,b43),(X3,R11,A1,b44),(X3,R11,A1,b45),(X3,R11,A1,b48),(X3,R1 1,A1,b47),(X3,R11,A1,b48),(X3,R11,A1,b49),(X3,R11,A1,b50),(X3,R11,A1,b51),(X3,R11,A 1,b52),(X3,R11,A1,b53),(X3,R11,A1,b54),(X3,R11,A1,b55),(X3,R11,A1,b56),(X3,R11,A1,b 57),(X3,R11,A1,b58),(X3,R11,A1,b59),(X3,R11,A1,b60),(X3,R11,A1,b61),(X3,R11,A1,b62) ,(X3,R11,A1,b63),(X3,R11,A1,b64),(X3,R11,A1,b65),(X3,R11,A1,b66),(X3,R11,A1,b67),(X 3_{,}R11,A1,b68),(X3,R11,A1,b69),(X3,R11,A1,b70),(X3,R11,A1,b71),(X3,R11,A1,b72),(X3,R 11,A1,b73),(X3,R11,A1,b74),(X3,R11,A1,b75),(X3,R11,A1,b76),(X3,R11,A1,b77),(X3,R11, A1,b78),(X3,R11,A1,b79),(X3,R11,A1,b80),(X3,R11,A1,b81),(X3,R11,A1,b82),(X3,R11,A1, b83),(X3,R11,A1,b84),(X3,R11,A1,b85),(X3,R11,A1,b86),(X3,R11,A1,b87),(X3,R11,A1,b88 ),(X3,R11,A1,b89),(X3,R11,A1,b90),(X3,R11,A1,b91),(X3,R11,A1,b92),(X3,R11,A1,b93),(X 3,R11,A1,b94),(X3,R11,A2,b1),(X3,R11,A2,b2),(X3,R11,A2,b3),(X3,R11,A2,b4),(X3,R11,A 2,b5),(X3,R11,A2,b6),(X3,R11,A2,b7),(X3,R11,A2,b8),(X3,R11,A2,b9),(X3,R11,A2,b10),(X 3,R11,A2,b11),(X3,R11,A2,b12),(X3,R11,A2,b13),(X3,R11,A2,b14),(X3,R11,A2,b15),(X3,R 11,A2,b16),(X3,R11,A2,b17),(X3,R11,A2,b18),(X3,R11,A2,b19),(X3;R11,A2,b20),(X3,R11, A2,b21),(X3,R11,A2,b22),(X3,R11,A2,b23),(X3,R11,A2,b24),(X3,R14,A2,b25),(X3,R11,A2, b26),(X3,R11,A2,b27),(X3,R11,A2,b28),(X3,R11,A2,b29),(X3,R11,A2,b30),(X3,R11,A2,b31 ),(X3,R11,A2,b32),(X3,R11,A2,b33),(X3,R11,A2,b34),(X3,R11,A2,b35),(X3,R11,A2,b36),(X 3,R11,A2,b37),(X3,R11,A2,b38),(X3,R11,A2,b39),(X3,R11,A2,b40),(X3,R11,A2,b41),(X3,R 11,A2,b42),(X3,R11,A2,b43),(X3,R11,A2,b44),(X3,R11,A2,b45),(X3,R11,A2,b46),(X3,R11, A2,b47),(X3,R11,A2,b48),(X3,R11,A2,b49),(X3,R11,A2,b50),(X3,R11,A2,b51),(X3,R11,A2, b52),(X3,R11,A2,b53),(X3,R11,A2,b54),
(X3,R11,A2,b55),(X3,R11,A2,b56),(X3,R11,A2,b57),(X3,R11,A2,b58),(X3,R11,A2,b59),(X3 ,R11,A2,b60),(X3,R11,A2,b61),(X3,R11,A2,b62),(X3,R11,A2,b63),(X3,R11,A2,b64),(X3,R1 1,A2,b65),(X3,R11,A2,b66),(X3,R11,A2,b67),(X3,R11,A2,b68),(X3,R11,A2,b69),(X3,R41,A 2,b70),(X3,R11,A2,b71),(X3,R11,A2,b72),(X3,R11,A2,b73),(X3,R11,A2,b74),(X3,R11,A2,b 75),(X3,R11,A2,b76),(X3,R11,A2,b77),(X3,R11,A2,b78),(X3,R11,A2,b79),(X3,R11,A2,b80) ,(X3,R11,A2,b81),(X3,R11,A2,b82),(X3,R11,A2,b83),(X3,R11,A2,b84),(X3,R11,A2,b85),(X 3,R11,A2,b86),(X3,R11,A2,b87),(X3,R11,A2,b88),(X3,R11,A2,b89),(X3,R11,A2,b90),(X3,R 11,A2,b91),(X3,R11,A2,b92),(X3,R11,A2,b93),(X3,R11,A2,b94),(X3,R11,A3,b1),(X3,R11,A 3,b2),(X3,R11,A3,b3),(X3,R11,A3,b4),(X3,R11,A3,b5),(X3,R11,A3,b6),(X3,R11,A3,b7),(X3, R11,A3,b8),(X3,R11,A3,b9),(X3,R11,A3,b10),(X3,R11,A3,b11),(X3,R11,A3,b12),(X3,R11,A 3,b13),(X3,R11,A3,b14),(X3,R11,A3,b15),(X3,R11,A3,b16),(X3,R11,A3,b17),(X3,R11,A3,b 18),(X3,R11,A3,b19),(X3,R11,A3,b20),(X3,R11,A3,b21),(X3,R11,A3,b22),(X3,R11,A3,b23) ,(X3,R11,A3,b24),(X3,R11,A3,b25),(X3,R11,A3,b26),(X3,R11,A3,b27),(X3,R11,A3,b28),(X 3,R11,A3,b29),(X3,R11,A3,b30),(X3,R11,A3,b31),(X3,R11,A3,b32),(X3,R11,A3,b33),(X3,R 11,A3,b34),(X3,R11,A3,b35),(X3,R11,A3,b36),(X3,R11,A3,b37),(X3,R11,A3,b38),(X3,R11, A3,b39),(X3,R11,A3,b40),(X3,R11,A3,b41),(X3,R11,A3,b42),(X3,R11,A3,b43),(X3,R11,A3, b44),(X3,R11,A3,b45),(X3,R11,A3,b46),(X3,R11,A3,b47),(X3,R11,A3,b48),(X3,R11,A3,b49 ),(X3,R11,A3,b50),(X3,R11,A3,b51),(X3,R11,A3,b52),(X3,R11,A3,b53),(X3,R11,A3,b54),(X 3,R11,A3,b55),(X3,R11,A3,b56),(X3,R11,A3,b57),(X3,R11,A3,b58),(X3,R11,A3,b59),(X3,R 11,A3,b60),(X3,R11,A3,b61),(X3,R11,A3,b62),(X3,R11,A3,b63),(X3,R11,A3,b64),(X3,R11, A3,b65),(X3,R11,A3,b66),(X3,R11,A3,b67),(X3,R11,A3,b68),(X3,R11,A3,b69),(X3,R11,A3, b70),(X3,R11,A3,b71),(X3,R11,A3,b72),
(X3,R11,A3,b73),(X3,R11,A3,b74),(X3,R11,A3,b75),(X3,R11,A3,b76),(X3,R11,A3,b77),(X3 ,R11,A3,b78),(X3,R11,A3,b79),(X3,R11,A3,b80),(X3,R11,A3,b81),(X3,R11,A3,b82),(X3,R1 1,A3,b83),(X3,R11,A3,b84),(X3,R11,A3,b85),(X3,R11,A3,b86),(X3,R11,A3,b87),(X3,R11,A 3,b88),(X3,R11,A3,b89),(X3,R11,A3,b90),(X3,R11,A3,b91),(X3,R11,A3,b92),(X3,R11,A3,b 93),(X3,R11,A3,b94),(X3,R12,A1,b1),(X3,R12,A1,b2),(X3,R12,A1,b3),(X3,R12,A1,b4),(X3, R12,A1,b5),(X3,R12,A1,b6),(X3,R12,A1,b7),(X3,R12,A1,b8),(X3,R12,A1,b9),(X3,R12,A1, b10),(X3,R12,A1,b11),(X3,R12,A1,b12),(X3,R12,A1,b13),(X3,R12,A1,b14),(X3,R 12,A1,b1 5),(X3,R12,A1,b16),(X3,R12,A1,b17),(X3,R12,A1,b18),(X3,R12,A1,b19),(X3,R12,A1,b20), (X3,R12,A1,b21),(X3,R12,A1,b22),(X3,R12,A1,b23),(X3,R12,A1,b24),(X3,R12,A1,b25),(X 3,R12,A1,b26),(X3,R12,A1,b27),(X3,R12,A1,b28),(X3,R12,A1,b29),(X3,R12,A1,b30),(X3, R12,A1,b31),(X3,R12,A1,b32),(X3,R12,A1,b33),(X3,R12,A1,b34),(X3,R12,A1,b35),(X3,R1 2,A1,b36),(X3,R12,A1,b37),(X3,R12,A1,b38),(X3,R12,A1,b39),(X3,R12,A1,b40),(X3,R12, A1,b41),(X3,R12,A1,b42),(X3,R12,A1,b43),(X3,R12,A1,b44),(X3,R12,A1,b45),(X3,R12,A1 ,b46),(X3,R12,A1,b47),(X3,R12,A1,b48),(X3,R12,A1,b49),(X3,R12,A1,b50),(X3,R12,A1,b5 1),(X3,R12,A1,b52),(X3,R12,A1,b53),(X3,R12,A1,b54),(X3,R12,A1,b55),(X3,R12,A1,b56), (X3,R12,A1,b57),(X3,R12,A1,b58),(X3,R12,A1,b59),(X3,R12,A1,b60),(X3,R12,A1,b61),(X 3,R12,A1,b62),(X3,R12,A1,b63),(X3,R12,A1,b64),(X3,R12,A1,b65),(X3,R12,A1,b66),(X3, R12,A1,b67),(X3,R12,A1,b68),(X3,R12,A1,b69),(X3,R12,A1,b70),(X3,R12,A1,b71),(X3,R1 2,A1,b72),(X3,R12,A1,b73),(X3,R12,A1,b74),(X3,R12,A1,b75),(X3,R12,A1,b76),(X3,R12, A1,b77),(X3,R12,A1,b78),(X3,R12,A1,b79),(X3,R12,A1,b80),(X3,R12,A1,b81),(X3,R12,A1 ,b82),(X3,R12,A1,b83),(X3,R12,A1,b84},(X3,R12,A1,b85),(X3,R12,A1,b86),
(X3,R12,A1,b87),(X3,R12,A1,b88),(X3,R12,A1,b89),(X3,R12,A1,b90),(X3,R12,A1,b91),(X 3,R12,A1,b92),(X3,R12,A1,b93),(X3,R12,A1,b94.),(X3,R12,A2,b1),(X3,R12,A2,b2),(X3,R1 2,A2,b3),(X3,R12,A2,b4),(X3,R12,A2,b5),(X3,R12,A2,b6),(X3,R12,A2,b7),(X3,R12,A2,b8), (X3,R12,A2,b9),(X3,R12,A2,b10),(X3,R12,A2,b11),(X3,R12,A2,b12),(X3,R12,A2,b13),(X3, R12,A2,b14),(X3,R12,A2,b15),(X3,R12,A2,b16),(X3,R12,A2,b17),(X3,R12,A2,b18),(X3,R1 2,A2,b19),(X3,R12,A2,b20),(X3,R12,A2,b21),(X3,R12,A2,b22),(X3,R12,A2,b23),(X3,R12, A2,b24),(X3,R12,A2,b25),(X3,R12,A2,b26),(X3,R12,A2,b27),(X3,R12,A2,b28),(X3,R12,A2 ,b29),(X3,R12,A2,b30),(X3,R12,A2,b31),(X3,R12,A2,b32),(X3,R12,A2,b33),(X3,R12,A2,b3 4),(X3,R12,A2,b35),(X3,R12,A2,b36),
(X3,R12,A2,b37),(X3,R12,A2,b38),(X3,R12,A2,b39),(X3,R12,A2,b40),(X3,R12,A2,b41),(X 3,R12,A2,b42),(X3,R12,A2,b43),(X3,R12,A2,b44),(X3,R12,A2,b45),(X3,R12,A2,b46),(X3, R12,A2,b47),(X3,R12,A2,b48),(X3,R12,A2,b49),(X3,R12,A2,b50),(X3,R12,A2,b51),(X3,R1 2,A2,b52),(X3,R12,A2,b53),(X3,R12,A2,b54),(X3,R12,A2,b55),(X3,R12,A2,b56),(X3,R12, A2,b57),(X3,R12,A2,b58),(X3,R12,A2,b59),(X3,R12,A2,b60),(X3,R12,A2,b61),(X3,R12,A2 ,b62),(X3,R12,A2,b63),(X3,R12,A2,b64),(X3,R12,A2,b65),(X3,R12,A2,b66),(X3,R12,A2,b6 7),(X3,R12,A2,b68),(X3,R12,A2,b69),(X3,R12,A2,b70),(X3,R12,A2,b71),(X3,R12,A2,b72), (X3,R12,A2,b73),(X3,R12,A2,b74),(X3,R12,A2,b75),(X3,R12,A2,b76),(X3,R12,A2,b77),(X 3,R12,A2,b78),(X3,R12,A2,b79),(X3,R12,A2,b80),(X3,R12,A2,b81),(X3,R12,A2,b82),(X3, R12,A2,b83),(X3,R12,A2,b84),(X3,R12,A2,b85),(X3,R12,A2,b86),(X3,R12,A2,b87),(X3,R1 2,A2,b88),(X3,R12,A2,b89),(X3,R12,A2,b90),(X3,R12,A2,b91),(X3,R12,A2,b92),(X3,R12, A2,b93),(X3,R12,A2,b94),(X3,R12,A3,b1),(X3,R12,A3,b2),(X3,R12,A3,b3),(X3,R12,A3,b4) ,(X3,R12,A3,b5),(X3,R12,A3,b6),(X3,R12,A3,b7),(X3,R12,A3,b8),(X3,R12,A3,b9),(X3,R12 ,A3,b10),(X3,R12,A3,b11),(X3,R12,A3,b12),(X3,R12,A3,b13),(X3,R12,A3,b14),(X3,R12,A 3,b15),(X3,R12,A3,b16),(X3,R12,A3,b17),(X3,R12,A3,b18),(X3,R12,A3,b19),(X3,R12,A3,b 20),(X3,R12,A3,b21),(X11,R12,A3,b22),(X3,R12,A3,b23),(X3,R12,A3,b24),(X3,R12,A3,b25 ),(X3,R12,A3,b26),(X3,R12,A3,b27),(X3,R12,A3,b28),(X3,R12,A3,b23,(X3,R12,A3,b30),( X3,R12,A3,b31),(X3,R12,A3,b32),(X3,R12,A3,b33),(X3,R12,A3,b34),(X3,R12,A3,b35),(X3 ,R12,A3,b36),(X3,R12,A3,b37),(X3,R12,A3,b38),(X3,R12,A3,b39),(X3,R12,A3,b40),(X3,R 12,A3,b41),(X3,R12,A3,b42),(X3,R12,A3,b43),(X3,R12,A3,b44),(X3,R12,A3,b45),(X3,R12, A3,b46),(X3,R12,A3,b47),(X3,R12,A3,b48),(X3,R12,A3,b49),(X3,R12,A3,b50),
(X3,R12,A3,b51),(X3,R12,A3,b52),(X3,R12,A3,b53),(X3,R12,A3,b54),(X3,R12,A3,b55),(X 3,R12,A3,b56),(X3,R12,A3,b57),(X3,R12,A3,b58),(X3,R12,A3,b59),(X3,R12,A3,b60),(X3, R12,A3,b61),(X3,R12,A3,b62),(X3,R12,A3,b63),(X3,R12,A3,b64),(X3,R12,A3,b65),(X3,R1 2,A3,b66),(X3,R12,A3,b67),(X3,R12,A3,b68),(X3,R12,A3,b69),(X3,R12,A3,b70),(X3,R12, A3,b71),(X3,R12,A3,b72),(X3,R12,A3,b73),(X3,R12,A3,b74),(X3,R12,A3,b75),(X3,R12,A3 ,b76),(X3,R12,A3,b77),(X3,R12,A3,b78),(X3,R12,A3,b79),(X3,R12,A3,b80),(X3,R12,A3,b8 1),(X3,R12,A3,b82),(X3,R12,A3,b83),(X3,R12,A3,b84),(X3,R12,A3,b85),(X3,R12,A3,b86), (X3,R12,A3,b87),(X3,R12,A3,b88),(X3,R12,A3,b89),(X3,R12,A3,b90),(X3,R12,A3,b91),(X 3,R12,A3,b92),(X3,R12,A3,b93) or (X3,R12,A3,b94).

The present compounds are useful in disease induced by the generation, secretion or deposition of-amyloid β protein, and are effective in treatment and/or prevention, and symptom improvement of such as dementia of the Alzheimer's type (Alzheimer's disease, senile dementia of Alzheimer type), Down's syndrome, memory impairment, prion disease (Creutzfeldt-Jakob disease), mild cognitive impairment (MBI), Dutch type of hereditary cerebral hemorrhage with amyloidosis, cerebral amyloid angiopathy, other type of degenerative dementia, mixed dementia with Alzheimer's and vascular type, dementia with Parkinson's Disease, dementia with progressive supranuclear palsy, dementia with Cortico-basal degeneration, Alzheimer's disease with diffuse Lewy body disease, age-related macular degeneration, Parkinson's Disease, amyloid angiopathy and so on.

Since the present compound has high inhibitory activity on BACE 1, and/or has high selectivity on other enzymes, it can be a medicament with reduced side effect. Further, since the compound has high effect of reducing amyloid β production in a cell system, particularly, has high effect of reducing amyloid P production in brain, it can be an excellent medicament- In addition, by converting the compound into an optically active compound having suitable stereochemistry, the compound can be a medicament having a larger safety margin on the side effect. In addition, the present compound also has advantages that metabolism stability is high, solubility is high, oral absorbability is high, good bioavailability is exhibited, clearance is good, brain transference is high, a half life is high, non-protein binding rate is high, hERG channel inhibition is low, CYP inhibition is low, CYP MBI (irreversible inhibition (mechanism-based inhibition)) is low and/or an Ames test is negative.

The present compounds can be administrated in combination with other pharmaceutical agents such as other therapeutic drugs for Alzheimer's disease, e.g., acetylcholinesterase inhibitors and the like. The present compounds can be treated with concomitantly with the anti-dementia agents such as Donepezil Hydrochloride, Tacrine, Galantamine, Rivastigmine, Zanapezil, Memantine, and Vinpocetine.

When the present compound is administered to a human, it can be administered orally as powders, granules, tablets, capsules, pills, solutions, or the like, or parenterally as injectables, suppositories, transdermal absorbable agents, insufflations, or the like. In addition, the present compound can be formulated into pharmaceutical preparations by adding pharmaceutical additives such as excipients, binders, wetting agents, disintegrating agents, lubricants and the like, which are suitable for formulations and an effective amount of the present compound.

A dose is different depending on state of disease, an administration route, and an age and a weight of a patient, and is usually 0.1 µg to 1 g/day, preferably 0.01 to 200 mg/day when orally administered to an adult, and is usually 1 µg to 10 g/day, preferably 0.1 to 2 g/day when parenterally administered.

### [Examples]

Following examples and test examples illustrate the present invention in more detail, but the present invention is not limited by these examples.

¹H-NMR was measured in deuterium chloroform (CDCl₃) using tetramethylsilane as an internal standard- δ values were shown as ppm. Binding constants (J) were shown as Hz. In the data, s, d, t, sext, m, br or brs means singlet, doublet, triplet, sextet, multiplet, broad or broad singlet, respectively.

In examples, the meaning of each abbreviation is as follows.
Me methyl
Et ethyl
Bz benzoyl
THF tetrahydrofuran

LC/MS data of the present compound were measured under the following condition, and a retention time and [M+H]+ are shown.
Column: Shim-pack XR-ODS (2.2 µm, i.d. 50 x 3.0 mm) (Shimadzu)
Flow rate: 1.6 mL/min.
Column oven: 50°C
UV detection wavelength: 254 nm
Mobile phase: [A] 0.1% formic acid-containing aqueous solution; [B] 0.1% formic acid-containing acetonitrile solution
Gradient: performing linear gradient of 10% to 100% solvent [B] for 3 minutes, and keeping 100% solvent [B] for 1 minute

### Reference Example 1

### Preparation of Compound 4

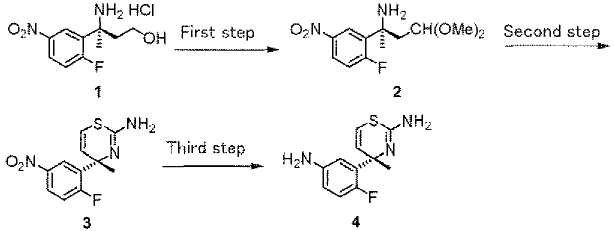

### First step

Aminoalcohol was prepared by the method described in Patent Literature 2 (WO 2008/133274) and was converted to hydrochloride salt, Compound 1, according to the conventional manner. To a solution of Compound 1 (6.1 g) in the tetrahydrofuran (60 ml) were added water (30 ml), sodium hydrogen carbonate (3.9 g) and N-((9-fluorenyl)methoxycarbonyloxy) succinic acid imide (7.8 g). The solution was stirred for 2 hours and 30 minutes at room temperature. After extraction with ethyl acetate, the organic layer was dried over anhydrous magnesium sulfate. The solvent was evaporated under reduced pressure. The residue was dissolved in dimethylsulfoxide (30 ml). 2-iodoxy benzoic acid (6.5 g) was added thereto. It was stirred at room temperature overnight. Water was added to the reaction mixture. After extraction by ethyl acetate, the organic layer was dried over anhydrous magnesium sulfate. The solvent was evaporated under reduced pressure. The residue was dissolved in methanol (100 ml). Amberlite 15 (2.0 g) was added thereto. It was heated under reflux for 2 hours and 30 minutes. After filtering, the solvent was evaporated under reduced pressure. The residue was dissolved in dimethylformamide. To the solution was added piperidine (2.8 ml). It was stirred at room temperature for 1 hour. Water was added thereto. The reaction mixture was extracted with ethyl acetate, washed with water and dried over anhydrous magnesium sulfate. The solvent was evaporated under reduced pressure. The residue was purified by column chromatography to give Compound 2 (3.4 g).
¹H-NMR (CDCl₃) δ:1.56(3H,d,J=0.9Hz), 2.10(1H,ddd,J=14.4,6.9,0.9Hz), 2.39(1H, ddd, J=14.4, 4.2, 2.1Hz), 3.18(3H,s), 3.19(3H,s), 4.13(1H, dd, J=6.9, 4.2Hz), 7.16(1H, dd, J =10.8, 8.9Hz), 8.16(1H,ddd,J=8.9,4.0,3.0Hz), 8.62(1H,dd,J=6.9,3.0Hz)

### Second step

To a solution in the acetone (12 ml) of Compound 2 (3.4 g) obtained at the first step was added at 0 °C, benzoyl isothiocyanate (1.7 ml). It was stirred for 30 minutes. The solvent was evaporated under reduced pressure. To the residue was added concentrated sulfuric acid (13 ml). It was stirred at 40 °C overnight. After adding ice (40 g) and tetrahydrofuran (10 ml) and making it alkaline with an aqueous ammonia 28%, it was extracted with ethyl acetate and dried over anhydrous magnesium sulfate. The solvent was removed under reduced pressure. The residue was purified by column chromatography to give Compound 3 (2.6 g).
¹H-NMR (CDCl₃) δ: 1.72(3H,d,J=0.9Hz), 6.26(1H,dd,J=9.5,4.6Hz), 6.35(1H,d,J=9.5Hz), 7.16(1H, dd, J=10.5, 8.9Hz), 8.13(1H,ddd,J=8.9,4.1,3.0Hz), 8.41(1H, dd, J=6.9, 3.0Hz)

### Third step

To a solution in acetic acid (13 ml) and water (1.3 ml) of Compound 3 (2.6 g) which were obtained at the second process was added at Iron (2.2g) at 50°C. It was stirred for 1 hour. After making it alkaline with an aqueous ammonia 28%, it was extracted with ethyl acetate. After removing an insoluble by filter, it was extracted with ethyl acetate and dried over anhydrous magnesium sulfate. The solvent was removed under reduced pressure to give Compound 4 (2.1 g).
¹H-NMR (CDCl₃) δ: 1.67(3H,s), 3.53(2H,br), 6.20-6.29(2H,m), 6.48(1H,ddd,J=8.4,4.0,3.0Hz), 6.75(1H, dd, J=6.6, 3.0Hz), 6.81(1H,dd,J=11.1,8.4Hz)

### Reference Example 2

### Preparation of Compound 8

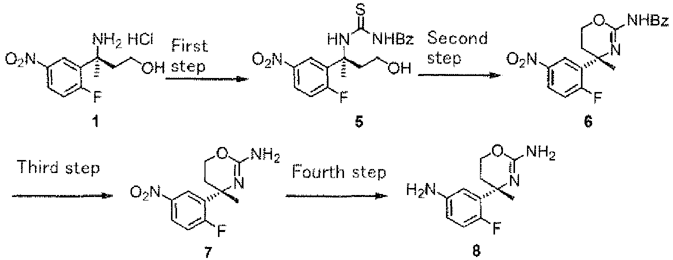

### First step

To a solution of Compound 1 (1.20 g) in acetone (70 ml) and water (40 ml) was added at 0 °C a solution of benzoyl isothiocyanate (0.82 g) in acetone (10 ml). It was stirred at room temperature for 2 hours. The solvent was removed under reduced pressure. The residue was purified by column chromatography to give Compound 5 (1.35 g).
¹H-NMR (CDCl₃) δ: 1.69(1H, t, J=4.7Hz), 2.14(3H,s), 2.21-2.31(1H,m), 2.73-2.83(1H,m), 3.78-3.98(2H,m), 7.15(1H, dd, J=11.1, 9.1Hz), 7.48-7.55(2H,m), 7.60-7.67(1H,m), 7.85(2H,d,7.2Hz), 8.14-8.20(1H,m), 8.30-8.34(1H,m), 8.81(1H,s), 11.56(1H,s)

### Second step

To a solution in acetonitrile (5 ml) of Compound 5 (1.26 g) obtained by the first step were added methyl iodide (0.30 ml) and diisopropyl ethyl amine (0.84 ml). It was stirred at room temperature for 32 hours and after then at 40 °C for 2 hours. Water was added thereto. The mixture was extracted with ethyl acetate, washed with water and brine, and dried over anhydrous magnesium sulfate. The solvent was removed under reduced pressure. The residue was purified by column chromatography to give Compound 6 (1.11 g).
¹H-NMR (CDCl₃) δ: 1.88(3H,s), 2.30-2.40(1H,m), 2.66-2.74(1H,m), 4.01-4.10(1H,m), 4.42-4.49(1H,m), 7.25-7.32(1H,m), 7.39-7.54(3H,m), 8.21-8.29(3H,m), 8.37(1H, dd, J=7.1, 2.9Hz), 11.90(1H,br)

### Third step

To Compound 6 (1.10 g) obtained at the second step was added concentrated sulfuric acid (3.28 ml). It was stirred at 80 °C for 1.5 hours. The reaction mixture was added to saturated sodium bicarbonate solution under ice-cooling. The mixture was extracted with ethyl acetate, washed with water and brine, and dried over anhydrous magnesium sulfate. The solvent was removed under reduced pressure. The residue was purified by column chromatography to give Compound 7 (0.615 g). ¹H-NMR (CDCl₃) δ: 1.58(3H,s), 2.22(2H, t, J=5.4Hz), 3.86-3.94(1H,m), 4.15-4.25(3H,m), 7.14(1H,dd,J=10.7,8.9Hz), 8.09-8.15(1H,m), 8.62(1H, dd, J=1.0, 3.0Hz)

### Fourth step

To a solution of Compound 7 (614 mg) obtained at the third step in THF (5 ml) was added 10% palladium-carbon (120 mg). It was stirred under hydrogen gas atmosphere for 20 hours. The reaction mixture was filtered through Celite. The filtrate was concentrated under reduced pressure. The obtained residue was purified by column chromatography. The obtained solid was recrystallized with ethyl acetate-hexane to give Compound 8 (400 mg).
¹H-NMR (CDCl₃) δ:1.54(3H,s), 1.97-2.07(1H,m), 2.30-2.38(1H,m) 3.54(2H,brs), 3.53(1H, dt, J=3.2, 10.6Hz), 4.10(1H,ddd,10.6,4.7,4.2Hz), 6.48(1H,ddd,8.4,3.7,3.2Hz), 6.78(1H,dd,J=11.8,8.4Hz), 6.86(1H,dd,J=6.9,3.0Hz)

### Example 1

### Preparation of Compound I-17

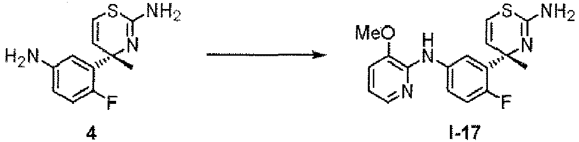

To a solution of Compound 4 (100 mg) in 2-propanol (5 ml) were added 2-chloro-3-methoxy pyridine (182 mg) and concentrated sulfuric acid (165 mg). It was stirred under refluxing for 48 hours. The solvent was removed under reduced pressure. Saturated sodium bicarbonate solution and water were added to the residue. The mixture was extracted with ethyl acetate. The organic layer was washed with brine and dried over anhydrous magnesium sulfate. The solvent was evaporated in vacuo. The obtained residue was purified by column chromatography. The obtained solid was recrystallized with diethylether-hexane to give Compound I-17 (76.8 mg).
¹H-NMR (CDCl₃) δ: 1.70(3H,s), 3.88(3H,s), 4.65(2H,br), 6.23-6.33(2H,m), 6.66(1H,dd,J=10.2,5.2Hz), 6.91-7.02(3H,m), 7.43(1H, dd, J=7.1, 2.9Hz), 7.78(1H, dd, J=5.0, 1.3Hz), 7.84-7.91(1H,m)

### Example 2

### Preparation of Compound 1-6

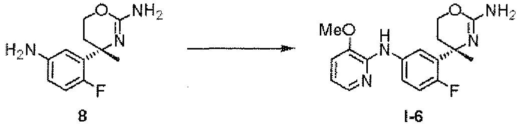

To a solution of Compound 8 (68 mg) in 2-propanol (5 ml) were added 2-chloro-3-methoxy pyridine (87 mg) and concentrated sulfuric acid (90 mg). It was stirred under reflux for 40 hours. Saturated sodium bicarbonate solution and water were added to the residue. The mixture was extracted with ethyl acetate. The organic layer was washed with brine and dried over anhydrous magnesium sulfate. The solvent was evaporated in vacuo. The obtained residue was purified by column chromatography. The obtained solid was recrystallized with diethylether-hexane to give Compound I-6 (25 mg).
¹H-NMR (CDCl₃) δ: 1.58(3H,s), 2.02-2.12(1H,m), 2.33-2.42(1H,m), 3.82-4.00(3H,m), 3.89(3H,s), 4.09-4.17(1H,m), 6.67(1H, dd, J=7.9, 5.0Hz), 6.93-6.99(2H,m), 7.01(1H,brs), 7.34(1H,dd,J=7.9,3.0Hz), 7.79(1H, dd, J=5.0, 2.3Hz), 8.02-8.08(1H,m)

### Example 3

### Preparation of Compound I-5

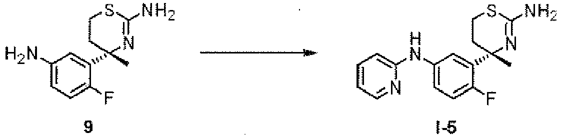

To a solution of Compound 9 (100 mg) prepared by the method described in Patent Literature 2 (WO2008/133274) in butanol (5 ml) were added 2-chloro pyridine (142 mg) and concentrated sulfuric acid (164 mg). It was stirred under reflux for 20 hours. The solvent was removed under reduced pressure. Saturated sodium bicarbonate solution and water were added to the residue. The mixture was extracted with ethyl acetate. The organic layer was washed with brine and dried over anhydrous magnesium sulfate. The solvent was evaporated in vacuo. The obtained residue was purified by column chromatography to give Compound 1-5 (106 mg).
¹H-NMR (CDCl₃) δ: 1.61(3H,s), 1.81-1.91(1H,m), 2.43-2.52(1H,m), 2.67-2.76(1H,m), 2.89-2.97(1H,m), 3.95(2H,br), 6.66-6.71(1H,m), 6.73(1H,d,J=8.6Hz), 6.83(1H,brs), 6.98(1H, dd, J=11.8, 8.7Hz), 7.17-7.21(1H,m), 7.30-7.36(1H,m), 7.41-7.48(1H,m), 8.15(1H,dd,J=5.0,1.0Hz)

### Example 4

### Preparation of Compound I-1

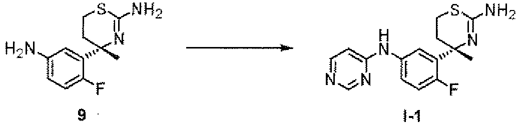

To a solution of Compound 9 (100 mg) in butanol (5 ml) were added 4-chloro pyrimidine hydrochloride (189 mg) and concentrated sulfuric acid (123 mg). It was stirred under reflux for 5 hours. The solvent was removed under reduced pressure. Saturated sodium bicarbonate solution and water were added to the residue. The mixture was extracted with ethyl acetate. The organic layer was washed with brine and dried over anhydrous magnesium sulfate. The solvent was evaporated in vacuo. The obtained residue was purified by column chromatography to give Compound I-1 (113 mg).
¹H-NMR (CDCl₃) δ: 1.55(3H,s), 1.80-1.90(1H,m), 2.43-2.52(1H,m), 2.60-2.70(1H,m), 2.89-2.97(1H,m), 5.00(2H,br), 6.41(1H,d,J=6.0Hz), 6.98(1H, dd, J=10.9, 9.2Hz), 7.17-7.23(2H,m), 8.17(1H,d,J=6.0Hz), 8.58(1H,s), 9.07(1H,br)

### Example 5

### Preparation of Compound I-2

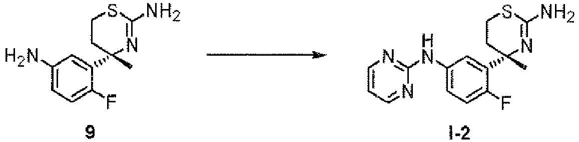

To a solution of Compound 9 (100 mg) in butanol (5 ml) were added 2-chloro pyrimidine (144 mg) and concentrated sulfuric acid (123 mg). It was stirred under reflux for 5 hours. The solvent was removed under reduced pressure. Saturated sodium bicarbonate solution and water were added to the residue. The mixture was extracted with ethyl acetate. The organic layer was washed with brine and dried over anhydrous magnesium sulfate. The solvent was evaporated in vacuo. The obtained residue was purified by column chromatography. The obtained solid was recrystallized with ethyl acetate-hexane to give Compound I-2 (16.5 mg).
¹H-NMR (CDCl₃) δ: 1.62(3H,s), 1.81-1.91(1H,m), 2.44-2.52(1H,m), 2.67-2.76(1H,m), 2.88-2.96(1H,m), 4.52(2H,br), 6.68(1H,t,J=4.7Hz), 7.01(1H, dd, J=11.7, 8.7Hz), 7.25-7.29(1H,m), 7.43(1H,brs), 7.75-7.81(1H,m), 8.38(2H,d,J=4.7Hz)

The following compounds in Table 1 were prepared in accordance with the above examples. In the tables, RT means a retention time (minutes).

Table 1

**[Table 1-1]**

| Comp. No. | Structure | **MS [M+1]** | **LC/MS RT** |
|---|---|---|---|
| **I-1** | 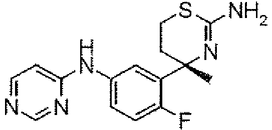 | **318** | **0.39** |
| **I-2** | 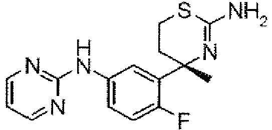 | **318** | **0.89** |
| **I-3** | 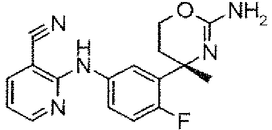 | **326** | **0.97** |
| **I-4** | 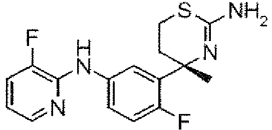 | **335** | **1.13** |
| **I-5** | 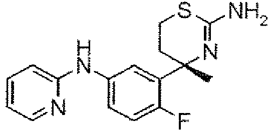 | **317** | **0.60** |
| **I-6** | 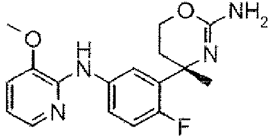 | **331** | **0.74** |
| **I-7** | 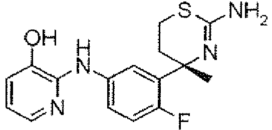 | **333** | **0.81** |

**[Table 1-2]**

| Comp. No. | Structure | **MS [M+1]** | **LC/MS RT** |
|---|---|---|---|
| **I-8** | 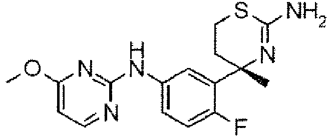 | **348** | **0.91** |
| **I-9** | 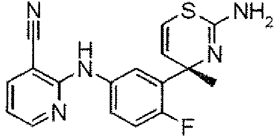 | **340** | **1.07** |
| **I-10** | 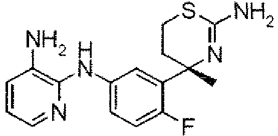 | **332** | **0.42** |
| **I-11** | 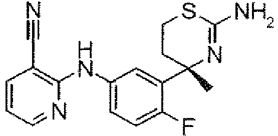 | **342** | **1.03** |
| **I-12** | 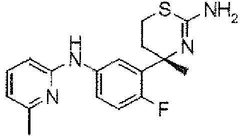 | **331** | **0.67** |
| **I-13** | 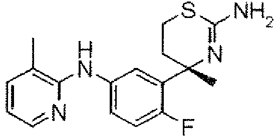 | **331** | **0.55** |
| **I-14** | 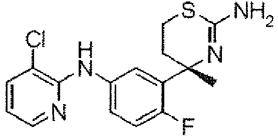 | **351, 353** | **1.17** |

**[Table 1-3]**

| Comp. No. | Structure | **MS [M+1]** | **LC/MS RT** |
|---|---|---|---|
| **I-15** | 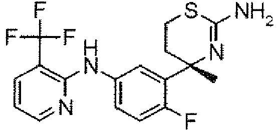 | **385** | **1.28** |
| **I-16** | 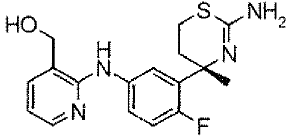 | **347** | **0.45** |
| **I-17** | 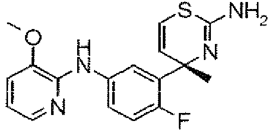 | **345** | **0.77** |
| **I-18** | 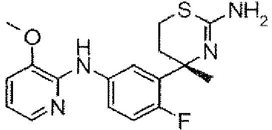 | **347** | **0.69** |
| **I-19** | 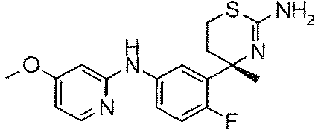 | **347** | **0.51** |
| **I-20** | 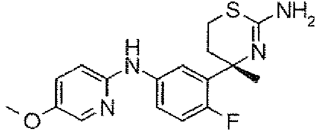 | **347** | **0.85** |
| **I-21** | 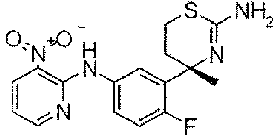 | **362** | **1.16** |

**[Table 1-4]**

| Comp. No | Structure | **MS [M+1]** | **LC/MS RT** |
|---|---|---|---|
| **I-22** | 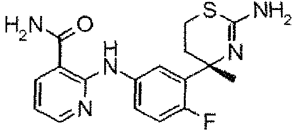 | **360** | **0.86** |
| **I-23** | 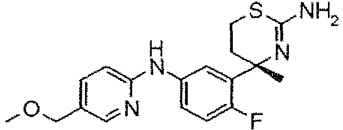 | **361** | **0.78** |
| **I-24** | 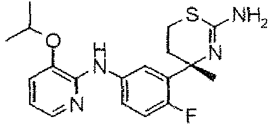 | **375** | **0.97** |
| **I-25** | 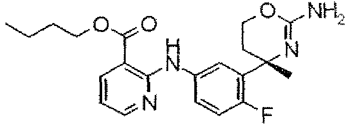 | **401** | **1.58** |
| **I-26** | 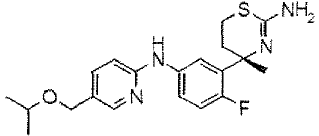 | **389** | **0.90** |
| **I-27** | 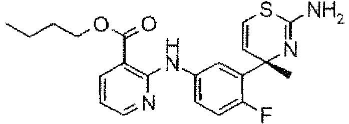 | **415** | **1.70** |
| **I-28** | 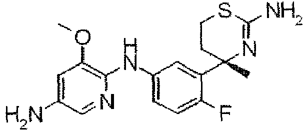 | **362** | **0.57** |

**[Table 1-5]**

| Comp. No. | Structure | **MS [M+1]** | **LC/MS RT** |
|---|---|---|---|
| **I-29** | 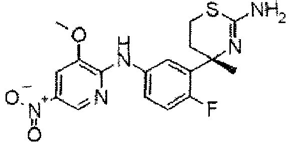 | **392** | **1.18** |
| **I-30** | 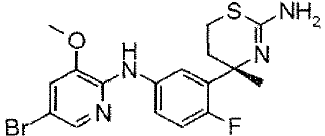 | **425, 427** | **1.35** |
| **I-31** | 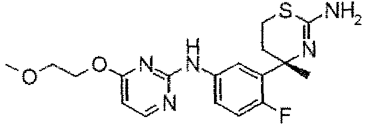 | **392** | **0.93** |

**[Table 1-6]**

| Comp. No. | Structure | **MS [M+1]** | **LC/MS RT** |
|---|---|---|---|
| **I-32** | 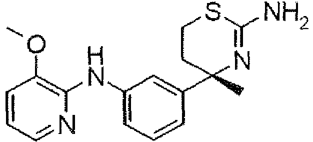 | **329** | **0.61** |
| **I-33** | 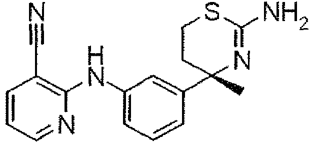 | **324** | **0.90** |
| **I-34** | 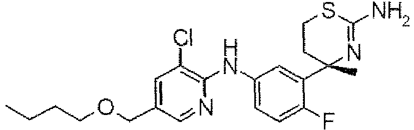 | **437, 439** | **1.60** |

**[Table 1-7]**

| Comp. No | Structure | **MS [M+1]** | **LC/MS RT** |
|---|---|---|---|
| **I-35** | 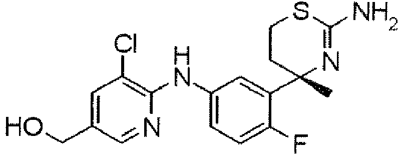 | **381, 383** | **0.97** |
| **I-36** | 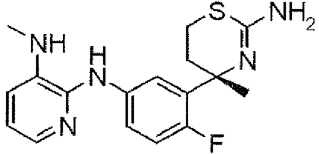 | **346** | **0.52** |
| **I-37** | 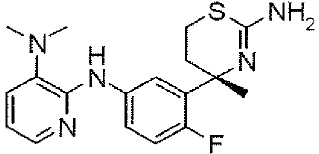 | **360** | **0.72** |
| **I-38** | 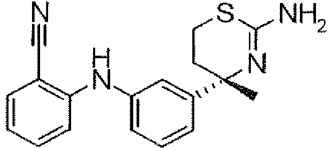 | **323** | **1.28** |
| **I-39** | 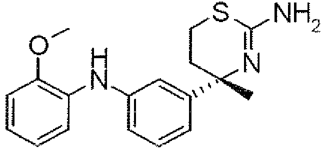 | **328** | **1.39** |

I-32:
   1H-NMR (CDCl₃) δ:1.57(s,3H), 1.80-1.90(m,1H), 2.15-2.24(m,1H), 2.72-2.90(m,2H), 3.89(s,3H), 4.35(br,2H), 6.68(dd,J=7.9,5.0Hz,1H), 6.90-6.98(m,1H), 7.06(brs,1H), 7.29(t,J=7.9Hz,1H), 7.41(s,1H), 7.80-7.87(m,2H).
I-33:
   1H-NMR (CDCl₃) δ:1.57(s,3H), 1.81-1.92(m,1H), 2.10-2.20(m,1H), 2.73-2.83(m,1H), 2.86-2.95(m,1H), 4.36(br,2H), 6.78(dd,J=7.7,5.0Hz,1H), 7.04(br,1H), 7.07.7.11(m,1H), 7.30-7.40(m,2H), 7.63-7.67(m,1H), 7.77(dd,J=7.7,2.0Hz,1H), 8.37(dd,J=5.0,2.0Hz,1H).
I-34:
   1H-NMR (CDCl₃) δ:0.92(t,J=7.2Hz,3H), 1.38(sext,J=7.2Hz,2H), 1.53-1.64(m,2H), 1.62(d,J=1.2Hz,3H), 1.86-1.96(m,1H), 2.40-2.50(m,1H), 2.71-2.81(m,1H), 2.91-3.00(m,1H), 3.45(t,J=6.5Hz,2H), 4.37(s,2H), 4.40(br,2H), 6.93(brs,1H), 7.01(dd,J=11.7,8.7Hz,1H), 7.24-7.30(m,1H), 7.59(d,J=2.0Hz,1H), 7.77-7.833(m,1H), 8.03(d,J=2.0Hz,1H).
I-35:
   1H-NMR (CDCl₃) δ:1.62(d,J=1.3Hz,3H), 1.85-1.96(m,1H), 2.42-2.52(m,1H), 2.70-2.80(m,1H), 2.90-3.00(m,1H), 3.70(br,2H), 4.56(s,2H), 6.92(brs,1H), 7.00(dd,J=11.8,8.9Hz,1H), 7.33(dd,J=7.2,2.9Hz,1H), 7.60(d,J=2.2Hz,1H), 7.70-7.76(m,1H), 8.02(d,J=2.2Hz,1H).
I-36:
   1H-NMR (CDCl₃) δ:1.59(s,3H), 1.79-1.90(m,1H), 2.44-2.53(m,1H), 2.74(dt,J=3.5,11.9Hz,1H), 2.81(d,J=4.9Hz,3H), 2.87-2.96(m,1H), 3.48(br,1H), 4.30(br,2H), 6.11(brs,1H), 6.79-7,03(m,4H), 7.26-7.34(m,1H), 7.70(d,J=4.7Hz,1H).
I-37:
   1H-NMR (CDCl₃) δ:1.62(d,J=1.3Hz,3H), 1.85-1.95(m,1H), 2.42-2.52(m,1H), 2.67(s,6H), 2.72-2.82(m,1H), 2.90-2.99(m,1H), 4.40(br,2H), 6.69(dd,J=7.6,5.0Hz,1H), 6.99(dd,J=11.9,8.7Hz,1H), 7.22(dd,J=7.2,2.9Hz,1H), 7.26(dd,J=7.6,1.7Hz,1H), 7.45(brs,1H), 7.91-7.97(m,2H).
I-38:
   1H-NMR (CDCl₃) δ:7.49(1H,dd,J=7.60,1.52Hz), 7.38-7.30(2H,m), 7.20(1H,d,J=8.62Hz), 7.15(1H,t,J=1.77Hz), 7.10-7.06(2H,m), 6.83(1H,dd,J=11.15,4.06Hz), 6.34(1H,s), 4.35(1H,brs), 2.96-2.90(1H,m), 2.79-2.72(1H,m), 2.15-2.09(1H,m), 1.90-1.83(1H,m), 1.56(3H,s).
I-39:
   1H-NMR (CDCl₃) δ:7.29(1H,dd,J=7.35,1.77Hz), 7.23(1H,t,J=7.86Hz), 7.08(1H,t,J=1.77Hz) 7.04(1H,dd,J=8,11,1.52Hz), 6.90-6.81(4H,m), 6.18(1H,s), 4.38(2H,brs), 3.89(3H,s), 2.91-2.85(1H,m) 2.80-2.74(1H,m), 2.18-2,12(1H,m), 1.86-1.79(1H,m), 1.56(3H,s).

The effect of the present compound is confirmed by the following test Examples.

### (Test Example 1: Assay of BACE 1 inhibiting activity)

48.5 µL of substrate peptide solution (Biotin-XSEVNLDAEFRHDSGC-Eu: X=ε-amino-n-capronic acid, Eu=Europium cryptate) was added to each well of 96-hole half-area plate (a black plate: Costar), and after addition of 0.5 µl of the test sample (dissolved in N,N'-dimethylformaldehyde) and 1 µl of Recombinant human BACE1(R&D Systems), the reaction mixture was incubated at 30°C for 3 hours. The substrate peptide was synthesized by reacting Cryptate TBPCOOH mono SMP (CIS bio international) with Biotin-XSEVNLDAEFRHDSGC (Peptide Institute, Inc.). The final concentrations of the substrate peptide and Recombinant human BACE1 were adjusted to 18 nmol/L and 7.4 nmol/L, respectively, and the reaction was performed in sodium acetate buffer (50 mmol/L sodium acetate, pH 5.0, 0,008% Triton X-100).
After the incubation for reaction, 50 µl of 8.0 µg/ml Streptavidin-XL665 (CIS bio international) dissolved in phosphate buffer (150 mmol/L K₂ HPO₄ -KH₂ PO₄, pH 7.0, 0.008 % Triton X-100, 0.8 mol/L KF) was added to each well and left stand at 30°C for an hour. After then, fluorescence intensity was measured (excitation wavelength: 320 nm, measuring wavelength: 620 nm and 665 nm) using Wallac 1420 multilabel counter (Perkin Elmer life sciences). Enzymatic activity was determined from counting ratio of each wavelength (10,000 x Count 665/Count 620) and 50% inhibitory concentration against the enzymatic activity was calculated. IC₅₀ values of the test compounds are indicated in Table 2. In the table, "µM" means "µmol/L".

**[Table 2]**

| Comp. No. | **IC50 (*µ*M)** |
|---|---|
| **I-11** | **0.100** |
| **I-14** | **0.083** |
| **I-18** | **0.052** |

Compounds I-2 to 9, 13, 15, 17, 21, 22 and 27 to 39 showed the IC₅₀ value of 5µM or less by the similar test.

### (Test Example 2: Measurement of β-amyloid (Aβ) production inhibitory effect in cell)

Neuroblastoma SH-SY5Y cells (SH/APPwt) with human wild-type β-APP excessively expressed therein were prepared at 8 X 105 cells/mL, and 150 µl portions thereof were inoculated into each well of a 96-well culture plate (Falcon). The cells were cultured for 2 hours at 37°C in a 5% gaseous carbon dioxide incubator. Then, a solution which had been preliminarily prepared by adding and suspending the test compound (DMSO (dimethyl sulfoxide) solution) so as to be 2 µl/50 µl medium was added to the cell sap. Namely, the final DMSO concentration was 1%, and the amount of the cell culture was 200 µl. After the incubation was performed for 24 hours from the addition of the test compound, 100 µl of the culture supernatant was collected from each fraction. The amount of the Aβ in each fraction was measured.

The Aβ amount was measured as follows. 10 µl of a homogeneous time resolved fluorescence (HTRF) measurement reagent (Amyloid β 1-40 peptide; IBA Molecular Holding, S.A.) and 10 µl of the culture supernatant were put into a 384-well half area microplate (black microplate, Costar) and mixed with each other, and then left standing overnight at 4°C while the light was shielded. Then, the fluorescence intensity (excitation wavelength: 337 nm, measurement wavelength: 620 nm and 665 nm) was measured with a Wallac 1420 multilabel counter (Perkin Elmer life sciences). The Aβ amount was determined from the count rate at each measurement wavelength (10000 X Count 665/Count 620), and the amount needed to inhibit Aβ production by 50 % (IC₅₀) was calculated from at least six different dosages. Table 3 shows the IC₅₀ value of each test compound.

**[Table 3]**

| Comp. No | **IC50** (*µ*M) |
|---|---|
| I-6 | **0.016** |
| I-11 | **0.009** |
| I-18 | **0.011** |

Compounds I-1, I-10, I-12, I-16, I-19, I-20, I-23, I-24, I-25 and I-26 showed the IC ₅₀ value of 3µM or less by the similar test.

### (Test Example 3: Lowering effect on brain β amyloid in rats)

A test compound was suspended in 0.5% methylcellulose, the final concentration was adjusted to 2 mg/mL, and this was orally administered to male Crj:SD rat (7 to 9 weeks old) at 10 mg/kg. In a vehicle control group, only 0.5% methylcellulose was administered, and an administration test was performed at 3 to 8 animals per group. A brain was isolated 3 hours after administration, a cerebral hemisphere was isolated, a weight thereof was measured, the hemisphere was rapidly frozen in liquid nitrogen, and stored at -80°C until extraction date. The frozen cerebral hemisphere was transferred to a homogenizer manufactured by Teflon (registered trade mark) under ice cooling, a 5-fold volume of a weight of an extraction buffer (containing 1% CHAPS ({3-[(3-chloroamidopropyl)dimethylammonio]-1-propanesulfonate}), 20 mM Tris-HCl (pH 8.0), 150 mM NaCl, Complete (Roche) protease inhibitor) was added, up and down movement was repeated, and this was homogenized to solubilize for 2 minutes. The suspension was transferred to a centrifugation tube, allowed to stand on an ice for 3 hours or more and, thereafter centrifuged at 100,000 x g, 4°C for 20 minutes. After centrifugation, the supernatant was transferred to an ELISA plate (product No. 294-62501, Wako Junyaku Kogyo) for measuring β amyloid 40. ELISA measurement was performed according to the attached instruction. The lowering effect was calculated as a ratio compared to the brain β amyloid 40 level of vehicle control group of each test.

### (Test Example 4: CYP3A4 fluorescent MBI test)

The CYP3A4 fluorescent MBI test is a test of investigating enhancement of CYP3A4 inhibition of a compound by a metabolism reaction, and the test was performed using, as CYP3A4 enzyme expressed in *Escherichia coli* and employing, as an index, a reaction in which 7-benzyloxytrifluoromethylchmarin (7-BFC) is debenzylated by the CYP3A4 enzyme to produce a metabolite, 7-hydroxytrifluoromethylchmarin (HFC) emitting fluorescent light.

The reaction conditions were as follows: substrate, 5.6 µmol/L 7-BFC; pre-reaction time, 0 or 30 minutes; reaction time, 15 minutes; reaction temperature, 25°C (room temperature); CYP3A4 content (expressed in *Escherichia coli*), at pre-reaction 62.5 pmol/mL, at reaction 6.25 pmol/mL (at 10-fold dilution); test drug concentration, 0.625, 1.25, 2.5, 5, 10, 20 µmol/L (six points).

An enzyme in a K-Pi buffer (pH 7.4) and a test drug solution as a pre-reaction solution were added to a 96-well plate at the composition of the pre-reaction, a part of it was transferred to another 96-well plate so that it was 1/10 diluted by a substrate in a K-Pi buffer, NADPH as a co-factor was added to initiate a reaction as an index (without preincubation) and, after a predetermined time of a reaction, acetonitrile/0.5 mol/L Tris (trishydroxyaminomethane) = 4/1 was added to stop the reaction. In addition, NADPH was added to a remaining preincubation solution to initiate a preincubation (with preincubation) and, after a predetermined time of a preincubation, a part was transferred to another plate so that it was 1/10 diluted with a substrate and a K-Pi buffer to initiate a reaction as an index. After a predetermined time of a reaction, acetonitrile/0.5 mol/L Tris (trishydroxyaminomethane) = 4/1 was added to stop the reaction. For the plate on which each index reaction had been performed, a fluorescent value of 7-HFC which is a metabolite was measured with a fluorescent plate reader. (Ex = 420 nm, Em = 535 nm).

Addition of only DMSO which is a solvent dissolving a drug to a reaction system was adopted as a control (100%), remaining activity (%) was calculated at each concentration of a test drug added as the solution, and IC₅₀ was calculated by reverse-presumption by a logistic model using a concentration and an inhibition rate. When a difference between IC₅₀ values is 5 µM or more, this was defined as (+) and, when the difference is 3 µM or less, this was defined as (-).

### (Result)

### Compound I-3: (-)

### (Test Example 5: CYP inhibition test)

Using commercially available pooled human hepatic microsome, and employing, as markers, 7-ethoxyresorufin O-deethylation (CYP1A2), tolbutamide methyl-hydroxylation (CYP2C9), mephenytoin 4'-hydroxylation (CYF2C19), dextromethorphan O-demethylation (CYP2D6), and terfenedine hydroxylation (CYP3A4) as typical substrate metabolism reactions of human main five CYP enzyme forms (CYP1A2, 2C9, 2C19, 2D6, 3A4), an inhibitory degree of each metabolite production amount by a test compound was assessed.

The reaction conditions were as follows: substrate, 0.5 µmol/L ethoxyresorufin (CYP1A2), 100 µmol/L tolbutamide (CYP2C9), 50 µmol/L S-mephenytoin (CYP2C19), 5 µmol/L dextromethorphan (CYP2D6), 1 µmol/L terfenedine (CYP3A4); reaction time, 15 minutes; reaction temperature, 37°C; enzyme, pooled human hepatic microsome 0.2 mg protein/mL; test drug concentration, 1, 5, 10, 20 µmol/L (four points).

Each five kinds of substrates, human hepatic microsome, and a test drug in 50 mmol/L Hepes buffer as a reaction solution was added to a 96-well plate at the composition as described above, NADPH, as a cofactor was added to initiate metabolism reactions as markers and, after the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent multilabel counter and tolbutamide hydroxide (CYP2C9 metabolite), mephenytoin 4' hydroxide (CYP2C19 metabolite), dextrorphan (CYP2D6 metabolite), and terfenadine alcohol (CYP3A4 metabolite) were quantified by LC/MS/MS.

Addition of only DMSO being a solvent dissolving a drug to a reaction system was adopted as a control (100%), remaining activity (%) was calculated at each concentration of a test drug added as the solution and IC₅₀ was calculated by reverse presumption by a logistic model using a concentration and an inhibition rate.

### (Result)

### Compound I-10: five kinds > 21 µM

### (Test Example 6: FAT Test)

Each 20 µL of freeze-stored *Salmonella typhimurium* (TA98 and TA100 strain) is inoculated in 10 mL of liquid nutrient medium (2.5% Oxoid nutrient broth No.2), and the cultures are incubated at 37°C under shaking for 10 hours. 9 mL of TA98 culture is centrifuged (2000 x g, 10 minutes) to remove medium, and the bacteria is suspended in 9 mL of Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄·7H₂O: 0.1 g/L), and the suspension is added to 110 mL of Exposure medium (Micro F buffer containing Biotin: 8 µg/mL, histidine: 0.2 µg/mL, glucose: 8 mg/mL). 3.16 mL of TA100 culture is added to 120 mL of Exposure medium to prepare the test bacterial solution. 588 µL of the test bacterial solution (or mixed solution of 498 µl of the test bacterial solution and 90 µL of the S9 mix in the case with metabolic activation system) are mixed with each 12 µL of the following solution: DMSO solution of the test substance (eight dose levels from maximum dose 50 mg/mL at 2-fold ratio); DMSO as negative control; 50 µg/mL of 4-nitroquinoline-1-oxide DMSO solution as positive control for TA98 without metabolic activation system; 0.25 µg/mL of 2-(2-furyl)-3-(5-nitro-2-furyl)acrylamide DMSO solution as positive control for TA100 without metabolic activation system; 40 µg/mL of 2-aminoanthracene DMSO solution as positive control for TA98 with metabolic activation system; or 20 µg/mL of 2-aminoanthracene DMSO solution as positive control for TA100 with metabolic activation system. 12 µL of the solution and 588 µL of the test bacterial solution (a mixed solution of 498 µl of the test bacterial solution and 90 µL of S9 mix with metabolic activation condition) were mixed and incubated at 37°C under shaking for 90 minutes. 460 µL of the bacterial solution exposed to the test substance is mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL was dispensed into 48 wells per dose in the microwell plates, and was subjected to stationary cultivation at 37°C for 3 days. A well containing the bacteria, which has obtained the ability of proliferation by mutation in the gene coding amino acid (histidine) synthetase, turns the color from purple to yellow due to pH change. The number of the yellow wells among the 48 total wells per dose is counted, and evaluate the mutagenicity by comparing with the negative control group. (-) means that mutagenicity is negative and (+) means positive.

### (Test Example 7: Solubility Test)

A 2-fold dilution series (12 points) of a 10 mM solution of a test compound in DMSO was added to a medium (JP-I, JP-II) (2%), and solubility was assessed by 3 stages (High; > 40 µM, Medium; 3-40 µM, Low; < 3 µM) from a turbidity after 4 hours (crystallization information).

### (Result)

### Compound I-9: High

### (Test Example 8: Metabolism Stability Test)

Using a commercially available pooled human hepatic microsomes, a test compound was reacted for a constant time, a remaining rate was calculated by comparing a reacted sample and an unreacted sample, thereby, a degree of metabolism in liver was assessed.

A reaction was performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution was added to 100 µL of a methanol/acetonitrile = 1/1 (v/v),,mixed and centrifuged at 3000 rpm for 15 minutes. The test compound in the supernatant was quantified by LC/MS/MS, and a remaining amount of the test compound after the reaction was calculated, letting a compound amount at 0 minute reaction time to be 100%.

### (Result)

### Compound I-33: 95%

### (Test Example 9: hERG Test)

For the purpose of assessing risk of an electrocardiogram QT interval prolongation, effects on delayed rectifier K+ current (I_{Kr}), which plays an important role in the ventricular repolarization process, was studied using HEK293 cells expressing human ether-a-go-go related gene (hERG) channel.

After a cell was retained at a membrane potential of -80 mV by whole cell patch clamp method using an automated patch clamp system (PatchXpress 7000A, Axon Instruments Inc.), I_{Kr} induced by depolarization pulse stimulation at +40 mV for 2 seconds and, further, repolarization pulse stimulation at -50 mV for 2 seconds was recorded. After the generated current was stabilized, extracellular solution (NaCl: 135 mmol/L, KCl: 5.4 mmol/L, NaH₂PO_{4:} 0.3 mmol/L, CaCl_{2·}H₂O: 1.8 mmol/L, MgCl_{2·}6H₂O: 1 mmol/L, glucose: 10 mmol/L, HEPES (4-(2-hydroxyethyl)-1-piperazine ethanesulfonic acid): 10 mmol/L, pH=7.4) in which the test compound had been dissolved at an objective concentration was applied to the cell under the room temperature condition for 10 minutes. From the recording I_{Kr}, an absolute value of the tail peak current was measured based on the current value at the resting membrane potential using an analysis software (DataXpress ver.1, Molecular Devices Corporation). Further, the % inhibition relative to the tail peak current before application of the test substance was calculated, and compared with the vehicle-applied group (0.1% dimethyl sulfoxide solution) to assess influence of the test substance on I_{Kr}.

### (Result)

### Compound I-1: 3.2%

### (Test Example 9: Powder solubility test)

Appropriate amounts of the test substances are put into appropriate containers. To the respective containers are added 200 µL of JP-1 fluid (sodium chloride 2.0 g, hydrochloric acid 7.0 mL and water to reach 1000 mL), 200 µL of JP-2 fluid (phosphate buffer (pH 6.8) 500 mL and water 500 mL), and 200 µL of 20 mmol/L TCA (sodium taurocholate)/JP-2 fluid (TCA 1.08 g and water to reach 100 mL). In the case that the test compound is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. The dilutions are observed for bubbles and precipitates, and then the containers are sealed and shaken. Quantification is performed by HPLC with an absolute calibration method.

### (Test Example 11: BA test)

Materials and methods for studies on oral absorption
(1) Animal: Mouse or Rat
(2) Breeding conditions: mouse or rat is allowed to freely take solid feed and sterilized tap water.
(3) Dose and grouping: orally or intravenously administered at a predetermined dose; grouping is as follows (Dose depends on the compound)
   Oral administration: 1 to 30 mg/kg (n=2 to 3)
   Intravenous administration: 0.5 to 10 mg/kg (n=2 to 3)
(4) Preparation of dosing solution: for oral administration, in a solution or a suspension state; for intravenous administration, in a solubilized state
(5) Administration method: in oral administration, forcedly administer into ventriculus with oral probe; in intravenous administration, administer from caudal vein with a needle-equipped syringe
(6) Evaluation items: blood is collected over time, and the plasma concentration of drug is measured by LC/MS/MS
(7) Statistical analysis: regarding the transition of the plasma concentration, the area under the plasma concentration-time curve (AUC) is calculated by non-linear least squares program WinNonlin (Registered trademark), and the bioavailability (BA) is calculated from the AUCs of the oral administration group and intravenous administration group.

### (Test Example 12: Brain distribution studies)

Intravenous administration is carried out to a rat by 0.5 mg/mL/kg dosage of the compound. 30 minutes later, all blood is drawn from vena cava inferior under isoflurane anesthesia for death from exsanguination. Then, the brain was extracted and 20-25% of homogenate thereof was prepared with distilled water. On the other hand, the obtained blood is used as plasma after centrifuging. Then, to the brain sample was added the control plasma at 1:1. To the plasma samples was added the control brains at 1:1. Each sample was measured using LC/MS/MS. The obtained area ratio (a brain/plasma) was used for the brain Kp value.

### (Result)

### Compound I-11: 2.2.

### Formulation Example 1

A granule containing the following ingredients is produced.

| Ingredient | Compound represented by the formula (I) | 10 mg |
|---|---|---|
| | Lactose | 700 mg |
| | Corn starch | 274 mg |
| | HPC-L | 16 mg |
| | | 1000 mg |

The compound represented by the formulae (I), and lactose are passed through a 60 mesh sieve. Corn starch is passed through a 120 mesh sieve. These are mixed with a V-type mixer. To the mixed powder is added a HPC-L (low viscosity hydroxypropylcellulose) aqueous solution, this is kneaded, granulated (extrusion granulation, pore diameter 0.5 to 1 mm), and dried. The resulting dry granule is passed through a vibration sieve (12/60 mesh) to give a granule.

### Formulation Example 2

A granule for filling a capsule containing the following ingredients is produced.

| Ingredient | Compound represented by the formula (I) | 15 mg |
|---|---|---|
| | Lactose | 90 mg |
| | Corn starch | 42 mg |
| | HPC-L | 3 mg |
| | | 150 mg |

The compound represented by the formula (I), and lactose are passed through a 60 mesh sieve. Corn starch is passed through a 120 mesh sieve. These are mixed, a HPC-L solution is added to the mixed powder, this is kneaded, granulated, and dried. The resulting dry granule is adjusted in a size, and 150 mg of it is filled into a No.4 hard gelatin capsule.

### Formulation Example 3

A tablet containing the following ingredients is produced.

| Ingredient | Compound represented by the formula (I) | 10 mg |
|---|---|---|
| | Lactose | 90 mg |
| | Microcrystalline cellulose | 30 mg |
| | CMC-Na | 15 mg |
| | Magnesium stearate | 5 mg |
| | | 150 mg |

The compound represented by the formula (I), lactose, microcrystalline cellulose, and CMC-Na (carboxymethylcellulose sodium salt) are passed through a 60 mesh sieve, and mixed. Magnesium stearate is mixed into the mixed powder to give a mixed powder for tabletting. The present mixed powder is directly compressed to give a 150 mg of a tablet.

### Formulation Example 4

The following ingredients are warmed, mixed, and sterilized to give an injectable.

| Ingredient | Compound represented by the formula (I) | 3 mg |
|---|---|---|
| | Nonionic surfactant | 15 mg |
| | Purified water for injection | 1 ml |

### [Industrial applicability]

The present compound can be a medicament useful as an agent for treating a disease induced by production, secretion and/or deposition of amyloid β protein.

## Claims

1. A compound of formula (I): 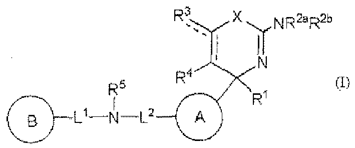 wherein R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group, R^{2a} and R^{2b} are each independently hydrogen, substituted or unsubstituted alkyl, substituted or unsubstituted acyl, substituted or unsubstituted alkoxycarbonyl or substituted or unsubstituted carbamoyl,
X is S or O,
when X is S, 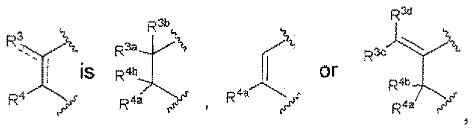 when X is O, 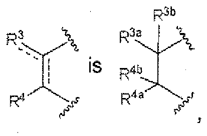 R^{3a}, R^{3b}, R^{4a} and R^{4b} are each independently
hydrogen, halogen, hydroxy, substituted or unsubstituted alkyl, substituted or unsubstituted alkenyl, substituted or unsubstituted alkynyl, substituted or unsubstituted acyl, substituted or unsubstituted acyloxy, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, carboxy, cyano, nitro, substituted or unsubstituted alkoxycarbonyl, substituted or unsubstituted alkenyloxycarbonyl,
substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, a substituted or unsubstituted carbocyclic group, a substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, a substituted or unsubstituted heterocyclic group, a substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{3a} and R^{3b} together with the carbon atom to which they are attached may form a substituted or unsubstituted carbocycle or a substituted or unsubstituted heterocycle, R^{4a} and R^{4b}together with the carbon atom to which they are attached may form a substituted or unsubstituted carbocycle or a substituted or unsubstituted heterocycle, R^{3c} and R^{3d} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, a substituted or unsubstituted carbocyclic group, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, a substituted or unsubstituted heterocyclic group, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl, or R^{3c} and
R^{3d} together with the carbon atom to which they are attached may form a substituted or unsubstituted non-aromatic carbocycle or a substituted or unsubstituted non-aromatic heterocycle,
R⁵ is hydrogen, substituted or unsubstituted alkyl, substituted or unsubstituted alkenyl, substituted or unsubstituted alkynyl or substituted or unsubstituted acyl,
L¹ and L² are each independently a bond;
substituted or unsubstituted alkylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy;
substituted or unsubstituted alkenylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; or
substituted or unsubstituted alkynylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; and
ring A is a substituted or unsubstituted carbocycle or a substituted or unsubstituted heterocycle,
provided that
1) when both of L¹ and L² are a bond, and 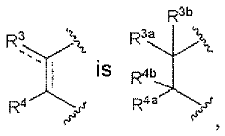 then ring B is a substituted or unsubstituted carbocycle, substituted or unsubstituted pyridine, substituted or unsubstituted pyrimidine or a substituted or unsubstituted 5-membered heterocycle,
2) when both of L¹ and L² are a bond, and 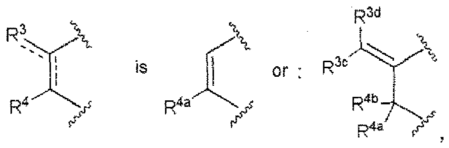 then ring B is a substituted or unsubstituted carbocycle or a substituted or unsubstituted heterocycle, and
3) when at least one of L¹ and L² is substituted or unsubstituted alkylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy;
substituted or unsubstituted alkenylene wherein the substituent is one or more selected from the group of halogen, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonyl imino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with one or more selected from the group of halogen, alkyl, hydroxy and alkoxy; or
substituted or unsubstituted alkynylene wherein the substituent is one or more selected from the group of halogen, alkoxy, halogenoalkoxy, hydroxyalkoxy, alkoxyalkoxy, acyl,
acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein each of a carbocyclic group and a heterocyclic group is optionally substituted with halogen, alkyl, hydroxy and alkoxy;
then ring B is substituted nitrogen-containing aromatic monocycle,
excluding the following compound 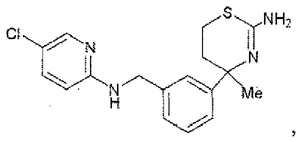 its pharmaceutically acceptable salt, or a solvate thereof.

2. The compound according to claim 1 wherein both of L¹ and L² are a bond, its pharmaceutically acceptable salt or a solvate thereof.

3. The compound according to claim 1 or 2 wherein ring A is substituted or unsubstituted benzene, ring B is substituted or unsubstituted pyridine or substituted or unsubstituted pyrimidine, its pharmaceutically acceptable salt or a solvate thereof.

4. The compound according to any one of claims 1 to 3 wherein 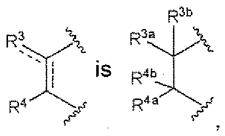 its pharmaceutically acceptable salt or a solvate thereof.

5. The compound according to claim 4 wherein R^{3a} is hydrogen, substituted or unsubstituted alkyl, cyano, substituted or unsubstituted alkoxycarbonyl, substituted or unsubstituted carbamoyl, a substituted or unsubstituted carbocyclic group or a substituted or unsubstituted heterocyclic group, R^{3b}, R^{4a} and R^{4b} are hydrogen, its pharmaceutically acceptable salt or a solvate thereof.

6. The compound according to claim 4 or 5 wherein X is S, its pharmaceutically acceptable salt or a solvate thereof.

7. The compound according to any one of claims 1 to 3 wherein 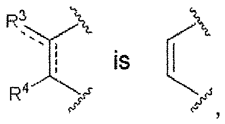 its pharmaceutically acceptable salt or a solvate thereof.

8. The compound according to any one of claims 1 to 7 wherein R¹ is Cl to C3 unsubstituted alkyl, its pharmaceutically acceptable salt or a solvate thereof.

9. The compound according to any one of claims 1 to 8 wherein both of R^{2a} and R^{2b} are hydrogen, its pharmaceutically acceptable salt or a solvate thereof.

10. A pharmaceutical composition comprising the compound according to any one of claims 1 to 9, its pharmaceutically acceptable salt or a solvate thereof as an active ingredient.

11. A pharmaceutical composition having BACE1 inhibitory activity comprising the compound according to any one of claims 1 to 9, its pharmaceutically acceptable salt or a solvate thereof as an active ingredient.

12. The pharmaceutical composition according to claim 10 or 11, which is a medicament for treating the diseases induced by production, secretion or deposition of amyloid-β proteins.

13. The pharmaceutical composition according to claim 10 or 11, which is a medicament for treating Alzheimer's disease.

14. A method for inhibiting BACE1 activity comprising administering the compound according to any one of claims 1 to 9, its pharmaceutically acceptable salt or a solvate thereof.

15. The compound according to any one of claims 1 to 9, its pharmaceutically acceptable salt or a solvate thereof for use in a method for inhibiting BACE1 activity.

16. A method for treating diseases induced by production, secretion or deposition of amyloid-β proteins comprising administering the compound according to any one of claims 1 to 9, its pharmaceutically acceptable salt or a solvate thereof.

17. The compound according to any one of claims 1 to 9, its pharmaceutically acceptable salt or a solvate thereof for use in a method for treating diseases induced by production, secretion or deposition of amyloid-β proteins.

18. A method for treating Alzheimer's disease comprising administering the compound according to any one of claims 1 to 9, its pharmaceutically acceptable salt or a solvate thereof.

19. The compound according to any one of claims 1 to 9, its pharmaceutically acceptable salt or a solvate thereof for use in a method for treating Alzheimer's disease.
